(19) Europäisches Patentamt
European Patent Office
Office européen des brevets

(11) EP 3 359 234 B1

(12) **EUROPÄISCHE PATENTSCHRIFT**

(45) Veröffentlichungstag und Bekanntmachung des Hinweises auf die Patenterteilung:
**28.04.2021 Patentblatt 2021/17**

(21) Anmeldenummer: **16781345.0**

(22) Anmeldetag: **06.10.2016**

(51) Int Cl.:
***A61M 15/00*** *(2006.01)* ***B05B 11/00*** *(2006.01)*

(86) Internationale Anmeldenummer:
**PCT/EP2016/073929**

(87) Internationale Veröffentlichungsnummer:
**WO 2017/060386 (13.04.2017 Gazette 2017/15)**

(54) **VERFAHREN ZUR BESCHICHTUNG MIKROSTRUKTURIERTER BAUTEILE**

METHOD FOR COATING MICROSTRUCTURED COMPONENTS

PROCEDE DE REVETEMENT DE COMPOSANTS MICRO-STRUCTURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.10.2015 EP 15189061**

(43) Veröffentlichungstag der Anmeldung:
**15.08.2018 Patentblatt 2018/33**

(73) Patentinhaber: **Boehringer Ingelheim International GmbH**
**55216 Ingelheim am Rhein (DE)**

(72) Erfinder: **FRACHE, Daniel**
**55216 Ingelheim am Rhein (DE)**

(74) Vertreter: **Von Rohr Patentanwälte Partnerschaft mbB**
**Rüttenscheider Straße 62**
**45130 Essen (DE)**

(56) Entgegenhaltungen:
**WO-A2-2004/089551** **WO-A2-2010/112358**
**DE-B4-102005 015 573**

- **FADEEV A Y ET AL: "TRIALKYLSILANE MONOLAYERS COVALENTLY ATTACHED TO SILICON SURFACES: WETTABILITY STUDIES INDICATING THAT MOLECULAR TOPOGRAPHY CONTRIBUTES TO CONTACT ANGLE HYSTERESIS", LANGMUIR, AMERICAN CHEMICAL SOCIETY, US, Bd. 15, 1. Januar 1999 (1999-01-01), Seiten 3759-3766, XP001053741, ISSN: 0743-7463, DOI: 10.1021/LA981486O**
- **DALBY R ET AL: "A review of the development of Respimat<(>R) Soft Mist(TM) Inhaler", INTERNATIONAL JOURNAL OF PHARMACEUTICS, ELSEVIER BV, NL, Bd. 283, Nr. 1-2, 28. September 2004 (2004-09-28), Seiten 1-9, XP004560626, ISSN: 0378-5173, DOI: 10.1016/J.IJPHARM.2004.06.018**

EP 3 359 234 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die vorliegende Erfindung betrifft das technische Gebiet der Mikrofluidik. Insbesondere betrifft die vorliegende Erfindung ein Verfahren zur Oberflächenmodifizierung, insbesondere Hydrophobierung, von mikrostrukturierten Substraten bzw. Bauteilen mit polaren Oberflächen.

Weiterhin betrifft die vorliegende Erfindung ein mikrostrukturiertes Bauteil, aufweisend eine Oberflächenmodifizierung. Darüber hinaus betrifft die vorliegende Erfindung ein mikrostrukturiertes Bauteil, insbesondere ein Düsensystem, eines mikrofluiden Systems mit modifizierter Oberfläche.

Weiterhin wird beschrieben eine Austragsvorrichtung, insbesondere einen Zerstäuber für Fluide, vorzugsweise auf dem Gebiet der Medizin.

Schließlich betrifft die vorliegende Erfindung ein Verfahren zur Evaluation von Oberflächenmodifizierungen mikrostrukturierter Bauteile.

In der Medizin, werden Zerstäuber insbesondere bei der Behandlung von Atemwegserkrankungen als Inhalationsgeräte eingesetzt. So werden beispielsweise asthmatische Erkrankungen und chronische Bronchitis durch Inhalationstherapien behandelt. Für die moderne, qualifizierte Inhalationstherapie stellen insbesondere das Asthma bronchiale und die chronische Bronchitis, die auch als COPD (Chronic Obstructive Pulmonary Disease) bezeichnet wird, das Hauptindikationsgebiet dar. Beide Erkrankungen gehören zu den sogenannten obstruktiven Atemwegserkrankungen, die insgesamt ca. 90 % aller Atemwegserkrankungen ausmachen.

Die chronische Bronchitis mit ihrem charakteristischen progressiven Rückgang der respiratorischen Leistungsfähigkeit ist eine der Hauptursachen für das Aufkommen von Invalidität und Tod weltweit. Insbesondere das Auftreten von Exazerbationen und die damit verbundenen Krankenhausaufenthalte machen sowohl das Asthma bronchiale als auch die COPD zu einem hohen Kostenpunkt in den weltweiten Gesundheitsbudgets. Aufgrund der global steigenden Erkrankungszahlen wird der Behandlung und Erforschung dieser beiden Atemwegserkrankungen auch in Zukunft ein hoher Stellenwert zukommen müssen.

Das Ziel einer inhalativen Arzneistofftherapie ist die Deposition einer Wirksubstanz in der Lunge. Da ein Großteil der therapeutisch eingesetzten Verbindungen potentiell auch systemisch wirksam ist, beinhaltet die inhalative Applikation dieser Wirkstoffe viele Vorteile gegenüber einer oralen oder intravenösen Gabe. Im Idealfall wird nur das betroffene Organ behandelt und es können lokal hohe wirksame Konzentrationen erreicht werden. Zudem erfolgt in der Regel ein schneller Wirkungseintritt und das Auftreten systemischer Nebenwirkungen ist selten. Durch die Markteinführung neuer Bronchodilatatoren und Entzündungshemmer konnte in den letzten Jahren die Situation für viele Asthma- bzw. COPD-Patienten verbessert werden. Aber auch Innovationen im Bereich der Geräte-Entwicklung haben mit einem hohen Anteil dazu beigetragen (vgl. Ambrosino, N. and P. Paggiaro, The management of asthma and chronic obstructive pulmonary disease: current status and future perspectives. Expert.Rev.Respir.Med., 2012. 6(1): p. 117-127).

Der Erfolg einer Inhalationstherapie ist abhängig von der inhalierten Arzneistoffmenge und dessen Verteilung in den Atemwegen. Diese wird auf vielfältige Weise von den unterschiedlichsten Faktoren beeinflusst. Hierzu zählen die charakteristischen Eigenschaften des Aerosols selbst, das zur Applikation benutzte Inhalationsgerät, die Art der Inhalation durch den Patienten und die Anatomie der Atemwege (vgl. Ganderton, D., Targeted delivery of inhaled drugs: current challenges and future goals. J.Aerosol Med., 1999. 12 Suppl 1: p. S3-S8; Pavia, D., Efficacy and safety of inhalation therapy in chronic obstructive pulmonary disease and asthma. Respirology., 1997. 2 Suppl 1: p. S5-10).

Eine besonders wichtige Eigenschaft des Aerosols ist dessen Teilchengrößenverteilung, denn sie beeinflusst die Deposition der Aerosolteilchen in den Atemwegen maßgebend. Aerosolpartikel mit einem aerodynamischen Durchmesser von 2 bis 5 $\mu$m scheiden sich bei der Inhalation in den kleineren Bronchiolen und den peripheren Atemwegen ab (vgl. Ariyananda, P.L., J.E. Agnew, and S.W. Clarke, Aerosol delivery systems for bronchial asthma. Postgrad.Med.J., 1996. 72(845): p. 151-156). Größere Partikel hingegen impaktieren in den oberen Luftwegen und wiederum kleinere Partikel gelangen in die Alveolen und können teilweise auch wieder ausgeatmet werden.

Für die Applikation eines Arzneistoffs per Inhalation stehen diverse tragbare Geräte, auch Devices oder Inhalator genannt, zur Verfügung. Hierzu zählen zum einen die Druckgasdosieraerosole (pMDI = engl. "**p**ressurized **m**etered **d**ose **i**nhaler"), die mit Chlorofluorcarbon (CFC) oder Hydrofluoralkanen (HFA) betrieben werden, und die Pulverinhalatoren (DPI). Lange Zeit waren die CFC-MDI die Basis in der Therapie von Asthma bronchiale und der chronischen Bronchitis. Doch viele Patienten haben Probleme mit der Anwendung dieser Inhalatorgruppe und bekommen nicht den optimalen therapeutischen Effekt in ihrer Inhalationstherapie. Die Grenzen der pMDI und die Bewegung zu umweltfreundlichen, treibgasfreien Inhalatoren haben die Entwicklung neuer Inhalationsgeräte beschleunigt.

Folgende Anforderungen werden nach Ganderton (s.o.) an ein optimales Inhalationsgerät gestellt:

- eine hohe Wirkstoffdeposition in der Lunge
- eine langsame Aerosolabgabe
- eine leichte Handhabung des Inhalationsgerätes
- ein Patienten-Feedback nach Dosisabgabe

- das Vorhandensein eines Zählwerks oder einer Inhaltsangabe
- ein handliches Format
- Umweltfreundlichkeit
- die Möglichkeit der Wiederverwendbarkeit.

Mit der Markteinführung des Respimat® Soft Mist™ Inhaltors der Firma Boehringer Ingelheim im Jahr 2003 gelangte ein neues Inhalationsgerät auf den Markt, das sehr viele der zuvor genannten Anforderungen erfüllt. Der Respimat® Soft Mist™ Inhalator (engl. soft mist = weicher Sprühnebel) ist ein treibgasfreier Zerstäuber, der die mechanische Energie einer Feder und das Aufeinanderprallen zweier Flüssigkeitsstrahlen ("Jets") zur Aerosolerzeugung nutzt. Aufgrund seines andersartigen Prinzips zur Aerosolerzeugung ließ er sich keiner der zuvor bestehenden Kategorien von Inhalationsgeräten zuordnen, sondern begründete eine neue Art von Inhalationsgeräten: die so genannten Soft Mist Inhalatoren (SMI). Die Aerosolwolke eines SMI ist langsamer als die von pMDI oder DPI und besitzt einen wesentlich höheren Feinpartikelanteil (FPF). Der Patient kann die Inhalation des Aerosols aufgrund der vergleichsweise langen Sprühdauer gut koordinieren und die Deposition in den tieferen Atemwegen ist durch den vergleichsweise hohen Feinpartikelanteil sehr effizient. Der hohe Anteil an lungengängigen Aerosolteilchen ermöglicht die Erniedrigung der applizierten Dosis und reduziert damit die Wahrscheinlichkeit des Auftretens unerwünschter Arzneimittelwirkungen (vgl. Dalby, R., M. Spallek, and T. Voshaar, A review of the development of Respimat Soft Mist Inhaler. Int.J.Pharm., 2004. 283(1-2): p. 1-9). Eine Vorrichtung vom SMI-Typ zur treibgasfreien Verabreichung einer dosierten Menge eines flüssigen Arzneimittels zur inhalativen Anwendung ist in der WO 91/14468 A1 offenbart.

Geräte bzw. Inhalatoren des SMI-Typs ermöglichen somit gegenüber den anderen Inhalationsverfahren des Standes der Technik eine deutlich effizientere und schonendere Behandlung von Atemwegserkrankungen. Die WO 2009/047173A2 zeigt ein Beispiel für ein SMI-Gerät. In dem beschriebenen Zerstäuber werden flüssige Arzneimittelformulierungen in einem Behälter gelagert und über ein Förderrohr in eine Druckkammer befördert, um schließlich durch eine Düse auszutreten. Die Düse weist eine Flüssigkeitseinlassseite und eine Flüssigkeitsauslassseite auf. Auf der Flüssigkeitseinlassseite befindet sich eine Öffnung durch die eine von der Druckkammer herkommende Flüssigkeit in die Düse eintreten kann. Auf der gegenüberliegenden Seite tritt die Flüssigkeit dann durch zwei Düsenöffnungen aus, die so ausgerichtet sind, dass die aus den Öffnungen austretenden Flüssigkeitsstrahlen aufeinanderprallen und dadurch zerstäubt werden. Dieses Zerstäubungsprinzip wird im Folgenden als "**d**ouble **j**et **i**mpinging" (DJI = engl. für "Zweistrahlimpaktion") bezeichnet.

Die Inhalatoren vom SMI-Typ eignen sich zur Ausbringung von flüssigen Formulierungen, vorzugsweise auf Basis von Wasser oder Wasser-Ethanol-Gemischen. Dabei können sie eine kleine Menge einer flüssigen Formulierung in den therapeutisch notwendigen Dosierungen binnen weniger Sekunden, vorzugsweise über eine Zeitdauer bzw. mit einer Sprühzeit von 1 bis 2 Sekunden, in ein therapeutischinhalativ geeignetes Aerosol vernebeln. Mit dem Gerät können Mengen von weniger als 100 μl, vorzugsweise weniger als 20 μl, mit beispielsweise einem Hub so zu einem Aerosol vernebelt werden, dass der inhalierbare Anteil des Aerosols vorzugsweise über 60% beträgt und/oder bereits der therapeutisch wirksamen Menge entspricht.

Mit den hier betrachteten Inhalatoren des SMI-Typs wird eine Arzneimittellösung mittels eines hohen Drucks (bevorzugt größer 50 bar) von bis zu 1000 bar, vorzugsweise bis zu 300 bar in eine lungengängige Aerosolwolke niedriger Geschwindigkeit überführt, die dann vom Patienten eingeatmet werden kann.

Bei der Verwendung von Zerstäubern mit kleinen Düsenöffnungen kann es in seltenen Fällen zu einer Verlegung der Düsenauslässe kommen, indem sich Rückstände aus der Formulierungslösung während der Benutzung des Zerstäubers als Verunreinigung an den Düsenauslässen anhaften. Dies führt zu einer Ablenkung der Flüssigkeitsstrahlen und - insbesondere bei der Verwendung von DJI-Düsen-zu einer Veränderung des Feinpartikelanteils. Durch Abscheidung von Partikeln im Bereich von Mikrodüsen kann es zum Auftreten von Düsenverlegungen bzw. Düsenverstopfungen kommen, wodurch die Funktion des Zerstäubers stark beeinflusst wird. Ein Phänomen, das im Folgenden unter dem Begriff "Jet-Divergency" zusammengefasst wird. Das Auftreten dieses Effekts ist selbstverständlich abhängig von den Bestandteilen und Zusammensetzungen der Formulierungen. Generell wäre jedoch auch für Formulierungen, für welche dieser Effekt eher als bei anderen auftritt, eine Reduktion dieser Düsenverlegungen, welche oftmals durch Partikelaggregationen verursacht wird, wünschenswert. Ein solches Auftreten von Aggregation ist beispielsweise aus mikrofluidischen Systemen mit fließenden Suspensionen bekannt.

Das Auftreten von Verstopfungen in Mikrokanälen ist ein komplexes Phänomen. Zu den potentiellen Mechanismen zählt zum einen die Anbindung der Partikel an die Kanalwandoberfläche aufgrund des Vorhandenseins von attraktiven Kräften und das anschließende Verstopfen durch Aggregation im Flussfeld oder durch sogenanntes "hydrodynamisches Bridging". "Hydrodynamisches Bridging" bezieht sich auf das Phänomen, dass Partikel kleiner als der Kanaldurchmesser simultan eine Verengung erreichen und diese daraufhin blockieren. Der Effekt ist zum einen abhängig von den kolloidalen Repulsionskräften als auch zum anderen von der anliegenden Zugkraft. Der Transport von Partikeln zur Kanalwand erfolgt z. B. durch Trägheitskräfte, durch Brownsche Molekularbewegung, durch Sedimentation oder aber durch Interzeption. Die Effizienz der Anbindung der Partikel ist dabei stets abhängig vom Verhältnis von attraktiven

und repulsiven Kräften.

Wünschenswert ist es beispielsweise, die Anwendbarkeit von SMIs und der DJI-Düsentechnologie auf komplexe Formulierungen, beispielsweise auf Basis von ethanolischen Lösungsmitteln auszudehnen. Interessant sind hierbei Anwendungen im Bereich der inhalativen Kortikoidtherapie. Die Möglichkeit, mit einem SMI kleine Formulierungsmengen in eine langsame Spraywolke zu überführen, lässt gerade im Bereich der stark wirksamen Kortikosteroide eine effizientere Inhalation erwarten, als dies derzeit bei herkömmlichen Inhalationsgeräts der Fall ist.

Die pharmakologische Basistherapie für das Asthma bronchiale bildet die alleinige oder auch kombinierte Gabe eines inhalativen Kortikosteroids (ICS) mit einem langwirksamen $\beta_2$-Agonisten (LABA). Erste Wahl in der Arzneimitteltherapie der chronischen Bronchitis ist die alleinige Gabe eines Bronchodilatators aus dem Bereich der LABAs und LAMAs (long-acting antimuscarinic antagonists). Je nach Sensitivität der COPD gegenüber einer Kortikoidtherapie ist aber auch die Therapie mit einem ICS möglich.

Die Ausweitung von Inhalationstherapien mittels der SMI- bzw. DJI-Technologie wäre generell wünschenswert, da diese eine sehr schonende und effiziente Verabreichung von Medikamenten ermöglicht. Hier stellt sich jedoch insbesondere bei komplexen Formulierungen das Problem, dass Wirkstoffe am Inhalator anhaften und so zu einer Verschlechterung des Spray-Bildes und schließlich zu einer Düsenverlegung führen können.

Darüber hinaus weisen langzeitstabile Formulierungen von Arzneimittel oftmals pH-Werte auf, welche zu Reaktionen innerhalb des Inhalators und beispielsweise zur Ausfällung von schwerlöslichen Verbindungen führen können, was wiederum in Düsenverlegungen durch Partikelanhaftungen resultieren kann.

Da durch die Verwendung von Inhalatoren des SMI-Typs das Auftreten unerwünschter Arzneimittelwirkungen weiter gesenkt werden könnte, hat es daher nicht an Versuchen gemangelt, Partikelanhaftungen zu vermeiden und eine Ausweitung der Anwendung von Inhalatoren des SMI-Typs bzw. von Düsen des DJI-Typs zu erweitern.

Um die Anhaftung von Partikeln von Verunreinigungen im Bereich der Düsenöffnungen zu vermeiden, offenbart die WO 2004/089551 A1 eine Mikro- oder Nanostrukturierung für Düsen vom DJI-Typ bzw. für Inhalatoren vom SMI-Typen, bei welcher die Außenoberfläche der Flüssigkeitsauslassseite mikro- oder nanostrukturiert ist.

Weiterhin offenbart die WO 2010/112358 A2 ein Verfahren zur Beschichtung einer insbesondere mikrostrukturierten Oberfläche eines aus unterschiedlichen Werkstoffen, insbesondere Glas und Silizium, bestehenden Bauteils, dessen Oberfläche zunächst aktiviert und anschließend beschichtet wird. Bei dem Bauteil handelt es sich bevorzugt um eine DJI-Düse, welche in Inhalatoren des SMI-Typs verwendet werden kann. Eine Aktivierung der Bauteiloberfläche erfolgt durch eine oxidierende Lösung, eine basische Lösung oder eine sauer-oxidierende Lösung.

Mit den vorgenannten Verfahren bzw. Modifikationen an den DJI-Düsen kann zwar eine Verstopfung bzw. eine Verlegung der Düsenauslassöffnungen verzögert bzw. in einigen Fällen verhindert werden, jedoch sind diese Verfahren teilweise sehr aufwendig und kostenintensiv, insbesondere im Fall der mikrostrukturierten Auslassöffnungen gemäß WO 2004/089551 A1, oder führen oftmals nicht verlässlich zu den gewünschten Ergebnissen.

Der vorliegenden Erfindung liegt somit die Aufgabe zugrunde, die zuvor genannten und mit dem Stand der Technik auftretenden Probleme abzuschwächen oder aber zumindest im Wesentlichen zu vermeiden. Die Erfindung ist nur limitiert durch seine Ansprüche.

Darüber hinaus ist eine weitere Aufgabe der vorliegenden Erfindung darin zu sehen, ein verbessertes Düsensystem zur treibgaslosen Ausbringung von Flüssigkeiten aus Inhalatoren bereitzustellen, welches eine breitere Anwendung an Wirkstoffkombinationen ermöglicht sowie deutlich verbesserte Anwendungs- und Performanceeigenschaften besitzt und insbesondere selbstreinigende Eigenschaften aufweist, bzw. ein Verfahren zur Verfügung zu stellen, mit dem die Anwendungseigenschaften von Düsen verbessert werden können und insbesondere selbstreinigende Eigenschaften an Düsen erzeugt oder Oberflächeneigenschaften von Düsen verändert werden können

Eine weitere Aufgabe der vorliegenden Erfindung ist es, das Anwendungsgebiet von Inhalatoren, insbesondere des SMI-Typs zu erweitern.

Gegenstand der vorliegenden Erfindung gemäß einem ersten Aspekt der vorliegenden Erfindung ist ein Verfahren zur Oberflächenmodifizierung von mikrostrukturierten Substraten nach Anspruch 1; weitere vorteilhafte Ausgestaltungen dieses Erfindungsaspektes sind Gegenstand der diesbezüglichen Unteransprüche.

Weiterer Gegenstand der vorliegenden Erfindung gemäß einem zweiten Aspekt der vorliegenden Erfindung ist ein mikrostrukturiertes Bauteil nach Ansprüche 9 bis 14; weitere vorteilhafte Ausgestaltungen dieses Erfindungsaspektes sind Gegenstand der diesbezüglichen Unteransprüche.

Weiter eine Austragsvorrichtung für Fluide beschrieben.

Schließlich ist wiederum weiterer Gegenstand der vorliegenden Erfindung gemäß einem weiteren Aspekt der vorliegenden Erfindung ein Verfahren zur Evaluation der Oberflächenmodifizierung als mikrostrukturierten Bauteils nach Anspruch 85; weitere vorteilhafte Ausgestaltungen dieses Erfindungsaspektes sind Gegenstand der diesbezüglichen Unteransprüche.

Es versteht sich von selbst, dass Besonderheiten, Merkmale, Ausgestaltungen und Ausführungsformen sowie Vorteile oder dergleichen, welche nachfolgend zu Zwecken der Vermeidung von unnötigen Wiederholungen nur zu einem Erfindungsaspekt ausgeführt werden, selbstverständlich in Bezug auf die übrigen Erfindungsaspekte entsprechend gelten, ohne dass es einer ausdrücklichen Erwähnung bedarf.

Weiterhin versteht es sich von selbst, dass bei der nachfolgenden Angabe von Werten, Zahlen und Bereichen die diesbezüglichen Werte-, Zahlen- und Bereichsangaben nicht beschränkend zu verstehen sind; es versteht sich für den Fachmann vielmehr von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen mit genormten bzw. standardisierten oder explizit angegebenen Bestimmungsverfahren oder mit dem Fachmann auf diesem Gebiet an sich geläufigen Bestimmungsmethoden ermittelt bzw. bestimmt werden können.

Darüber hinaus versteht es sich für den Fachmann von selbst, dass alle gewichts- oder mengenbezogenen Prozentangaben vom Fachmann derart ausgewählt werden, dass in der Summe 100% resultieren; dies versteht sich jedoch von selbst.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist somit ein Verfahren zur Oberflächenmodifizierung, insbesondere Hydrophobierung, von Substraten oder fluidischen Bauteilen, insbesondere mikrostrukturierten Bauteilen (wie Düsenkörpern), mit polaren Oberflächen, insbesondere für Hochdruckanwendungen, wobei ein Substrat oder mikrostrukturiertes Bauteil mit einem Modifizierungsreagenz in Kontakt gebracht, insbesondere behandelt, wird, wobei durch chemische und/oder physikalische Wechselwirkung der Bauteiloberfläche und des Modifizierungsreagenzes die Oberflächeneigenschaften des Substrats modifiziert werden, wobei das Modifizierungsreagenz mindestens ein Modifizierungsmittel aufweist und das Modifizierungsmittel ausgewählt ist aus der Gruppe von Silanen, Siloxanen, Polysiloxanen und/oder Silikonaten sowie deren Mischungen.

Im Rahmen der vorliegenden Erfindung ist es gelungen, durch eine Modifizierung der Oberfläche von Substraten oder mikrostrukturierten Bauteilen, deren Oberflächeneigenschaften derart zu verändern, dass die Anhaftung von Substanzen, insbesondere von Rückständen flüssiger Lösungen oder Dispersionen, welche über oder durch das mikrostrukturierte Substrat bzw. Bauteil geleitet werden, je nach Anforderung verstärkt oder vermindert werden kann.

Im Rahmen der vorliegenden Erfindung ist es insbesondere möglich, durch Beschichtung des Substrats bzw. mikrostrukturierten Bauteils die Oberflächeneigenschaften des Substrats bzw. Bauteils gezielt zu manipulieren, so dass die Wechselwirkung zwischen der Substrat- bzw. Bauteiloberfläche und den Substanzen, insbesondere Fluiden, welche über die Oberfläche geleitet werden, gezielt verändert bzw. reduziert werden können.

Insbesondere ist es im Rahmen der vorliegenden Erfindung möglich, die Benetzung der Substratoberfläche mit Fluiden, welche durch oder über das Substrat geleitet werden, zu verringern. Dies geschieht insbesondere durch eine Hydrophobierung der Oberfläche, d. h. die Oberfläche wird vorzugsweise mit einer hydrophoben Substanz behandelt, wodurch insbesondere die Oberflächenenergie des Substrates erniedrigt wird und hydrophile Substanzen nicht mehr in dem Maße wie zuvor mit der Oberfläche in Wechselwirkung treten können.

Unter einer Behandlung der Oberfläche mit dem Modifizierungsreagenz ist dabei im Rahmen der vorliegenden Erfindung zu verstehen, dass die Substratoberfläche in ihrem physikalischen und/oder chemischen Eigenschaften verändert wird.

Die Behandlung des Substrats oder mikrostrukturierten Bauteils mit dem Modifizierungsreagenz kann dabei auf jede geeignete Weise erfolgen. Üblicherweise wird das Substrat jedoch mit einer Lösung oder Dispersion des Modifizierungsreagenzes in Kontakt gebracht, insbesondere mit einer Lösung oder Dispersion des Modifizierungsreagenzes besprüht oder in diese getaucht.

Insbesondere ist im Rahmen der vorliegenden Erfindung erstaunlicherweise möglich, dass die Oberflächenmodifizierung auch bei Hochdruckanwendung hervorragende Ergebnisse liefert. Die mit dem erfindungsgemäßen Verfahren erhältliche Oberflächenmodifizierung wird auch bei hohen Drücken von bis zu 1.000 bar, insbesondere bei Drücken im Bereich von 50 bis 1.000 bar, insbesondere 200 bis 600 bar, vorzugsweise 250 bis 350 bar, üblicherweise nicht beschädigt oder gar zerstört (getestet für laminare, im Wesentliche parallel zur modifizierten Oberfläche verlaufende Strömung).

Die polare Oberfläche des mikrostrukturierten Substrats wird im Rahmen der vorliegenden Erfindung vorzugsweise durch polare funktionelle chemische Gruppen gebildet. Unter polaren funktionellen chemischen Gruppen sind dabei im Rahmen der vorliegenden Erfindung chemische funktionelle Gruppen, wie beispielsweise Hydroxylgruppen, Estergruppen, Carbonylgruppen, Amingruppen, Sulfangruppen oder ähnliche Gruppen zu verstehen, welche über ein permanentes Dipolmoment verfügen und die Oberfläche des mikrostrukturierten Substrats insbesondere für Wechselwirkung mit anderen polaren Gruppen empfänglich machen.

Unter einem mikrostrukturierten Bauteil sind im Rahmen der vorliegenden Erfindungen insbesondere Bauteile zu verstehen, welche an ihrer äußeren oder inneren Oberfläche Strukturen, beispielsweise in Form von Kanälen, Reliefs, etc. aufweisen mit einer Ausdehnung in zumindest einer Raumrichtung von nicht mehr als 100 $\mu$m, insbesondere nicht mehr als 50 $\mu$m, vorzugsweise nicht mehr als 20 $\mu$m, besonders bevorzugt im Bereich von 2 $\mu$m bis 8 $\mu$m. Im Rahmen der vorliegenden Erfindung ist es möglich, die Oberflächeneigenschaften besonders feiner Mikrostrukturen zu modifizieren, wobei die Ausprägung der Struktur erhalten bleibt, und nicht etwa durch die Umsetzung mit einem Beschichtungsreagenz bzw. durch die Aufbringung einer Beschichtung verdeckt wird oder an Konturschärfe verliert.

Im Rahmen der vorliegenden Erfindung kann es vorgesehen sein, dass das mikrostrukturierte Bauteil mindestens zwei unterschiedliche Materialien aufweist. Wenn das mikrostrukturierte Bauteil im Rahmen der vorliegenden Erfindung mindestens zwei unterschiedliche Materialien aufweist, so besitzen diese Materialien üblicherweise jeweils polare Oberflächen.

Im Rahmen der vorliegenden Erfindung hat es sich darüber hinaus bewährt, wenn alle Materialien des mikrostrukturierten Bauteils polare Oberflächen aufweisen.

Im Allgemeinen ist es im Rahmen der vorliegenden Erfindung vorgesehen, dass die Oberflächen der Materialien des Bauteils gemeinsam modifiziert werden. Die gemeinsame bzw. simultane Modifizierung der Bauteiloberflächen resultiert in einer besonders einfachen und schnell durchzuführenden Modifizierung, da nicht jedes Material einzeln hydrophobiert werden muss. Unter einer gemeinsamen bzw. simultanen Beschichtung ist dabei zu verstehen, dass die verschiedenen Materialien des Bauteils, vorzugsweise zeitlich und/oder örtlich gemeinsam, modifiziert werden. Dies bedeutet, dass die verschiedenen Materialien des Bauteils vorzugsweise zuerst miteinander verbunden werden und anschließend dieser Verbund oberflächenmodifiziert wird, insbesondere durch Umsetzung mit einem Modifizierungsreagenz bzw. Modifizierungsmittel.

Was nun das Material des mikrostrukturierten Bauteils anbelangt, welches im Rahmen der vorliegenden Erfindung verwendet werden kann, so hat es sich bewährt, wenn das Bauteil Glas, insbesondere silikatisches Glas, vorzugsweise Quarzglas und/oder Borosilikatglas, bevorzugt Borosilikatglas, z.B. in Form eines Deckels auf Mikrostrukturen, aufweist. Glas, insbesondere silikatisches Glas ist ein formbeständiges, stabiles und chemisch inertes Material. Insbesondere geht Glas üblicherweise keine Reaktion mit organischen Verbindungen, wie sie beispielsweise in Medikamenten bzw. pharmazeutischen Zusammensetzungen enthalten sind, ein.

Darüber hinaus werden im Rahmen der vorliegenden Erfindung gleichfalls gute Ergebnisse erhalten, wenn das Bauteils Silizium, insbesondere elementares Silizium, aufweist. Wenn das Bauteil elementares Silizium aufweist, so hat es sich bewährt, wenn zumindest ein Teil des Bauteils aus einem Siliziumwafer besteht bzw. diesen enthält. Elementares Silizium besitzt eine polare Oberfläche, da Silizium als unedles Halbmetall stets mit einer dünnen nativen Oxidschicht überzogen ist. Diese Oxidschicht eignet sich in hervorragender Weise zur Anbindung von Modifizierungsreagenzien bzw. -mitteln. Darüber hinaus lassen sich auf Silizium sehr gut Mikrostrukturen erzeugen, d. h. Mikrostrukturen können in einfacher Art und Weise auf Silizium, insbesondere Siliziumwafer, aufgebracht werden. Dies kann beispielsweise durch in der Halbleitertechnik etablierte Verfahren wie das Ätzen etc. geschehen.

Besonders gute Ergebnisse werden im Rahmen der vorliegenden Erfindung erhalten, wenn das Bauteil elementares Silizium und Glas aufweist, insbesondere hieraus besteht. Im Rahmen der vorliegenden Erfindung wird es somit bevorzugt, wenn das mikrostrukturierte Bauteil aus Glas und elementarem Silizium, welches insbesondere eine native Oxidschicht aufweist, besteht.

Was nun die Form der verwendeten Materialien des Bauteils anbelangt, so können diese vielgestaltig sein. Es hat sich jedoch bewährt, wenn die unterschiedlichen Materialien des Bauteils insbesondere zumindest im Wesentlichen quaderförmig, vorzugsweise plattenförmig sind. Bevorzugt handelt es sich bei den unterschiedlichen Materialien des Bauteils um Glaswafer und/oder Siliziumwafer. Eine quader- bzw. plattenförmige Ausbildung der Materialien erleichtert die maschinelle Verarbeitung und ermöglicht ein einfaches Verbinden der Materialien. Da die Materialien üblicherweise über ihre größten Flächen verbunden werden, können insbesondere bei plattenförmigen Werkstoffen, wie beispielsweise Wafern, sehr stabile Verbundstoffe erhalten werden.

Die Materialien des mikrostrukturierten Bauteiles können im Rahmen der vorliegenden Erfindung auf jede erdenkliche Art und Weise verbunden sein, wobei ein stoffschlüssiger Verbund erreicht werden sollte, welcher sowohl gas- als auch flüssigkeitsundurchlässig ist. Vorzugsweise sind die unterschiedlichen Materialien, insbesondere die Glas- und Siliziumwafer, fest miteinander verbunden, beispielsweise verklebt, verpresst oder mittels so genanntem "bonding" verbunden. Für weitere Einzelheiten bzgl. des "bonding" wird an dieser Stelle auf die europäische Patentschrift EP 1 644 129 B1 verweisen.

Im Rahmen der vorliegenden Erfindung werden die unterschiedlichen Materialien vorzugsweise ohne Fügemittel, wie beispielsweise Klebstoffe, miteinander verbunden, da bei Verwendung von Fügemitteln Rückstände in den Mikrostrukturen des Bauteils verbleiben und somit die Funktionsfähigkeit des Bauteils herabsetzen oder aufheben können.

Es hat sich gezeigt, dass das erfindungsgemäße Verfahren auch für die Oberflächenmodifizierung von Substraten oder Bauteilen mit Mikrostrukturen in jeglicher Art und Weise angewendet werden kann. Gute Ergebnisse werden für Bauteile mit Mikrostrukturen in Form von Kanälen erzielt. Hierbei kann es vorgesehen sein, dass die Kanäle einen Durchmesser im Bereich von 0,1 bis 50 $\mu$m, insbesondere 0,5 bis 40 $\mu$m, vorzugsweise 1 bis 20 $\mu$m, bevorzugt 2 bis 15 $\mu$m, besonders bevorzugt 2,5 bis 10 $\mu$m, ganz besonders bevorzugt 3 bis 8 $\mu$m, aufweisen. Das erfindungsgemäße Verfahren erlaubt eine Oberflächenmodifizierung, insbesondere ein Beschichten, sehr feiner Mikrostrukturen, ohne dass die Konturschärfe der Mikrostrukturen verloren geht bzw. abgeschwächt wird. Unter den Kanälen des mikrostrukturierten Bauteils sind im Rahmen der vorliegenden Erfindung Kanäle im Inneren des mikrostrukturierten Bauteils zu verstehen.

Was nun die Einbringung der Mikrostrukturen in das Bauteil bzw. die Bauteiloberfläche betrifft, so kann diese gleichfalls in vielfältiger Weise erfolgen. Üblicherweise werden im Rahmen der vorliegenden Erfindung die Mikrostrukturen jedoch in mindestens eines der Bauteilmaterialien eingebracht. In diesem Zusammenhang kann das Einbringen der Mikrostrukturen in mindestens eines der Bauteilmaterialien beispielsweise durch Bohren, Fräsen, Lasern, Ätzen, vorzugsweise Ätzen, vorgenommen werden. Bevorzugt wird elementares Silizium mit Mikrostrukturen versehen. Falls elementares Silizium verwendet wird, so wird die Mikrostruktur bevorzugt mittels Ätzen in das Bauteilmaterial eingebracht. In Silizium lassen sich mittels Ätztechnik auf besonders einfache Art und Weise in großtechnischem Maßstab feine Mikrostrukturen einbringen. Zur Erzeugung innerer Oberflächen, d. h. von Hohlräumen, wie beispielsweise Kanälen, in dem mikrostrukturierten Bauteil wird bevorzugt die Mikrostruktur in die Oberfläche eines der Bauteilmaterialien, insbesondere elementaren Siliziums, eingebracht und anschließend das Material mit einem zweiten Material, insbesondere Glas, gefügt, so dass die Mikrostruktur durch das zweite Material abgedeckt ist und sich im Innern des Kompositmaterials, des sogenannten Sandwich-Komposits, befindet.

Im Rahmen der vorliegenden Erfindung ist es im Allgemeinen vorgesehen, dass das Bauteil ein mikrofluides System ist. Das mikrostrukturierte Bauteil weist somit vorzugsweise Kanäle bzw. Hohlräume auf, welche einen Durchmesser von nur wenigen Mikrometern besitzen und durch welche Fluide geleitet werden. Eine Besonderheit der Mikrofluidik ist, dass sich Fluide, insbesondere Flüssigkeiten und Gase, vorzugsweise Flüssigkeiten, auf engem Raum anders verhalten als makroskopische Fluide. Beispielsweise spielen Reibungskräfte in mikrofluiden Systemen oftmals eine stärkere Rolle als in makroskopischen Systemen. Auch Kapillarkräfte sowie die Wechselwirkung zwischen Fluid und Oberfläche des mikrofluidischen Systems besitzen eine deutlich höhere Gewichtung als beispielsweise in makroskopischen Systemen. Auf diese Weise ist es möglich, die Anwendungseigenschaften von mikrostrukturierten Bauteilen durch eine Oberflächenmodifizierung entscheidend zu verändern. Im Rahmen der vorliegenden Erfindung werden darüber hinaus mikrofluide Systeme bevorzugt, da gerade bei Arzneimittelanwendungen besonders geringe Mengen an hochpotenten Arzneimittel ausgebracht und möglichst gleichmäßig zerstäubt werden müssen.

Nach dem zugrundeliegenden Prinzip lassen sich mikrofluide Systeme in grundsätzlich fünf verschiedene Gruppen unterteilen. Dies sind kapillargetriebene Systeme, druckgetriebene Systeme, zentrifugalgetriebene Systeme, elektrokinetische Systeme und die akustisch getriebenen Systeme. Im Folgenden sollen einige Beispiele kurz erläutert werden.

In kapillargetriebenen Systeme, wie den sogenannten "Lateral Flow Tests" (LAT), die auch als "Test strips" (z.B. Schwangerschaftsteststreifen) bezeichnet werden, wird der Flüssigkeitstransport durch vorhandene Kapillarkräfte erzielt.

In den sogenannten "linear actuated devices", die zu den druckgetriebenen Systemen gezählt werden, wird die Flüssigkeitsbewegung hingegen durch mechanische Dislokation (z.B. einem Pumpenkolben) erzielt. Dabei ist die Bewegung der Flüssigkeit oft nur auf eine Richtung begrenzt. Der Integrierungsgrad dieser Technik mit Kalibrierlösungen und Reaktionspuffern ist sehr hoch.

Zentrifugalgetriebene Systeme, auch "Centrifugal microfluidics" genannt, nutzen im System vorhandene Trägheits- und Kapillarkräfte. Relevante Trägheitskräfte sind dabei die Zentrifugalkraft, die Eulerkraft und die Corioliskräfte. Die zugrundeliegenden Substrate sind häufig scheibenförmig gestaltet und der aktive Flüssigkeitstransport ist in der Regel nach außen gerichtet.

Die elektrokinetischen Systeme benutzen elektrische Ladungen, elektrische Felder, elektrische Feldgradienten oder temporär flukturierende elektrische Felder. Ermöglicht wird dies durch die Verwendung von Elektroden, wodurch sich die unterschiedlichsten Effekte (Elektrophorese, Dielektrophorese, osmotischer Fluss, Polarisation) je nach verwendeter Flüssigkeit überlagern können.

Akustisch getriebene Systeme sind beispielsweise Surface Acoustic Waves zum Flüssigkeitstransport. Unter "Surface Acoustic Waves" (SAW) versteht man akustische Schockwellen auf einer festen Oberfläche. Eine akustische Schockwelle induziert beim Auftreffen auf einen Tropfen einen Druck. Übersteigt dieser Druck einen kritischen Wert, so bewegt sich der Tropfen auf der Substratoberfläche. Die feste Oberfläche ist in der Regel hydrophob beschichtet und erleichtert so die Tropfenbewegung. Durch Positionierung von verschiedenen SAW-Quellen auf einem Substrat, kann die Bewegung des Tropfens auf dem Substrat je nach Wunsch gestaltet werden.

Bevorzugt handelt es sich bei dem mikrofluiden System, welches im Rahmen der vorliegenden Erfindung verwendet wird, um ein druckgetriebenes mikrofluides System, vorzugsweise um ein Düsensystem für einen Inhalator nach dem SMI-Typ, wie er nachfolgend noch beschrieben wird. Der nachfolgend beschriebene Inhalator zählt mit seinem mikrofluiden Bauteil, dem Düsenkörper, der vorzugsweise sowohl Kanal-, Filter- und Düsenstrukturen enthält, zu dem druckgetriebenen System, insbesondere zu den "linear actuated devices".

Was nun die Ausgestaltung des mikrostrukturierten Bauteils anbelangt, so kann diese gleichfalls in weiten Bereichen variieren. Für die erfindungsgemäß bevorzugte Anwendung hat es sich jedoch bewährt, wenn das Bauteil mindestens eine Einlassöffnung und mindestens eine Auslassöffnung, vorzugsweise zwei Auslassöffnungen, für Fluide, insbesondere Flüssigkeiten, aufweist. Die Einlass- und Auslassöffnungen sind vorzugsweise verbunden, d. h. im Inneren des Bauteils befinden sich Hohlräume und Kanäle, durch welche vorzugsweise ein Fluid, insbesondere eine Flüssigkeit,

vorzugsweise eine flüssige pharmazeutische Zusammensetzung, geleitet werden kann.

Besonders gute Ergebnisse werden im Rahmen der vorliegenden Erfindung erhalten, wenn die Auslassöffnungen Düsen zur Ausbringung einer Flüssigkeit bilden. Im Rahmen der vorliegenden Erfindung ist es bevorzugt vorgesehen, dass die Auslassöffnungen Düsen bilden, durch welche eine Flüssigkeit, insbesondere eine flüssige pharmazeutische Zusammensetzung, aus dem mikrostrukturieren Bauteil herausgeleitet, insbesondere unter Druck herausgepresst, vorzugsweise gesprüht wird. Die Flüssigkeit wird vorzugsweise bei oder nach Austritt aus den Auslassöffnungen zerstäubt; dies kann beispielsweise durch speziell gestaltete Düsen oder durch Impaktion von aus den Düsen austretenden Flüssigkeitsstrahlen (beispielsweise bei den hier bevorzugten DJI-Düsen) geschehen.

Gemäß einer bevorzugten Ausführungsform der vorliegenden Erfindung weist das Bauteil zwischen der Einlassöffnung und der Auslassöffnung bzw. den Auslassöffnungen einen Filterbereich auf. Durch einen Filterbereich ist es möglich, größere Partikel, welche sich beispielsweise in einem Flüssigkeitsreservoir befinden, in dem die auszubringende Flüssigkeit, insbesondere die pharmazeutische Zusammensetzung, gelagert wird, von den Auslassöffnungen, insbesondere den Düsen fernzuhalten, so dass diese nicht durch größere Partikel verstopft werden bzw. der aus den Auslassöffnungen austretende Flüssigkeitsstrahl abgelenkt wird.

Üblicherweise ist es im Rahmen der vorliegenden Erfindung vorgesehen, dass die innere Oberfläche des Bauteils insbesondere zumindest im Wesentlichen modifiziert wird. Im Rahmen der vorliegenden Erfindung wird vorzugsweise die gesamte innere Oberfläche des Bauteils modifiziert. Besonders bedeutsam ist in diesem Zusammenhang jedoch, dass die Kanäle, welche in den Auslassöffnungen, insbesondere den Düsen, münden, modifiziert sind, so dass Ablagerungen in diesem Bereich, welche schnell zu einer Verjüngung der Düsen und somit zu einer Änderung des Sprühbildes führen, vermieden werden. Darüber hinaus sollen auch Düsenverlegungen bzw. ein Verstopfen der Düsen möglichst verhindert werden.

Unter einer inneren Oberfläche des mikrostrukturierten Bauteiles ist dabei im Rahmen der vorliegenden Erfindung die Oberfläche im Inneren des mikrostrukturierten Bauteils zu verstehen, welche durch Hohlräume, wie beispielsweise Kanäle und Düsen, gebildet werden. Diese Hohlräume bilden das mikrofluidische System, durch welches Fluide, insbesondere Flüssigkeiten, geleitet werden, um schlussendlich zerstäubt zu werden.

Gemäß einer bevorzugten Ausführungsform der vorliegenden Erfindung ist auch die äußere Oberfläche des Bauteils modifiziert, insbesondere im Bereich der Auslassöffnungen. Eine Modifizierung der äußeren Oberfläche des Bauteils, insbesondere im Bereich der Auslassöffnung, d. h. im Düsenbereich, verhindert gleichfalls eine Ablagerung von Partikeln bzw. von getrockneten Bestandteilen der ausgebrachten Flüssigkeit.

Unter der äußeren Oberfläche des mikrostrukturierten Bauteils ist dabei im Rahmen der vorliegenden Erfindung die äußere, d. h. die üblicherweise außen sichtbare, Oberfläche des mikrostrukturierten Bauteils zu verstehen.

Innere Oberflächen können im Rahmen der vorliegenden Erfindung beispielsweise erzeugt werden, indem die äußere Oberfläche eines Materials oder ersten Bauteilkomponente mit einer Mikrostruktur versehen wird und anschließend ein weiteres Material oder eine zweite Bauteilkomponente auf dieses erste Material, insbesondere die erzeugte Mikrostruktur, (z.B. in Form eines Deckels) aufgebracht wird. Hierdurch wird die äußere Mikrostruktur des einen Materials bzw. der ersten Bauteilkomponente zu einer inneren Mikrostruktur des Verbundmaterials bzw. des Bauteils.

Es kann im Rahmen der vorliegenden Erfindung vorgesehen sein, dass die gesamte Oberfläche, d. h. innere und äußere Oberflächen, modifiziert ist.

Im Rahmen der vorliegenden Erfindung ist es möglich, dass durch Oberflächenmodifizierung, insbesondere die Hydrophobisierung, die Eigenschaften der Bauteiloberfläche gezielt eingestellt werden können. Im Rahmen der vorliegenden Erfindung können die Eigenschaften der Bauteiloberfläche insbesondere durch geeignete Wahl eines Modifizierungsmittels bzw. eines Modifizierungsreagenzes eingestellt werden.

Das Modifizierungsreagenz kann in diesem Zusammenhang auf jede erdenkliche Weise mit der Oberfläche des Substrats bzw. Bauteils in Kontakt gebracht werden. Besonders gute Ergebnisse werden jedoch erhalten, wenn durch das Inkontaktbringen des Modifizierungsreagenzes mit der Oberfläche des Substrats eine Schicht, insbesondere eine Hydrophobisierungsschicht, auf die Oberfläche des Substrats bzw. Bauteils aufgebracht wird.

Unter einer Schicht ist dabei im Rahmen der vorliegenden Erfindung nicht nur die Schicht eines Bindemittels, wie beispielsweise eine Lackschicht mit üblicherweise mehreren Mikrometern Dicke zu verstehen, sondern vielmehr auch monomolekulare Schichten, welche aus einer einzigen Molekülschicht, sogenannten Monolagen bzw. Monoschichten (englisch: Monolayers), bestehen.

Eine derartige Schicht wird oftmals auch als Oberflächenfunktionalisierung oder -modifizierung bezeichnet, insbesondere wenn die Schicht in Form einer Monolage bzw. als Monolayer ausgebildet ist. Im Rahmen der vorliegenden Erfindung ist es dabei möglich, dass die Modifizierung, insbesondere die Beschichtung mehrfach wiederholt wird, so dass mehrere Schichten aufgetragen werden. Es wird jedoch bevorzugt, wenn nur ein einziger Beschichtungsverfahrensschrift durchgeführt, d. h. nur eine Schicht, insbesondere eine Monolage, aufgebracht wird.

Besonders gute Ergebnisse werden im Rahmen der vorliegenden Erfindung erhalten, wenn die Schicht in Form einer Monolage auf das Substrat oder Bauteil aufgebracht wird. Durch die Aufbringung von Monolagen ist es möglich, extrem dünne Beschichtungen zu erzielen, d. h. es wird nun ein Minimum an Beschichtungs- bzw. Modifizierungsmittel

verwendet. Wenn im Rahmen der vorliegenden Erfindung die Schicht in Form einer Monolage auf das Substrat oder Bauteil aufgebracht wird, so wird die Monolage üblicherweise als sogenannter Self-Assembled-Monolayer auf das Substrat oder Bauteil aufgebracht. Die Aufbringung der Schicht in Form einer Monolage hat den Vorteil, dass die Strukturen des Bauteils in voller Ausprägung erhalten bleiben und nicht etwa durch das Beschichtungsmittel bzw. Modifizierungsmittel an Konturschärfe verlieren.

Die auf das Substrat oder Bauteil aufgebrachte Schicht sollte vorzugsweise so dünn sein, dass keine Änderungen in der Geometrie oder Topologie der Mikrostrukturen auftreten, sondern deren jeweilige Konturschärfe erhalten bleibt. Üblicherweise treten Ablagerungen durch Partikel im vorderen Bereich der Kanalstrukturen beispielsweise des Düsenkörpers auf. Die Beschichtungen müssten daher hinreichend dünn sein, damit die Kanalgeometrie in diesem Bereich nicht oder nur unwesentlich geändert wird. Zusätzlich ist eine ausreichende mechanische Stabilität essentiell, um die starken Kräfte während der Anwendung zu überstehen. Zur Erzeugung dünner Schichten bzw. von Monoschichten kann eine Vielzahl von Methoden verwendet werden. Üblicherweise werden die Langmuir-Blodgett Methode (bevorzugt für Flachsubstrate; bzgl. der Herstellung und Charakterisierung von so genannten Langmuir-Filmen sei an dieser Stelle lediglich auf den einschlägigen Stand der Technik verwiesen, z.B. Paso, K., et al., Hydrophobic monolayer preparation by Langmuir-Blodgett and chemical adsorption techniques. Journal of colloid and interface science, 2008. 325(1): p. 228-235) und die Adsorption aus einer Gasphase oder, wie hier bevorzugt, aus einer hergestellten Lösung ("solution adsorption") verwendet. Die mit dem zuletzt genannten Verfahren erzeugten Schichten werden auch als sogenannte SAMs ("Self-Assembled Monolayers") bezeichnet. Im Folgenden wird auf die Herstellung von Self-Assembled Monolayers (SAMs) durch Flüssigbeschichtung näher eingegangen. Für die Beschichtung eines Kompositmaterials wie es im Rahmen der vorliegenden Erfindung vorzugsweise verwendet wird und welche oftmals auch als Sandwich-Systeme bezeichnet werden, stellt das Verfahren der Flüssigbeschichtung (solution adsorption) üblicherweise die geeignetste Methode dar, da sie aufgrund einer hervorragenden Spaltgängigkeit eine gleichmäßige Abscheidung von Molekülen in einer komplexen Mikrostruktur ermöglicht. Außerdem begünstigen Kapillareffekte in der Regel die Benetzung von innen liegenden Mikrostrukturen mit einem flüssigen Modifizierungsreagenz.

Der Ausdruck "Self-Assembled Monolayer" heißt übersetzt so viel wie "sich selbst organisierende Monolage". Ein Self-Assembled Monolayer ist eine Einzelschicht eines organischen Moleküls, das aus einer Flüssig- oder Gasphase auf ein festes Substrat aufgebracht wird. Bei geeigneter Auswahl eines Substrats und eines organischen Moleküls, werden die Moleküle beim Einbringen des Substrates in die Gas- bzw. Flüssigkeitsphase durch chemische Reaktion mit der SubstratOberfläche chemisch adsorbiert.

Bei der Ausbildung eines Self-Assembled Monolayers aus einer Flüssigphase, positionieren sich die Moleküle auf dem Substrat aufgrund intermolekularer Wechselwirkungen und richten sich zueinander aus. Es entsteht eine Monolage mit einem hohen Ordnungsgrad. Wenn die Ausbildung der Monolage abgeschlossen ist, endet das Wachstum der Schicht bzw. Layers, da die mit Molekülen bedeckte Substratoberfläche nicht weiter an der Reaktion teilnehmen kann. Einen solchen ausgebildeten, organischen, ultra-dünnen Film nennt man aufgrund seiner spontanen, sich selbst organisierenden Entstehung SAM ("Self-Assembled Monolayer"). Die zwei-dimensionale Anordnung der SAMs wird in Prozessen ausgebildet, in welchen das System einen Gleichgewichtszustand erreicht. Dies bedeutet, dass SAMs einem thermodynamisch stabilen Zustand durchaus sehr nahe kommen können.

Praktische Beispiele für Substratmaterialien bei der Kombinationen von Substraten und organischen Molekülen sind: Aluminiumoxid, Silberoxid oder Glas, Gold, Silber, Kupfer oder Galliumarsenid und Siliziumoxid, Titanoxid oder andere Oxide. Zugehörige Beispiele für organische Molekülen bei der Kombination mit Substraten sind: Carbonsäuren, organische Schwefelverbindungen wie Thiole oder (andere) organische Komponenten. Eine typische Kombination ist beispielsweise Gold als Substratmaterial mit Thiol.

Da die Moleküle flächendeckend über das gesamte Substrat auf der Oberfläche adsorbieren, ist es möglich, je nach Auswahl der terminalen Endgruppe, die Oberflächeneigenschaften des Substrates nach Wunsch zu modifizieren. Dies betrifft nicht nur die Reduktion der freien Oberflächenenergie durch Auswahl einer Alkyl- oder Fluoralkylgruppe, sondern kann durch die Auswahl von z.B. einer Amino- oder Epoxygruppe auch deren Reaktivität betreffen.

Die Knüpfung der Bindung (einer Siloxanbindung im Falle eines siliziumhaltigen Substrats) verleiht organosilanischen SAMs eine Stabilität von kovalentem Charakter. Sie wird nicht nur mit der Oberfläche verknüpft, sondern kann auch, je nach Molekülauswahl, mit den Nachbarmolekülen geknüpft werden, so dass ein Bindungs-Netzwerk (ein Siloxan-Netzwerk im Falle eines siliziumhaltigen Substrats) entsteht. Dies ist die Ursache, warum organosilanische SAMs hinsichtlich mechanischer Festigkeit und chemischer Stabilität weitaus stabiler als andere SAMS sind.

Sie bieten daher die größten Möglichkeiten hinsichtlich eines praktischen Einsatzes bezüglich der Oberflächenmodifikation bzw. Oberflächenfunktionalisierung.

Die Dicke eines SAMs beträgt oftmals nur 1 bis 2 nm und hängt von der Moleküllänge ab. Sie haben den Vorteil, dass sie gegenüber den vergleichsweise großen Polymeren keine Variation in der Molekülgröße haben, wie es bei Polymeren üblich ist. Bezüglich der Vermeidung von Adhäsion in Mikrostrukturen durch Oberflächenmodifizierung sind SAMs daher die im Rahmen der vorliegenden Erfindung bevorzugten eingesetzten Beschichtungen.

Was nun die Schichtdicke der auf das Substrat oder Bauteil aufgetragenen Schicht anbelangt, so kann diese

in insbesondere abhängig von der Kombination aus Substratmaterial und organischen Molekülen variieren. Beispielsweise kann die Schichtdicke nicht nur von der Länge der zur Beschichtung verwendeten Moleküle, sondern auch von ihrer räumlichen Anordnung auf der Oberfläche und/oder der Ausbildung von mehreren Molekülschichten übereinander beeinflusst werden. Besonders gute Ergebnisse werden im Rahmen der vorliegenden Erfindung jedoch erhalten, wenn die Schicht mit einer Schichtdicke von 0,1 bis 200 nm, insbesondere 0,2 bis 100 nm, vorzugsweise 0,3 bis 50 nm, bevorzugt 0,4 bis 10 nm, besonders bevorzugt 0,5 bis 5 nm, auf das Substrat oder Bauteil aufgebracht wird.

Üblicherweise wird im Rahmen der vorliegenden Erfindung die Schicht über chemische Bindungen, insbesondere über kovalente Bindungen, an das Substrat oder Bauteil gebunden. Das heißt, im Rahmen der vorliegenden Erfindung werden zwischen dem Modifizierungsmittel bzw. Beschichtungsmittel und der Substrat- oder Bauteiloberfläche kovalente Bindungen ausgebildet, da diese besonders dauerhafte und stabile Verbindungen ermöglichen.

Gemäß einer bevorzugten Ausführungsform der vorliegenden Erfindung weist das Modifizierungsreagenz mindestens ein Modifizierungsmittel, insbesondere mindestens eine der vorgenannten Organosiliziumverbindungen, auf. Bei dem Modifizierungsmittel kann es sich entweder um eine Einzelsubstanz oder aber um eine Mischung aus verschiedenen Substanzen handeln.

Bei einem Modifizierungsreagenz handelt es sich im Rahmen der vorliegenden Erfindung um die Substanz bzw. das Substanzgemisch, mit welchem das Bauteil bzw. Substrat in Kontakt gebracht wird. Das Modifizierungsreagenz kann beispielsweise in Form einer Lösung oder Dispersion vorliegen.

Unter einem Modifizierungsmittel ist im Rahmen der vorliegenden Erfindung eine chemische Verbindung zu verstehen, welche mit der Oberfläche in physikalische oder chemische Wechselwirkung, beispielsweise durch Physisorption oder Chemisorption, tritt an die Oberfläche des Bauteils gebunden wird, gegebenenfalls nach einer chemischen Reaktion.

Wenn die Oberflächenmodifizierung im Rahmen der vorliegenden Erfindung als Beschichtung durchgeführt wird, so werden das Modifizierungsreagenz und das Modifizierungsmittel auch als Beschichtungsreagenz und Beschichtungsmittel bezeichnet.

Wie zuvor ausgeführt, ist das Modifizierungsmittel ausgewählt aus der Gruppe von Silanen, Siloxanen, Polysiloxanen und/oder Silikonaten sowie deren Mischungen, insbesondere Silanen, Siloxanen und/oder Silikonaten sowie deren Mischungen, vorzugsweise Silanen.

Insbesondere mit Silanen lassen sich besonders dünne Schichten, insbesondere Monolagen, erzielen, welche darüber hinaus einen besonders festen Verbund mit der Substrat- oder Bauteiloberfläche eingehen können. Darüber hinaus sind Silane mit vielfältigen organischen Gruppen einerseits kommerziell erhältlich und andererseits ohne Weiteres herstellbar, so dass die Substrat- oder Bauteiloberfläche durch Reaktion mit Silanen im gewünschten Maße modifiziert werden kann, insbesondere im gewünschten Maße hydrophobiert werden kann.

Ein Molekül mit der Struktur $SiR_nX_{4-n}$ (R = organischer Rest, X = reaktive chemische Gruppe) wird in der Regel als Organosilan bezeichnet. Es leitet sich von der Struktur $SiH_4$ ab, in der Wasserstoff durch reaktive chemische Gruppen bzw. organischen Seitenketten ersetzt wird. Ein organischer Film kann beispielsweise durch Reaktion der funktionellen Gruppe mit einer Hydroxylgruppe einer oxidierten Substrat- oder Bauteiloberfläche gebildet werden.

Als Ausgangsstoff für die Synthese einer silanbasierten Schicht, insbesondere eines organosilanischen SAM, werden häufig Chlorsilane, wie z.B. die Trichloralkylsilane und/oder Alkoxyalkylsilane, verwendet. Die Trialkoxyalkylsilane sind Precursor-Verbindungen und müssen im Vorfeld der Reaktion mit der Substrat- oder Bauteiloberfläche hydrolytisch gespalten werden. Die Reaktionswege dieser Verbindungen sind im Vergleich mit anderen SAMs verhältnismäßig komplex, da bis zu drei reaktive Gruppen pro Molekül vorhanden sind, die eine Verbindung mit der Oberfläche oder untereinander eingehen können. Ein kleiner Wasseranteil ist für die Bereitstellung des für die Reaktion mit der Substrat- oder Bauteiloberfläche vorgesehenen Silanols notwendig, da es üblicherweise aus einer Hydrolysereaktion der Chlorgruppen bzw. Alkoxygruppen hervorgeht. Anschließend reagieren diese Silanolmoleküle mit freien Hydroxylgruppen der Substrat- oder Bauteiloberfläche und das Siloxangerüst kann über eine Kondensationsreaktion ausgebildet werden; dieser Vorgang wird auch als Chemisorption bezeichnet. Die Moleküle sind nach dieser Reaktion an der Oberfläche immobilisiert. Aufgrund der Tatsache, dass jedes Molekül mehrere reaktive Gruppen trägt, wird die Siloxanbindung nicht nur mit Molekülen auf der Substrat- oder Bauteiloberfläche geknüpft, sondern auch horizontal zwischen benachbarten Molekülen. Diese horizontale Polymerisation hat eine bedeutende Rolle in der zwei-dimensionalen Anordnung eines organosilanischen "Self-Assembled Monolayers" und generiert mit den Van-der Waals-Kräften, den hydrophoben Wechselwirkungen und den elektrostatischen Wechselwirkungen die Stabilität einer solchen Beschichtung.

Wenn das Modifizierungsmittel ein Siloxan ist, so hat es sich bewährt, wenn das Modifizierungsmittel ein Siloxan ausgewählt aus der Gruppe von Alkylsiloxanen, Alkylalkoxysiloxanen, Arylsiloxanen und Arylalkoxysiloxanen sowie deren Mischungen, insbesondere Alkylsiloxanen und Alkylalkoxysiloxanen sowie deren Mischungen, wobei das Siloxan ein Alkylalkoxysiloxan ist.

In diesem Zusammenhang kann es vorgesehen sein, dass das Siloxan ausgewählt ist aus $C_1$- bis $C_{20}$-Alkylsiloxanen, insbesondere $C_1$- bis $C_{15}$-Alkylsiloxanen, vorzugsweise $C_1$- bis $C_{12}$-Alkylsiloxanen, Gleichfalls ist es möglich, dass das Siloxan ausgewählt ist aus $C_1$- bis $C_{20}$-Alkylalkoxysiloxanen, insbesondere $C_1$- bis $C_{15}$-Alkylalkoxysiloxanen,

vorzugsweise $C_1$- bis $C_{12}$-Alkylalkoxysiloxanen, Darüber hinaus kann das Siloxan ausgewählt sein aus $C_6$- bis $C_{20}$-Arylsiloxanen, insbesondere $C_6$- bis $C_{18}$-Arylsiloxanen, vorzugsweise $C_6$- bis $C_{15}$-Arylsiloxanen, Weiterhin kann das Siloxan ausgewählt sein aus $C_6$- bis $C_{20}$-Arylalkoxysiloxanen, insbesondere $C_6$-bis $C_{18}$-Arylalkoxysiloxanen, vorzugsweise $C_6$- bis $C_{15}$-Arylalkoxysiloxanen.

Gleichfalls werden im Rahmen der vorliegenden Erfindung gute Ergebnisse erhalten, wenn das das Siloxan ein gewichtsmittleres Molekulargewicht $M_w$ im Bereich von 200 bis 10.000 g/mol, insbesondere 500 bis 8.000 g/mol, vorzugsweise 700 bis 5.000 g/mol, bevorzugt 1.000 bis 4.000 g/mol, besonders bevorzugt 1.500 bis 3.000 g/mol, aufweist. Im Rahmen der vorliegenden Erfindung werden somit bevorzugt niedermolekulare Siloxane eingesetzt, falls Siloxane verwendet werden.

Die im Rahmen der vorliegenden Erfindung eingesetzten Siloxane können darüber hinaus fluoriert, insbesondere teil- oder perfluoriert, sein, um die Hydrophobizität der erhaltenen Beschichtung weiter zu erhöhen.

Falls im Rahmen der vorliegenden Erfindung das Modifizierungsmittel ein Polysiloxan ist, so hat es sich gleichfalls bewährt, wenn das Modifizierungsmittel ein Polysiloxan ausgewählt aus der Gruppe von Alkylpolysiloxanen, Alkylalkoxypolysiloxanen, Arylpolysiloxanen und Arylalkoxypolysiloxanen sowie deren Mischungen, insbesondere Alkylpolysiloxanen und Alkylalkoxypolysiloxanen sowie deren Mischungen, vorzugsweise ein Alkylalkoxypolysiloxan ist.

In diesem Zusammenhang kann es vorgesehen sein, dass das Polysiloxan ausgewählt ist aus $C_1$- bis $C_{20}$-Alkylpolysiloxanen, insbesondere $C_1$- bis $C_{15}$- Alkylpolysiloxanen, vorzugsweise $C_1$- bis $C_{12}$- Alkylpolysiloxanen.

Weiterhin kann es vorgesehen sein, dass das Polysiloxan ausgewählt ist aus $C_1$-bis $C_{20}$-Alkylalkoxypolysiloxanen, insbesondere $C_1$- bis $C_{15}$-Alkylalkoxypolysiloxanen, vorzugsweise $C_1$- bis $C_{12}$-Alkylalkoxypolysiloxanen,

Gleichfalls ist es möglich, dass das Polysiloxan ausgewählt ist aus $C_6$- bis $C_{20}$-Arylpolysiloxanen, insbesondere $C_6$- bis $C_{18}$-Arylpolysiloxanen, vorzugsweise $C_6$- bis $C_{15}$-Arylpolysiloxanen.

Darüber hinaus kann es vorgesehen sein, dass das Polysiloxan ausgewählt ist aus $C_6$- bis $C_{20}$-Arylalkoxypolysiloxanen, insbesondere $C_6$- bis $C_{18}$-Aryl-alkoxypolysiloxanen, vorzugsweise $C_6$- bis $C_{15}$-Arylalkoxypolysiloxanen,

Darüber hinaus werden im Rahmen der vorliegenden Erfindung gleichfalls gute Ergebnisse erhalten, wenn das Polysiloxan ein gewichtsmittleres Molekulargewicht $M_w$ im Bereich von 3.000 bis 25.000 g/mol aufweist.

Auch im Hinblick auf die Verwendung von Polysiloxanen ist es möglich teil- oder perfluorierte Polysiloxane zu verwenden, um die Hydrophobizität der Beschichtung zu erhöhen.

Aufgrund des hohen Molekulargewichts der Polysiloxane ist im Rahmen der vorliegenden Erfindung die Verwendung von Polysiloxanen gegenüber einer Verwendung von niedermolekularen Verbindungen, insbesondere von Siloxanen und Silanen, vorzugsweise Silanen, weniger bevorzugt. Bei der Auswahl der Polysiloxane ist darauf zu achten, dass diese in Abhängigkeit von der zu beschichtenden Mikrostruktur in Schichtdicken auf die Substrat- oder Bauteiloberfläche aufgebracht werden können, welche die Mikrostrukturen weder verdeckt bzw. verstopft noch deren Konturschärfe abschwächt oder ihre Geometrie ändert. Besonders bevorzugt ist im Rahmen der vorliegenden Erfindung die Verwendung von Silanen, da mit Silanen besonders geringe Schichtdicken, insbesondere Monolagen, erzielt werden können.

Wenn im Rahmen der vorliegenden Erfindung ein Silan als Modifizierungsmittel eingesetzt wird, so kann ein Silan gemäß der allgemeinen Formel I

$$R^1_{4-(n+m)}SiR^2_mX_n \qquad (I)$$

mit

$R^1$ = Alkyl, insbesondere $C_1$- bis $C_{20}$-Alkyl, vorzugsweise $C_8$- bis $C_{18}$-Alkyl, bevorzugt $C_{10}$- bis $C_{16}$-Alkyl; Aryl, insbesondere $C_6$- bis $C_{20}$-Aryl, bevorzugt $C_6$- bis $C_{10}$-Aryl; Olefin, insbesondere terminales Olefin, vorzugsweise $C_2$- bis $C_{20}$-Olefin, bevorzugt $C_8$- bis $C_{18}$-Olefin, besonders bevorzugt $C_{10}$- bis $C_{16}$-Olefin; Fluoralkyl, insbesondere $C_1$- bis $C_{20}$- Fluoralkyl, vorzugsweise $C_8$- bis $C_{18}$-Fluoralkyl, bevorzugt $C_{10}$- bis $C_{16}$-Fluoralkyl, insbesondere aufweisend 1 bis 40 Fluoratome, vorzugsweise 5 bis 35 Fluoratome, bevorzugt 10 bis 30 Fluoratome; Fluoraryl, insbesondere $C_6$- bis $C_{20}$- Fluoraryl, bevorzugt $C_6$- bis $C_{10}$- Fluoraryl, insbesondere aufweisend 3 bis 20 Fluoratome, bevorzugt 5 bis 20 Fluoratome; Fluorolefin, insbesondere terminales Fluorolefin, vorzugsweise $C_2$- bis $C_{20}$-Fluorolefin, bevorzugt $C_8$- bis $C_{18}$-Fluorolefin, besonders bevorzugt $C_{10}$- bis $C_{16}$-Fluorolefin, insbesondere aufweisend 1 bis 30 Fluoratome, vorzugsweise 3 bis 25 Fluoratome, bevorzugt 5 bis 25 Fluoratome;

$R^2$ = Alkyl, insbesondere $C_1$- bis $C_3$-Alkyl, vorzugsweise Methyl;

X = Halogenid, insbesondere Chlorid und/oder Bromid, vorzugsweise Chlorid; Alkoxy, insbesondere $C_1$- bis $C_6$-Alkoxy, besonders bevorzugt $C_1$- bis $C_4$-Alkoxy, ganz besonders bevorzugt $C_1$- und/oder $C_2$-Alkoxy; und

n = 1 bis 3, insbesondere 3, und

m = 0 bis 2, insbesondere 0 oder 2, vorzugsweise 0,

eingesetzt werden.

Darüber hinaus kann im Rahmen des vorliegenden die Gruppe X auch durch weitere reaktive, insbesondere hydrolisierbare, chemische Gruppen gebildet werden.

Besonders gute Ergebnisse werden im Rahmen der vorliegenden Erfindung erhalten, wenn als Modifizierungsmittel ein Silan gemäß der allgemeinen Formel II

$$R^1_{4-(n+m)}SiR^2_mX_n \qquad (II)$$

mit

$R^1$ = Alkyl, insbesondere $C_1$- bis $C_{20}$-Alkyl, vorzugsweise $C_8$- bis $C_{18}$-Alkyl, bevorzugt $C_{10}$- bis $C_{16}$-Alkyl; Fluoralkyl, insbesondere $C_1$- bis $C_{20}$- Fluoralkyl, vorzugsweise $C_8$- bis $C_{18}$-Fluoralkyl, bevorzugt $C_{10}$- bis $C_{16}$-Fluoralkyl, insbesondere aufweisend 1 bis 40 Fluoratome, vorzugsweise 5 bis 35 Fluoratome, bevorzugt 10 bis 30 Fluoratome;

$R^2$ = Alkyl, insbesondere $C_1$- bis $C_3$-Alkyl, vorzugsweise Methyl;

X = Halogenid, insbesondere Chlorid; Alkoxy, insbesondere $C_1$- bis $C_6$-Alkoxy, besonders bevorzugt $C_1$- bis $C_4$-Alkoxy, ganz besonders bevorzugt $C_1$- und/oder $C_2$-Alkoxy; und

n = 1 bis 3, insbesondere 3 und

m = 0 bis 2, insbesondere 0 oder 2, vorzugsweise 0,

eingesetzt wird.

Besonders bevorzugt wird es in diesem Zusammenhang, wenn als Modifizierungsmittel ein Silan gemäß der allgemeinen Formel III

$$R_{4-n}SiX_n \qquad (III)$$

mit

R = Alkyl, insbesondere $C_1$- bis $C_{20}$-Alkyl, vorzugsweise $C_8$- bis $C_{18}$-Alkyl, bevorzugt $C_{10}$- bis $C_{16}$-Alkyl; Fluoralkyl, insbesondere $C_1$- bis $C_{20}$- Fluoralkyl, vorzugsweise $C_8$- bis $C_{18}$-Fluoralkyl, bevorzugt $C_{10}$- bis $C_{16}$-Fluoralkyl, insbesondere aufweisend 1 bis 40 Fluoratome, vorzugsweise 5 bis 35 Fluoratome, bevorzugt 10 bis 30 Fluoratome;

X = Alkoxy, bevorzugt $C_1$- bis $C_4$- Alkoxy, ganz besonders bevorzugt $C_1$- und/oder $C_2$-Alkoxy; und

n = 1 bis 4, insbesondere 3,

eingesetzt wird.

Gemäß einer bevorzugten Ausführungsform der vorliegenden Erfindung werden fluorierte Silane als Modifizierungsmittel verwendet. In diesem Zusammenhang werden gute Ergebnisse erhalten, wenn als Modifizierungsmittel ein Silan gemäß der allgemeinen Formel IV

$$R^1_{4-(n+m)}SiR^2_mX_n \qquad (IV)$$

mit

$R^1$ = Fluoralkyl, insbesondere $C_1$- bis $C_{20}$- Fluoralkyl, vorzugsweise $C_8$- bis $C_{18}$-Fluoralkyl, bevorzugt $C_{10}$- bis $C_{16}$-Fluoralkyl, insbesondere aufweisend 1 bis 40 Fluoratome, vorzugsweise 5 bis 35 Fluoratome, bevorzugt 10 bis 30 Fluoratome; Fluoraryl, insbesondere $C_6$- bis $C_{20}$- Fluoraryl, bevorzugt $C_6$- bis $C_{10}$- Fluoraryl, insbesondere aufweisend 3 bis 20 Fluoratome, bevorzugt 5 bis 20 Fluoratome; Fluorolefin, insbesondere terminales Fluorolefin, vorzugsweise $C_2$- bis $C_{20}$-Fluorolefin, bevorzugt $C_8$- bis $C_{18}$-Fluorolefin, besonders bevorzugt $C_{10}$- bis $C_{16}$-Fluorolefin, insbesondere aufweisend 1 bis 30 Fluoratome, vorzugsweise 3 bis 25 Fluoratome, bevorzugt 5 bis 25

Fluoratome;

$R^2$ = Alkyl, insbesondere $C_1$- bis $C_3$-Alkyl, vorzugsweise Methyl;

X = Halogenid, insbesondere Chlorid und/oder Bromid, vorzugsweise Chlorid; Alkoxy, insbesondere $C_1$- bis $C_6$-Alkoxy, besonders bevorzugt $C_1$- bis $C_4$-Alkoxy, ganz besonders bevorzugt $C_1$- und/oder $C_2$-Alkoxy; und

n = 1 bis 3, insbesondere 3, und

m = 0 bis 2, insbesondere 0 oder 2, vorzugsweise 0,

eingesetzt wird.

In diesem Zusammenhang hat es bewährt, wenn als Modifizierungsmittel ein Silan gemäß der allgemeinen Formel V

$$R_{4-n}SiX_n \qquad (V)$$

mit

R = Fluoralkyl, insbesondere $C_1$- bis $C_{20}$- Fluoralkyl, vorzugsweise $C_8$- bis $C_{18}$-Fluoralkyl, bevorzugt $C_{10}$- bis $C_{16}$-Fluoralkyl, insbesondere aufweisend 1 bis 40 Fluoratome, vorzugsweise 5 bis 35 Fluoratome, bevorzugt 10 bis 30 Fluoratome;

X = Alkoxy, bevorzugt $C_1$- bis $C_4$- Alkoxy, ganz besonders bevorzugt $C_1$- und/oder $C_2$-Alkoxy; und

n = 1 bis 4, insbesondere 3,

eingesetzt wird.

Mit Fluorsilanen, insbesondere mit Fluoralkylsilanen, welche Alkoxygruppen aufweisen, lassen sich besonders dauerhafte Beschichtung auf mikrostrukturierten Bauteilen bzw. Substraten erzielen, welche eine hervorragende Langzeitperformance aufweisen. Die Beschichtungen weisen eine hervorragende Hydrophobizität auf und bleiben auch bei Hochdruckanwendungen dauerhaft an das Substrat oder Bauteil gebunden.

Gemäß einer weiteren, besonders bevorzugten Ausführungsform der vorliegenden Erfindung wird als Modifizierungsmittel ein Silan gemäß der allgemeinen Formel VI

$$R^1_{4-(n+m)}SiR^2_mX_n \qquad (VI)$$

mit

$R^1$ = Alkyl, insbesondere $C_1$- bis $C_{20}$-Alkyl, vorzugsweise $C_8$- bis $C_{18}$-Alkyl, bevorzugt $C_{10}$- bis $C_{16}$-Alkyl; Aryl, insbesondere $C_6$- bis $C_{20}$-Aryl, bevorzugt $C_6$- bis $C_{10}$-Aryl; Olefin, insbesondere terminales Olefin, vorzugsweise $C_2$- bis $C_{20}$-Olefin, bevorzugt $C_8$- bis $C_{18}$-Olefin, besonders bevorzugt $C_{10}$- bis $C_{16}$-Olefin;

$R^2$ = Alkyl, insbesondere $C_1$- bis $C_3$-Alkyl, vorzugsweise Methyl;

X = Halogenid, insbesondere Chlorid und/oder Bromid, vorzugsweise Chlorid; Alkoxy, insbesondere $C_1$- bis $C_6$-Alkoxy, besonders bevorzugt $C_1$- bis $C_4$-Alkoxy, ganz besonders bevorzugt $C_1$- und/oder $C_2$-Alkoxy; und

n = 1 bis 3, insbesondere 3, und

m = 0 bis 2, insbesondere 0 oder 2, vorzugsweise 0,

eingesetzt.

In diesem Zusammenhang werden besonders gute Ergebnisse erhalten, wenn als Modifizierungsmittel ein Silan gemäß der allgemeinen Formel VII

$$R_{4-n}SiX_n \qquad (VII)$$

mit

R = Alkyl, insbesondere $C_1$- bis $C_{20}$-Alkyl, vorzugsweise $C_8$- bis $C_{18}$-Alkyl, bevorzugt $C_{10}$- bis $C_{16}$-Alkyl;

X = Alkoxy, bevorzugt $C_1$- bis $C_4$- Alkoxy, ganz besonders bevorzugt $C_1$- und/oder $C_2$-Alkoxy; und

n = 1 bis 4, insbesondere 3,

eingesetzt wird.

Gleichfalls hat es sich bewährt, wenn das Silan drei reaktive chemische Funktionen und/oder Gruppen, insbesondere drei hydrolysierbare chemische Funktionen und/oder Gruppen, aufweist, vorzugsweise ein Trialkoxysilan ist.

Darüber hinaus werden im Rahmen der vorliegenden Erfindung gute Ergebnisse erhalten, wenn als Modifizierungsmittel ein Silan mit organischem $C_1$- bis $C_{20}$-Resten, insbesondere $C_8$- bis $C_{18}$-Resten, vorzugsweise $C_{10}$- bis $C_{16}$-Resten, eingesetzt wird. In diesem Zusammenhang hat es sich bewährt, wenn die organischen Reste nicht hydrolysierbar sind und vorzugsweise ausgewählt sind aus Alkyl-, Aryl- und Olefinresten, welche teil- oder perfluoriert sein können.

Gute Ergebnisse werden im Rahmen der vorliegenden Erfindung erhalten, wenn das Silan ausgewählt ist aus Fluoralkyltrialkoxysilanen, insbesondere wobei das Silan 1H,1H,2H,2H-Tridecafluorooctyltriethoxysilan ist.

Darüber hinaus werden besonders gute Ergebnisse erhalten, wenn das Silan ein Alkyltrialkoxysilan, insbesondere ausgewählt aus Gruppe von $C_{12}$-Alkyltrilalkoxysilanen, $C_{14}$-Alkyltrilalkoxysilanen und $C_{16}$-Alkyltrilalkoxysilanen sowie deren Mischungen.

Wie die Anmelderin überraschenderweise herausgefunden hat, lassen sich auf Basis von Organosilanen, insbesondere Alkylsilanen vorzugsweise mit $C_{10}$- bis $C_{16}$-Alkylgruppen, besonders bevorzugt mit $C_{12}$-, $C_{14}$- oder $C_{16}$-Alkylgruppen, Beschichtungen erzielen, welche in ihren Eigenschaften mit durch fluorierte Verbindungen, insbesondere Fluoralkysilanen, erhaltenen Beschichtungen vergleichbar sind. Die erfindungsgemäßen Beschichtungen auf Basis von Alkylsilanen lassen sich in Hochdruckanwendungen nicht von der Oberfläche des Substrats oder Bauteil entfernen und zeigen darüber hinaus in Belastungstests, sogenannten Provokationstests, hervorragende Ergebnisse, insbesondere auch im Hinblick auf die Langzeitperformance.

Was die Zusammensetzung und die Applikationsform des Modifizierungsreagenzes anbelangt, so kann hier grundsätzlich jede geeignete Möglichkeit verwendet werden. Es hat sich jedoch bewährt, wenn das Modifizierungsreagenz im Rahmen der vorliegenden Erfindung in Form einer Lösung und/oder Dispersion eingesetzt wird.

Insbesondere bei Verwendung flüssiger Dispersionen bzw. Lösungen lassen sich Modifizierungsreagenzien mit einem sehr geringen Gehalt an Modifizierungsmittel einstellen, welche geeignet sind, mikrostrukturierte Bauteile mit Monolagen zu beschichten.

Im Rahmen der vorliegenden Erfindung ist es im Allgemeinen vorgesehen, dass als Löse- und/oder Dispersionsmittel Wasser und/oder organische Lösemittel eingesetzt werden.

Was nun die konkrete Auswahl des Löse- bzw. Dispersionsmittel anbelangt, so können als Löse- und/oder Dispersionsmittel Wasser und/oder polare organische Lösemittel ausgewählt werden. In diesem Zusammenhang hat es sich bewährt, wenn das polare organische Lösemittel ausgewählt ist aus der Gruppe von primären und sekundären Alkoholen, Ketonen, Ethern, Aminen, Amiden, Estern, Sulfoxiden sowie deren Mischungen, insbesondere Methanol, Ethanol, Isopropanol, Dimethylether, Diethylether, Essigsäureethylether, THF, DMF, DMSO und deren Mischungen, vorzugsweise Ethanol und Isopropanol sowie deren Mischungen. Im Rahmen der vorliegenden Erfindung werden bevorzugt Mischungen aus Wasser und Alkoholen, insbesondere Ethanol und/oder Isopropanol, eingesetzt.

Gleichfalls kann es im Rahmen der vorliegenden Erfindung jedoch auch vorgesehen sein, dass als Löse- und/oder Dispersionsmittel unpolare organische Lösemittel, insbesondere Toluol, Tetrachlormethan, Chloroform, Alkane und deren Mischungen, insbesondere Toluol, Tetrachlormethan, $C_5$- bis $C_9$-Alkane, insbesondere Pentan, Hexan, Heptan, und/oder Oktan, sowie deren Mischungen, eingesetzt werden. Die Verwendung unpolarer organischer Lösemittel erfolgt vorrangig dann, wenn unter wasserfreien Bedingungen gearbeitet werden soll.

Das Modifizierungsmittel kann in jeder Konzentration die eingesetzt werden, die geeignet ist, besonders dünne Schichten, vorzugsweise Monolagen, auf der Substrat- oder Bauteiloberfläche zu erzeugen. Es hat sich allerdings im Rahmen der vorliegenden Erfindung bewährt, wenn das Modifizierungsreagenz das Modifizierungsmittel in Mengen 0,003 bis 2 mol/l, insbesondere 0,006 bis 0,5 mol/l, vorzugsweise 0,01 bis 0,1 mol/l, bevorzugt 0,02 bis 0,05 mol/l, bezogen auf das Modifizierungsreagenz, enthält.

Was nun die Dauer anbelangt, mit welcher das Substrat oder Bauteil, insbesondere die Substrat- oder Bauteiloberfläche, mit dem Modifizierungsreagenz in Kontakt gebracht wird, so kann diese in Abhängigkeit von den weiteren Bedingungen in weiten Bereichen variieren. Es hat sich jedoch im Rahmen der vorliegenden Erfindung gezeigt, dass sehr gute Ergebnisse erhalten werden, wenn das Substrat oder Bauteil für einen Zeitraum von 5 Minuten bis 20 Stunden, insbesondere 30 Minuten bis 15 Stunden, vorzugsweise 1 bis 10 Stunden, bevorzugt 2 bis 8 Stunden, besonders

bevorzugt 4 bis 7 Stunden, mit dem Modifizierungsreagenz in Kontakt gebracht wird.

Im Hinblick auf die Temperaturen, bei welchen das Substrat oder Bauteil mit dem Modifizierungsreagenz in Kontakt gebracht wird, so gilt auch hier, dass prinzipiell jede Temperatur geeignet ist, welche eine besonders dünnschichtige homogene Beschichtung des Substrates oder Bauteils ermöglicht. Es hat sich jedoch herausgestellt, dass mit den im Rahmen der vorliegenden Erfindung eingesetzten Modifizierungsmitteln bzw. Modifizierungsreagenzien gute Ergebnisse erhalten werden, wenn das Substrat oder Bauteil bei einer Temperatur im Bereich von 10 bis 50 °C, insbesondere 15 bis 40 °C, vorzugsweise 20 bis 30 °C, mit dem Modifizierungsreagenz in Kontakt gebracht wird.

Im Rahmen der vorliegenden Erfindung hat es insbesondere bewährt, wenn das Substrat oder Bauteil während des Inkontaktbringens mit dem Modifizierungsmittel zumindest zeitweise, insbesondere in vorzugsweise festgelegten Intervallen, mit Ultraschall behandelt wird. Durch eine Ultraschallbehandlung wird ein Austausch des Modifizierungsmittels in den Kapillaren und Kanälen des mikrostrukturierten Bauteils ermöglicht, wodurch besonders gleichmäßige Beschichtungen zugänglich werden.

Gemäß einer bevorzugten Ausführungsform der vorliegenden Erfindung ist es vorgesehen, dass das Modifizierungsmittel nach dem Inkontaktbringen mit dem Substrat oder Bauteil, insbesondere nach dem Aufbringen auf das Substrat oder Bauteil, getrocknet und/oder ausgehärtet wird. Durch das Trocknen bzw. Aushärten bzw. Vernetzen des Modifizierungsmittels wird eine zumindest im Wesentlichen geschlossene Schicht, vorzugsweise in Form einer Monolage, auf dem Substrat oder Bauteil erhalten, welche dauerhaft und fest an das Substrat oder Bauteil gebunden ist.

Die Temperaturbereiche, in welchen das Modifizierungsmittel getrocknet bzw. ausgehärtet oder vernetzt wird, können in Abhängigkeit des Modifizierungsmittels in weiten Bereichen variieren. Es werden jedoch, insbesondere bei Verwendung von Silanen bzw. Siloxanen als Modifizierungsmittel, besonders gute Ergebnisse erzielt, wenn das Modifizierungsmittel bei Temperaturen im Bereich von 20 bis 250 °C, insbesondere 30 bis 220 °C, vorzugsweise 50 bis 200 °C, bevorzugt 80 bis 180 °C, besonders bevorzugt 100 bis 150 °C, ganz besonders bevorzugt 110 bis 140 °C, getrocknet und/oder ausgehärtet wird. Bevorzugt wird das Modifizierungsmittel bzw. die mit dem Modifizierungsmittel erhaltene Beschichtung in den vorgenannten Temperaturbereichen getempert, so dass einerseits eine Vernetzung innerhalb der Schicht stattfindet und andererseits die Schicht über chemische Bindungen, insbesondere kovalente Bindungen, an die Substrat- oder Bauteiloberfläche gebunden wird.

Was nun die Zeit anbelangt, für welche das Modifizierungsmittel getrocknet und/oder ausgehärtet wird, so ist diese gleichfalls stark von den Eigenschaften des jeweilig verwendeten Modifizierungsmittels abhängig. Es hat sich im Rahmen der vorliegenden Erfindung jedoch als vorteilhaft erwiesen, wenn das Modifizierungsmittel für einen Zeitraum von 0,1 bis 10 Stunden, insbesondere 0,2 bis 8 Stunden, vorzugsweise 0,5 bis 5 Stunden, bevorzugt 0,75 bis 3 Stunden, besonders bevorzugt 1 bis 2 Stunden, getrocknet und/oder ausgehärtet wird.

Weiterhin kann es im Rahmen der vorliegenden Erfindung vorgesehen sein, das überschüssiges Modifizierungsreagenz vor oder nach dem Verfahrensschritt des Trocknens und/oder Aushärtens entfernt wird. Um eine spätere dauerhafte Funktionsfähigkeit des beschichteten Substrats bzw. des oberflächenmodifizierten Substrats oder Bauteils zu gewährleisten, hat es sich bewährt, wenn überschüssiges Modifizierungsreagenz bzw. nicht an die Substrat- oder Bauteiloberfläche gebundenes Modifizierungsmittel entfernt wird. Dies kann entweder vor dem Trocknen oder Aushärten geschehen, beispielsweise durch mechanische Entfernung, insbesondere über Tücher oder Vliese oder berührungslos, oder nach dem Trocknen oder Aushärten, beispielsweise durch Abspülen mit Wasser oder einem Lösemittel. Wenn die beschichtete mikrostrukturierte Oberfläche mit einem Lösemittel von überschüssigem Modifizierungsreagenz bzw. Modifizierungsmittel befreit wird, so wird üblicherweise mindestens ein Alkohol, insbesondere Isopropanol und/oder Ethanol, vorzugsweise Isopropanol, verwendet.

Besonders gute Ergebnisse werden in diesem Zusammenhang erhalten, wenn überschüssiges Modifizierungsreagenz vor dem Trocknen und/oder Aushärten von der Substrat- oder Bauteiloberfläche, insbesondere von der Mikrostruktur, entfernt wird. Auf diese Weise kann ein Verstopfen oder eine Geometrieänderung der Mikrostruktur effektiv verhindert werden. Insbesondere für den Fall, dass das mikrostrukturierte System ein Düsensystem darstellt, lassen sich große Steigerungen in der Homogenität und Reproduzierbarkeit der Beschichtung erzielen. Vorzugsweise wird überschüssiges Modifizierungsreagenz in diesem Zusammenhang mechanisch entfernt, insbesondere berührungslos. Unter einer berührungslosen Entfernung des überschüssigen Modifizierungsreagenz ist dabei im Rahmen der vorliegenden Erfindung zu verstehen, dass das Modifizierungsreagenz von der Oberfläche des Substrats oder Bauteils, insbesondere im Bereich der Mikrostruktur, entfernt wird, ohne dass Teile der Reinigungsvorrichtung mit der zu reinigenden Oberfläche in Kontakt kommen. Vorzugsweise erfolgt die Entfernung des überschüssigen Modifizierungsreagenzes mittels eines druckbeaufschlagten Gases, insbesondere mittels Druckluft, oder durch Einwirkung von Rotationskräften, beispielsweise in einem Spin Rinse Dryer.

Im Rahmen der vorliegenden Erfindung wird es bevorzugt, wenn die Entfernung überschüssigen Modifizierungsreagenzes mittels eines Spin Rinse Dryers, das heißt unter Einwirkung von Rotationskräften, durchgeführt wird. Besonders gute Ergebnisse werden in diesem Zusammenhang erhalten, wenn die auf das Bauteil einwirkenden Rotationskräfte im Bereich von 5 bis 12 g, insbesondere 6 bis 11 g, vorzugsweise 8 bis 10 g, liegen, wobei g für die Erdbeschleunigung von 9,81 $ms^{-2}$ steht.

Die Entfernung überschüssigen Modifizierungsreagenzes mittels Rotationskräften, insbesondere mittels eines Spin Rinse Dryers, hat den Vorteil, dass einerseits das Modifizierungsreagenz zuverlässig auch aus feinsten Kapillarstrukturen des mikrostrukturierten Bauteils entfernt wird und dass andererseits darüber hinaus bei der Entfernung des Modifizierungsreagenzes keine Verdunstungseffekte auftreten, welche beispielsweise zum Ausfallen bzw. zur Anlagerung weiteren Modifizierungsmitteln aufgrund von Konzentrationseffekten führen.

Die Entfernung des überschüssigen Modifizierungsreagenzes mittels Rotationskräften ist in diesem Zusammenhang bei Raumtemperatur möglich, insbesondere im Temperaturbereich von 15 bis 30°C. Weiterhin kann das überschüssige Modifizierungsmittel mittels Rotationskräften üblicherweise an der normalen Umgebungsluft entfernt werden. Die Entfernung von überschüssigem Modifizierungsreagenz mittels Rotationskräften ist darüber hinaus auch im großindustriellen Maßstab einfach durchzuführen.

Gemäß einer bevorzugten Ausführungsform der vorliegenden Erfindung wird vor dem Inkontakbringen des Substrats oder Bauteils mit dem Modfizierungsreagenz die Oberfläche des Substrats oder Bauteils aktiviert. In diesem Zusammenhang hat es sich als vorteilhaft erwiesen, wenn sämtliche Materialien des Bauteils in einem Arbeitsgang, d. h. in einem Verfahrensschritt, aktiviert werden.

Unter einer Aktivierung der Substrat- oder Bauteiloberfläche ist dabei im Rahmen der vorliegenden Erfindung zu verstehen, dass die Substrat- oder Bauteiloberfläche chemisch oder physikalisch auf die nachfolgende Oberflächenmodifizierung vorbereitet wird. Dies kann beispielsweise durch Erzeugung funktioneller reaktiver Gruppen auf der Substrat- oder Bauteiloberfläche erfolgen, welche nachfolgend mit dem Modifizierungsreagenz bzw. dem Modifizierungsmittel Bindungen, insbesondere kovalente chemische Bindungen, eingehen können. Eine vorangehende Aktivierung erhöht die Qualität der nachfolgenden Oberflächenmodifizierung bzw. Beschichtungen in der Regel beträchtlich, insbesondere wird die Langzeitperformance der oberflächenmodifizierten Substrate oder Bauteile deutlich verbessert, da durch Aktivierung eine deutlich bessere Bindung des Modifizierungsmittels an die Substrat- oder Bauteiloberfläche gewährleistet wird.

Gemäß einer bevorzugten Ausführungsform des erfindungsgemäßen Verfahrens ist das erfindungsgemäße Verfahren ein Verfahren zur Oberflächenmodifizierung, insbesondere Hydrophobierung, von mikrostrukturierten Bauteilen, insbesondere für Hochdruckanwendungen, wie zuvor beschrieben, wobei

(a) in einem ersten Verfahrensschritt die Oberfläche eines mikrostrukturiertes Bauteiles aktiviert wird (optional nach einem vorherigen zusätzlichen Reinigungsschritt),

(b) in einem nachfolgenden Verfahrensschritt das mikrostrukturierte Bauteil mit einem Modifizierungsreagenz enthaltend mindestens ein Modifizierungsmittel in Kontakt gebracht, insbesondere behandelt, wird und

(c) in einem wiederum nachfolgenden Verfahrensschritt das Modifizierungsmittel getrocknet und/oder ausgehärtet und/oder vernetzt wird.

Was den Verfahrensschritt der Aktivierung der Substrat- oder Bauteiloberfläche anbelangt, so kann dieser auf vielfältige Weise geschehen. Üblicherweise wird das Substrat oder Bauteil jedoch chemisch und/oder physikalisch, vorzugsweise chemisch, aktiviert. Bei einer physikalischen Aktivierung wird beispielsweise die Substrat- oder Bauteiloberfläche mit Teilchen oder elektromagnetischen Strahlen beschossen, wie beispielsweise beim Sputtern oder der Plasma- bzw. Koronabehandlung. Durch die physikalische Behandlung wird die Oberfläche der Substrate oder Bauteile zum einen gereinigt und (vorzugsweise zeitgleich) werden zum anderen reaktive, insbesondere polare reaktive chemische Gruppen auf der Oberfläche des Substrats oder Bauteils gebildet, welche bei der nachfolgenden Oberflächenmodifizierung eine Anbindung des Modifizierungsmittels, insbesondere von Silanen, ermöglichen. Bei der chemischen Aktivierung wird die Substrat- oder Bauteiloberfläche durch Inkontaktbringen, insbesondere Behandlung oder Umsetzung, mit einem chemischen Reagenz chemisch derart verändert, dass reaktive chemische Gruppen, insbesondere polare reaktive Gruppen, an der Oberfläche des Substrates oder Bauteils gebildet werden.

Im Rahmen der vorliegenden Erfindung hat es sich bewährt, wenn das Substrat oder Bauteil durch Einwirkung eines Aktivierungsreagenzes, insbesondere einer Aktivierungslösung, aktiviert wird. Das Aktivierungsreagenz bzw. die Aktivierungslösung enthält chemische Substanzen, welche mit der Substrat- oder Bauteiloberfläche in Wechselwirkung treten können und diese beispielsweise durch chemische Umsetzung aktivieren. Eine Aktivierung beispielsweise von Glassubstraten bzw. von Siliziumsubstraten, welche eine native Oxidschicht besitzen, erfolgt üblicherweise dergestalt, dass an der Oberfläche des Glases bzw. der Siliziumdioxidschicht Silanolgruppen, d. h. freie Hydroxifunktionen, gebildet werden, welche nachfolgend mit dem Modifizierungsmittel reagieren können.

Im Allgemeinen wird das Substrat oder Bauteil unter sauren und/oder basischen Bedingungen aktiviert. Bei den Säuren bzw. Laugen handelt es sich üblicherweise um Brönsted-Säuren bzw. -Basen.

Im Rahmen der vorliegenden Erfindung hat es sich jedoch als vorteilhaft erwiesen, wenn das Substrat oder Bauteil unter oxidierenden Bedingungen, insbesondere unter sauren und/oder basischen oxidierenden Bedingungen,

vorzugsweise unter sauren oxidierenden, Bedingungen, aktiviert wird. Unter oxidierenden Bedingungen, insbesondere sauren bis leicht basischen oxidierenden Bedingungen, wird nicht nur eine hervorragende Aktivierung der Substrat- oder Bauteiloberfläche erreicht. Vorzugsweise werden dabei die optischen Eigenschaften der Glas- oder Siliziumsubstrate erhalten, was eine einfache Qualitätskontrolle der mikrostrukturierten Bauteile über optische Methoden ermöglicht. Durch die Aktivierung unter oxidierenden Bedingungen wird darüber hinaus die Silanolgruppendichte auf dem Substrat oder Bauteil deutlich erhöht.

Was nun das Aktivierungsreagenz anbelangt, welches im Rahmen der vorliegenden Erfindung verwendet wird, so ist hierfür prinzipiell eine Vielzahl von Reagenzien geeignet. Besonders gute Ergebnisse werden jedoch erhalten, wenn das Aktivierungsreagenz ausgewählt ist aus der Gruppe von Säuren und Laugen, insbesondere Alkalilaugen, Lösungen von Tetramethylammoniumhydroxid, Mineralsäuren, wie beispielsweise Schwefelsäure, Salzsäure oder Salpetersäure, halogenierten organischen Säuren, Piranha-Lösung, SC-1-Lösungen ("SC-1" = Standard **C**lean 1 gemäß dem RCA-Verfahren; "RCA" = ***R**adio **C**orporation of **A**merica*) sowie deren Mischungen, vorzugsweise SC-1-Lösungen. Bzgl. weiterer Informationen zum RCA-Verfahren wird verwiesen auf Kern, W., Cleaning solutions based on hydrogen peroxide for use in silicon semiconductor technology. RCA review, 1970. 31: p. 187-206.

Gemäß einer Ausführungsform der vorliegenden Erfindung kann es vorgesehen sein, dass das Aktivierungsreagenz Wasser, wässrige Ammoniaklösung und wässrige Wasserstoffperoxidlösung in einem volumenbezogenen Verhältnis im Bereich von 10 : 1 : 1 bis 5 : 2 : 2, bevorzugt 8 : 1 : 1 bis 5 : 2 : 1, besonders bevorzugt 5:1 : 1, aufweist. Diese Lösungen werden auch als SC-1-Lösungen bezeichnet und wurden für das RCA-Verfahren zur Reinigung von Siliziumwafern in der Halbleitertechnik entwickelt.

In diesem Zusammenhang werden besonders gute Ergebnisse erhalten, wenn die wässrige Ammoniaklösung 5 bis 30 Gew.-% $NH_3$, besonders bevorzugt 25 Gew.-% $NH_3$, bezogen auf die Ammoniaklösung, aufweist. Gleichfalls kann es vorgesehen sein, dass die wässrige Wasserstoffperoxidlösung 10 bis 40 Gew.-% $H_2O_2$, bevorzugt 30 Gew.-% $H_2O_2$, bezogen auf die wässrige Wasserstoffperoxidlösung, aufweist. Aktivierungsreagenzien der vorstehenden Art eignen sich in hervorragender Weise zur Aktivierung von Glas und Siliziumoberflächen unter milden, leicht alkalischen Bedingungen, wobei nahezu keine schwierig zu handhabenden Abfallstoffe entstehen.

Gemäß einer weiteren Ausführungsform der vorliegenden Erfindung kann es vorgesehen sein, dass die Aktivierungslösung Schwefelsäure und wässrige Wasserstoffperoxidlösung in einem Volumenverhältnis von 20 : 1 bis 1 : 1, insbesondere 10 : 1 bis 1 : 1, vorzugsweise 5 : 1 bis 1 : 1, bevorzugt 3 : 1 bis 1 : 1, besonders bevorzugt 7 : 3, aufweist. Diese Aktivierungslösungen werden üblicherweise als Piranha-Lösungen bzw. Carosche Säure bezeichnet.

In diesem Zusammenhang kann es vorgesehen sein, dass die Schwefelsäure konzentriert ist, insbesondere einen Gehalt von 99,0 bis 99,9 Gew.-% $H_2SO_4$, bezogen auf die Schwefelsäure, aufweist. Gleichfalls kann es vorgesehen sein, dass die wässrige Wasserstoffperoxidlösung 10 bis 40 Gew.-% $H_2O_2$, besonders bevorzugt 30 Gew.-% $H_2O_2$, bezogen auf die wässrige Wasserstoffperoxidlösung, aufweist.

Darüber hinaus kann es gleichfalls vorgesehen sein, dass das Aktivierungsreagenz eine Alkalilauge, insbesondere Natronlauge, ist. In diesem Zusammenhang werden besonders gute Ergebnisse erhalten, wenn das Aktivierungsreagenz mindestens ein Alkalimetallhydroxid, insbesondere Natrium- und/oder Kaliumhydroxid, vorzugsweise Natriumhydroxid, in Mengen von 5 bis 25 Gew.-%, bevorzugt 20 Gew.-%, bezogen auf das Aktivierungsreagenz, aufweist. Bei den vorgenannten basischen Aktivierungsreagenzien lässt sich gleichfalls eine hervorragende Aktivierung der Substrat- oder Bauteiloberflächen erzielen. Jedoch wird in einigen Fällen insbesondere bei Verwendung stark alkalischer Aktivierungsreagenzien eine Verfärbung von Glas- und/oder Siliziumsubstraten beobachtet, was die optische Kontrolle der Bauteile erschwert, weshalb diese Methode im Rahmen der vorliegenden Erfindung weniger bevorzugt ist.

Was nun die Dauer anbelangt, über welchen die Substrat- oder Bauteiloberfläche mit dem Aktivierungsreagenz behandelt wird, so kann diese in Abhängigkeit vom jeweils verwendeten Aktivierungsreagenz in weiten Bereichen variieren. Es hat sich jedoch im Rahmen der vorliegenden Erfindung bewährt, wenn das Substrat oder Bauteil für einen Zeitraum von 0,1 bis 10 Stunden, insbesondere 0,5 bis 8 Stunden, vorzugsweise 1 bis 5 Stunden, mit dem Aktivierungsreagenz behandelt wird. Eine Behandlung der Substrat- oder Bauteiloberfläche mit dem Aktivierungsreagenz in den vorgenannten Zeiträumen erlaubt eine vollständige Aktivierung der Substrat- oder Bauteiloberfläche, wobei jedoch kein nennenswerter Abtrag bzw. keine nennenswerte Erosion der Substrat- oder Bauteiloberfläche erfolgt.

Was nun die Temperaturen anbelangt, bei welchen das Substrat oder Bauteil mit dem Aktivierungsreagenz behandelt wird, so können diese auch wiederum in Abhängigkeit vom jeweiligen Aktivierungsreagenz in weiten Bereichen variieren. Es hat sich jedoch als vorteilhaft erwiesen, wenn das Substrat oder Bauteil bei Temperaturen im Bereich von 20 bis 100 °C, insbesondere 30 bis 90 °C, vorzugsweise 40 bis 85 °C, bevorzugt 50 bis 80°C, besonders bevorzugt 60 bis 75 °C, mit dem Aktivierungsreagenz behandelt wird. Eine leichte Erhöhung der Temperatur führt zu einer Steigerung der Aktivierungsgeschwindigkeit, wodurch selbst bei Verwendung milder Aktivierungsreagenzien eine sehr gute und gleichmäßige Aktivierung der Substrat- oder Bauteiloberfläche erreicht wird.

Im Rahmen der vorliegenden Erfindung wird es darüber hinaus bevorzugt, wenn das aktivierte Substrat oder Bauteil, insbesondere wenn es sich um ein Substrat oder Bauteil auf Basis von Glas bzw. elementarem Silizium handelt, nach Behandlung mit dem Aktivierungsreagenz mit Wasser gewaschen und anschließend unter Wasser bis zum Inkontaktbringen mit dem Modifizierungsreagenz gelagert wird. Darüber hinaus werden besonders gute Ergebnisse erhalten, wenn während der Aktivierung der Substrat- oder Bauteiloberfläche mit dem Aktivierungsreagenz die Aktivierungslösung zumindest intervallweise mit Ultraschall behandelt wird. Durch eine Ultraschallbehandlung wird eine besonders gleichmäßige Durchmischung der Aktivierungslösung erzielt und insbesondere auch in eventuell vorhandenen Kapillaren und feinen Kanälen im Inneren des Substrats oder Bauteil eine Durchmischung des Aktivierungsreagenzes und ein Austausch des Aktivierungsreagenzes ermöglicht.

Üblicherweise wird im Rahmen der vorliegenden Erfindung das Substrat oder Bauteil vor der Aktivierung und/oder Oberflächenmodifizierung gereinigt, insbesondere entfettet und/oder von Partikeln befreit. Eine Reinigung, insbesondere Entfettung, der Substrat- oder Bauteiloberfläche hat sich insbesondere dann bewährt, wenn eine besonders gleichmäßige Beschichtung erzielt werden soll. So werden beispielsweise Fettrückstände nicht immer vollständig durch die zuvor beschriebenen bevorzugt eingesetzten Aktivierungsreagenzien von der Substrat- oder Bauteiloberfläche entfernt. Das heißt die von dem Fett bzw. den Fettsäuren bedeckten Teile des Substrats oder Bauteil sind somit der Aktivierung nicht oder nur unzureichend zugänglich, weshalb die Oberflächenmodifizierung an diesen Stellen nicht oder nur in schlechter Qualität erfolgt. Aus diesem Grund sollte vor der Behandlung der Substrat- oder Bauteiloberfläche mit dem Aktivierungsreagenz eine Reinigung bzw. Entfettung der Substratoberfläche, vorzugsweise mit organischen Lösemitteln, vorgenommen werden.

In diesem Zusammenhang hat es sich bewährt, wenn das Substrat oder Bauteil durch Behandlung mit einem insbesondere flüchtigen organischen Lösemittel, insbesondere einem Alkohol, vorzugsweise Ethanol oder Isopropanol, oder unpolaren aprotischen Lösemittel, gereinigt wird. In diesem Zusammenhang haben sich insbesondere Alkane, wie beispielsweise Pentan, Hexan, Heptan oder Octan bewährt, um insbesondere auch unpolare Stoffe von der Oberfläche des Substrates oder Bauteil zu entfernen.

Gemäß einer besonderen und bevorzugten Ausführungsform der vorliegenden Erfindung wird in einem abschließenden Verfahrensschritt, insbesondere einen Verfahrensschritt (d), die Qualität der Oberflächenmodifizierung, insbesondere der Hydrophobierung, bestimmt. In diesem Zusammenhang wird es bevorzugt, wenn die Qualität der Oberflächenmodifizierung für jedes Bauteil bestimmt wird. Eine abschließende umfassende Qualitätskontrolle der mikrostrukturierten Bauteile ist stets von Vorteil und insbesondere dann notwendig, wenn das mikrostrukturierte Bauteil in medizinische Geräte, beispielsweise Inhalationsgeräte, eingebaut werden soll.

Was nun die Bestimmung der Qualität der Oberflächenmodifizierung anbelangt, so kann diese in vielfältiger Art und Weise durchgeführt werden. Besonders gute Ergebnisse werden jedoch im Rahmen der vorliegenden Erfindung erhalten, wenn die Qualität der Oberflächenmodifizierung mittels optischer Methoden, insbesondere anhand von Bilddaten, bestimmt wird. In diesem Zusammenhang hat es sich bewährt, wenn die Oberflächenmodifizierung mittels optischer Methoden, insbesondere durch ortsaufgelösten Vergleich von gemessenen Parametern mit Sollwerten bestimmt wird.

Wenn das Bauteil teilweise aus Glas, vorzugsweise aus Glas und Silizium, besteht, kann beim Werkstoff Glas die Durchlässigkeit von sichtbarem Licht für eine besonders effektive Qualitätskontrolle genutzt werden. An einem zumindest teilweise für sichtbares Licht durchlässigen mikrostrukturierten Bauteil kann eine einfache automatisierte Kontrolle anhand optischer Methoden erfolgen, da Abweichungen in der Dicke der Oberflächenmodifizierung, insbesondere der Beschichtung, sowie Verstopfungen der Mikrostruktur ohne Weiteres erkannt werden können.

Im Rahmen der vorliegenden Erfindung ist es in diesem Zusammenhang üblicherweise vorgesehen, dass auf Grundlage der Bestimmung der Qualität der Oberflächenmodifizierung die Bauteile kategorisiert werden, insbesondere fehlerhafte Bauteile aussortiert werden. Insbesondere eine optische Qualitätskontrolle der mikrostrukturierten Bauteile ermöglicht es im großindustriellen Maßstab mikrostrukturierte Bauteile für medizinische Anwendungen herzustellen.

Gemäß einer bevorzugten Ausführungsform der vorliegenden Erfindung betrifft die vorliegende Erfindung ein Verfahren zur Oberflächenmodifizierung, insbesondere Hydrophobierung, von mikrostrukturierten Bauteilen mit polarer Oberfläche, insbesondere für Hochdruckanwendungen, wobei ein mikrostrukturiertes Bauteil mit einem Modifizierungsreagenz in Kontakt gebracht, insbesondere behandelt, wird, wobei durch chemische und/oder physikalische Wechselwirkung der Bauteiloberfläche und des Modifizierungsreagenzes die Oberflächeneigenschaften des Substrats modifiziert werden, wobei das Modifizierungsreagenz mindestens ein Modifizierungsmittel aufweist und wobei als Modifizierungsmittel ein Silan gemäß der allgemeinen Formel VI

$$R^1_{4-(n+m)}SiR^2_mX_n \qquad (VI)$$

mit

$R^1$ = Alkyl, insbesondere $C_1$- bis $C_{20}$-Alkyl, vorzugsweise $C_8$- bis $C_{18}$-Alkyl, bevorzugt $C_{10}$- bis $C_{16}$-Alkyl; Aryl, insbesondere $C_6$- bis $C_{20}$-Aryl, bevorzugt $C_6$- bis $C_{10}$-Aryl; Olefin, insbesondere terminales Olefin, vorzugsweise $C_2$- bis $C_{20}$-Olefin, bevorzugt $C_8$- bis $C_{18}$-Olefin, besonders bevorzugt $C_{10}$- bis $C_{16}$-Olefin

$R^2$ = Alkyl, insbesondere $C_1$- bis $C_3$-Alkyl, vorzugsweise Methyl;

X = Halogenid, insbesondere Chlorid und/oder Bromid, vorzugsweise Chlorid; Alkoxy, insbesondere $C_1$- bis $C_6$-Alkoxy, besonders bevorzugt $C_1$- bis $C_4$- Alkoxy, ganz besonders bevorzugt $C_1$- und/oder $C_2$-Alkoxy; und

n = 1 bis 3, insbesondere 3, und

m = 0 bis 2, insbesondere 0 oder 2, vorzugsweise 0,

eingesetzt wird.

Es versteht sich von selbst, dass weitere vorteilhafte Ausgestaltungen des Erfindungsgemäßen Verfahrens, welche im Zusammenhang mit anderen Ausführungsformen des erfindungsgemäßen Verfahrens beschrieben sind, auch in Bezug auf diese spezielle Ausführungsform entsprechend gelten.

Gemäß einer weiteren bevorzugten Ausführungsform der vorliegenden Erfindung betrifft die vorliegende Erfindung ein Verfahren zur Oberflächenmodifizierung, insbesondere Hydrophobierung, von mikrostrukturierten Bauteilen mit polarer Oberfläche, insbesondere für Hochdruckanwendungen, wobei ein mikrostrukturiertes Bauteil mit einem Modifizierungsreagenz in Kontakt gebracht, insbesondere behandelt, wird, wobei durch chemische und/oder physikalische Wechselwirkung der Bauteiloberfläche und des Modifizierungsreagenzes die Oberflächeneigenschaften des Substrats modifiziert werden, wobei das Modifizierungsreagenz mindestens ein Modifizierungsmittel aufweist und das Modifizierungsmittel ausgewählt ist aus der Gruppe von Silanen, Siloxanen, Polysiloxanen und/oder Silikonaten sowie deren Mischungen, wobei das Modifizierungsmittel nach dem Inkontaktbringen mit dem Bauteil getrocknet und/oder ausgehärtet wird und wobei überschüssiges Modifizierungsreagenz nach dem Verfahrensschritt des Trocknens und/oder Aushärtens durch Behandlung des Bauteils in einem Spin Rinse Dryer entfernt wird.

Es versteht sich von selbst, dass weitere vorteilhafte Ausgestaltungen des Erfindungsgemäßen Verfahrens, welche im Zusammenhang mit anderen Ausführungsformen des erfindungsgemäßen Verfahrens genannt sind, auch in Bezug auf diese spezielle Ausführungsform entsprechend gelten.

Es versteht sich von selbst, dass weitere vorteilhafte Ausgestaltungen des Erfindungsgemäßen Verfahrens, welche im Zusammenhang mit anderen Ausführungsformen des erfindungsgemäßen Verfahrens genannt sind, auch in Bezug auf diese spezielle Ausführungsform entsprechend gelten.

Gemäß einer gleichfalls bevorzugten Ausführungsform der vorliegenden Erfindung betrifft die vorliegende Erfindung ein Verfahren zur Oberflächenmodifizierung, insbesondere Hydrophobierung, von mikrostrukturierten Bauteilen mit polarer Oberfläche, insbesondere für Hochdruckanwendungen, wobei ein mikrostrukturiertes Bauteil mit einem Modifizierungsreagenz in Kontakt gebracht, insbesondere behandelt, wird, wobei durch chemische und/oder physikalische Wechselwirkung der Bauteiloberfläche und des Modifizierungsreagenzes die Oberflächeneigenschaften des Substrats modifiziert werden, wobei das Modifizierungsreagenz mindestens ein Modifizierungsmittel aufweist und das Modifizierungsmittel ausgewählt ist aus der Gruppe von Silanen, Siloxanen, Polysiloxanen und/oder Silikonaten sowie deren Mischungen, wobei in einem abschließenden Verfahrensschritt die Qualität der Oberflächenmodifizierung bestimmt wird.

Es versteht sich von selbst, dass weitere vorteilhafte Ausgestaltungen des Erfindungsgemäßen Verfahrens, welche im Zusammenhang mit anderen Ausführungsformen des erfindungsgemäßen Verfahrens genannt sind, auch in Bezug auf diese spezielle Ausführungsform entsprechend gelten.

Weiterer Gegenstand der vorliegenden Erfindung gemäß einem **zweiten** Aspekt der vorliegenden Erfindung ist ein mikrostrukturiertes Bauteil aufweisend eine Oberflächenmodifizierung, insbesondere ein Beschichtung, welche nach dem zuvor genannten Verfahren erhältlich ist.

Für weitergehende Einzelheiten zu diesem Erfindungsaspekt kann auf die vorangehenden Ausführungen zu dem erfindungsgemäßen Verfahren verwiesen werden, welche in Bezug auf das erfindungsgemäße mikrostrukturierte Bauteil entsprechend gelten.

Wiederum weiterer Gegenstand der vorliegenden Erfindung - gemäß einem **dritten** Aspekt der vorliegenden Erfindung - ist ein mikrostrukturiertes Bauteil, insbesondere ein Düsensystem, eines mikrofluiden Systems, aufweisend mindestens eine Einlassöffnung, mindestens eine Auslassöffnung sowie durch Mikrostrukturen gebildete innere Oberflächen, wobei die inneren Oberflächen zumindest teilweise modifiziert, insbesondere beschichtet, sind.

Bei dem erfindungsgemäßen mikrostrukturierten Bauteil handelt es sich vorzugsweise um einen Düsenkörper, welches in Inhalatoren des SMI-Typs eingesetzt werden. Besonders bevorzugt ist das erfindungsgemäße Bauteil eine DJI-Düsen.

Gemäß einer bevorzugten Ausführungsform der vorliegenden Erfindung ist die äußere Oberfläche des Bauteils modifiziert, insbesondere beschichtet, insbesondere im Bereich der Auslassöffnung. Eine Beschichtung nicht nur der inneren Oberflächen des erfindungsgemäßen mikrostrukturierten Bauteils, sondern vielmehr auch eine zumindest bereichsweise Beschichtung der äußeren Oberfläche, zumindest im Bereich der Auslassöffnung(en) ist bevorzugt, da eine Beschichtung zumindest im Bereich der Auslassöffnung(en) eine Ablagerung von Partikeln in diesem Bereich verhindert

und somit einer Verstopfung bzw. Verlegung der Düsen effektiv entgegenwirkt.

Besonders gute Ergebnisse werden im Rahmen der vorliegenden Erfindung erhalten, wenn die Oberfläche des Bauteils insbesondere zumindest im Wesentlichen modifiziert, insbesondere beschichtet, ist. Im Rahmen der vorliegenden Erfindung wird es somit bevorzugt, wenn die gesamte Oberfläche des Bauteils insbesondere zumindest im Wesentlichen modifiziert, insbesondere beschichtet, ist.

Es hat sich insbesondere bewährt, wenn die Oberfläche des Bauteils hydrophob modifiziert, insbesondere hydrophobiert, ist. Speziell bei Verwendung hydrophiler Substrate, wie beispielsweise von Glas- oder Siliziumsubstraten, können die Oberflächeneigenschaften durch eine Hydrophobierung gezielt eingestellt werden.

Im Allgemeinen ist die Oberfläche des Bauteils durch Umsetzung mit einem Modifizierungsreagenz modifiziert.

Was nun das Modifizierungsreagenz anbelangt, so kann dieses aus sämtlichen geeigneten Reagenzien ausgewählt werden. Im Rahmen der vorliegenden Erfindung werden jedoch besonders gute Ergebnisse erhalten, wenn dass Modifizierungsreagenz mindestens ein Silan, ein Siloxan, ein Polysiloxan und/oder ein Silikonat enthält. Für weitere Einzelheiten zur bevorzugten Modifizierungsreagenzien kann auf die vorhergehenden Ausführungen zu dem erfindungsgemäßen Verfahren verwiesen werden.

Gemäß einer bevorzugten Ausführungsform der vorliegenden Erfindung weist das mikrostrukturierte Bauteil zwei Auslassöffnungen auf. In diesem Zusammenhang ist es insbesondere vorgesehen, dass das Bauteil Kanäle aufweist, welche in die Auslassöffnung bzw. die Auslassöffnungen münden.

Darüber hinaus ist es üblicherweise vorgesehen, dass die Kanäle die Einlassöffnungen und die Auslassöffnungen direkt oder indirekt verbinden. Unter einer indirekten Verbindung der Einlassöffnung und Auslassöffnung durch die Kanäle ist dabei zu verstehen, dass weitere Teile der Mikrostruktur zwischen den Einlass- und Auslassöffnungen vorgesehen sind, so dass die Kanäle die einzelnen Bereiche bzw. Teile der Mikrostruktur verbinden.

Gemäß einer bevorzugten Ausführungsform der vorliegenden Erfindung ist es vorgesehen, dass die Kanäle der Auslassöffnungen in einem Winkel von 50 bis 130°, insbesondere 60 bis 120°, vorzugsweise 70 bis 110°, bevorzugt 80 bis 100°, besonders bevorzugt 85 bis 95°, ganz besonders bevorzugt 90°, aufeinander gerichtet sind. Vorzugsweise sind die Kanäle so ausgerichtet, dass sich ihre linearen Verlängerungen schneiden und die genannten Winkel einschließen. Dadurch, dass die Kanäle aufeinander gerichtet sind, treffen unter Druck aus den Auslassöffnungen austretende Fluide in einiger Entfernung des mikrostrukturierten Bauteils aufeinander und werden durch den Aufprall zerstäubt. Es bildet sich eine Impaktionsscheibe aus fein verteiltem Aerosol mit geringer kinetischer Energie, welches ein tiefes Eindringen von pharmazeutischen Wirkstoffen in die Lunge ermöglicht.

Im Allgemeinen ist es im Rahmen der vorliegenden Erfindung vorgesehen, dass das Bauteil weiterhin einen Filterbereich aufweist, welcher vorzugsweise zwischen der Einlassöffnung und der Auslassöffnung angeordnet ist. Durch den Filterbereich werden feine Verunreinigungen zurückgehalten, welche das Fluid, das durch die Auslassöffnungen, insbesondere die Düsen, austreten soll, enthält. Derartige Verunreinigungen können beispielsweise durch eine Reaktion des Fluids mit den Wandungen des Lagerbehälters des Fluids zustande kommen. Darüber hinaus ist es auch möglich, dass im Laufe der Zeit Feststoffe während der Lagerung des Fluids ausfallen.

Im Rahmen der vorliegenden Erfindung ist es weiterhin vorgesehen, dass die Mikrostrukturen, insbesondere die Kanäle der Auslassöffnungen, eine Tiefe und/oder einen Durchmesser im Bereich von 0,1 bis 50 $\mu$m, insbesondere 0,5 bis 40 $\mu$m, vorzugsweise 1 bis 20 $\mu$m, bevorzugt 2 bis 15 $\mu$m, besonders bevorzugt 2,5 bis 10 $\mu$m, ganz besonders bevorzugt 3 bis 8 $\mu$m, aufweisen. Unter einer Tiefe der Mikrostrukturen ist dabei die Höhendifferenz zwischen der Mikrostruktur und der umgebenden Bauteiloberfläche bei äußeren Oberflächenstrukturen zu verstehen. Die Tiefe der Mikrostrukturen kann z. B. durch materialentfernende Verfahren, wie Fräsen, Bohren, Laser etc. oder im Falle von Siliziumwafern durch Ätzen präzise eingestellt werden.

Gemäß einer besonderen (nicht weiter dargestellten) Ausführungsform der vorliegenden Erfindung sind auch äußere Oberflächen des Bauteils insbesondere zumindest teilweise mikrostrukturiert, insbesondere im Bereich der Auslassöffnungen. Durch eine Mikrostrukturierung im Bereich der Auslassöffnungen ist es möglich, Partikelanhaftungen nochmals effektiv entgegenzuwirken, wodurch eine Verlegung bzw. Verstopfung der Auslassöffnung bzw. der Auslassöffnungen entgegengewirkt wird.

Im Rahmen der vorliegenden Erfindung besteht das Bauteil üblicherweise aus mindestens zwei unterschiedlichen Materialien, insbesondere Glas und Silizium.

Darüber hinaus kann es vorgesehen sein, dass die unterschiedlichen Materialien insbesondere zumindest im Wesentlichen quader-, insbesondere plattenförmig, ausgebildet sind. Eine quader- bzw. plattenförmige Ausbildung der Materialien erlaubt eine besonders feste und beständige Verbindung der beiden Materialien, da große Oberflächen miteinander verbunden werden können.

Weiterhin wird es bevorzugt, wenn die unterschiedlichen Materialien fest miteinander verbunden, insbesondere gebondet, sind.

Im Rahmen der vorliegenden Erfindung werden besonders gute Ergebnisse erhalten, wenn mindestens eines der unterschiedlichen Materialien, vorzugsweise an seiner Oberfläche, mikrostrukturiert ist. Wie zuvor bereits erläutert, können Mikrostrukturen im Inneren eines Bauteils erhalten werden, welches mehrere Materialien aufweist, indem mindestens eines der Materialien in einer seiner Oberfläche eine Mikrostruktur aufweist. Durch Verbinden der beiden Materialien kann aus der äußeren Oberfläche des einen Materials eine innere Oberfläche des Verbundmaterials entstehen.

Gemäß einer bevorzugten Ausführungsform der vorliegenden Erfindung wird die Mikrostruktur im Inneren des Bauteils durch die Verbindung der unterschiedlichen Materialien erhalten.

Für weitergehende Einzelheiten zu diesem Erfindungsaspekt kann auf die vorherigen Ausführungen zu den übrigen Erfindungsaspekten verwiesen werden, welche in Bezug auf das erfindungsgemäße mikrostrukturierte Bauteil entsprechend gelten.

Schließlich ist wiederum weiterer Gegenstand der vorliegenden Erfindung - gemäß einem **vierten** Aspekt der vorliegenden Erfindung - eine Austragsvorrichtung, insbesondere ein Zerstäuber, für Fluide, insbesondere für medizinische Flüssigkeiten, vorzugsweise flüssige Arzneimittel, aufweisend mindestens ein mikrostrukturiertes Bauteil wie zuvor beschrieben.

Im Rahmen der vorliegenden Erfindung ist es im Allgemeinen vorgesehen, dass die Austragsvorrichtung mindestens ein flüssiges Arzneimittel aufweist.

Bei den Arzneimitteln, welche in der Austragsvorrichtung vorhanden sind bzw. durch diese ausgetragen werden, handelt es sich üblicherweise um eine Dispersion, Suspension oder Lösung mindestens eines pharmazeutischen Wirkstoffs.

In diesem Zusammenhang kann es vorgesehen sein, dass der pharmazeutische Wirkstoff ausgewählt ist aus der Gruppe von Kortikosteroiden und/oder (Para-) Sympathomimetika.

Besonders gute Ergebnisse werden im Rahmen der vorliegenden Erfindung erhalten, wenn der pharmazeutische Wirkstoff ausgewählt ist aus Terbutalin, Salbutamol, Trospium, insbesondere Trospiumchlorid, Flutropium, insbesondere Flutropiumbromid, Tiotropium, insbesondere Tiotropiumbromid, Oxitropium, insbesondere Oxitropiumbromid, Iporatropium, insbesondere Ipratropiumbromid, Fenoterol, Budesonid, Fluticason, insbesondere Fluticasonpropionat, Glycopyrronium, insbesondere Glycopyrroniumbromid, Ciclesonid, und Beclometason, insbesondere, Beclometasondipropionat, sowie deren physiologisch verträglichen Salzen und Derivaten.

Was nun die Konzentration des pharmazeutischen Wirkstoffs in der Lösung oder Dispersion anbelangt, so kann dieser naturgemäß in weiten Bereichen variieren. Besonders gute Ergebnisse werden jedoch erhalten, wenn die Lösung oder Dispersion den Wirkstoff in Mengen von 0,01 bis 100 mmol/l, insbesondere 0,05 bis 80 mmol/l, vorzugsweise 0,1 bis 50 mmol/l, bevorzugt 0,5 bis 20 mmol/l, besonders bevorzugt 0,6 bis 10 mmol/l, ganz besonders bevorzugt 0,8 bis 5 mmol/l, bezogen auf die Lösung oder Dispersion, enthält. In den vorgenannten Konzentrationsbereichen lassen sich die Zusammensetzungen besonders gut aus der Vorrichtung ausbringen und zerstäuben, wobei insbesondere auch ein unerwünschtes Ausfallen der Substanzen wirksam verhindert wird.

Das Löse- oder Dispersionsmittel kann dabei aus allen physiologisch verträglichen Löse- oder Dispersionsmitteln ausgewählt sein. Üblicherweise ist das Löse- oder Dispersionsmittel ausgewählt aus organischen Lösemitteln, insbesondere Alkoholen, vorzugsweise Ethanol oder Isopropanol, bevorzugt Ethanol, und Wasser sowie deren Mischungen.

Insbesondere werden beschrieben Formulierungen mit Löse- oder Dispersionsmittel aus Ethanol und Wasser sowie deren Mischungen betrachtet, vorzugsweise werden Mischungen von Wasser und Ethanol im Bereich 1 : 80 bis 1 : 100, besonders bevorzugt 1 : 90, ausgewählt. Prinzipiell kann das jeweilige volumenbezogene Verhältnisse von Wasser zu Ethanol in den als Löse- oder Dispersionsmittel verwendeten Mischungen aber in größeren Bereichen variieren. Geeignete Ergebnisse, insbesondere im Hinblick auf die Partikelgröße und Partikelgrößenverteilung des erhaltenen Aerosols, sind angesichts der vorliegenden Untersuchungen auch für Mischungen zu erwarten, in denen das volumenbezogene Verhältnis von Wasser zu Ethanol im Bereich von 10 : 1 bis 1 : 50, insbesondere 5 : 1 bis 1 : 30, vorzugsweise 2 : 1 bis 1 : 20, bevorzugt 1 : 1 bis 1 : 15, besonders bevorzugt 1 : 2 bis 1 : 10, variiert.

Darüber hinaus hat es sich bewährt, wenn die Lösung oder Dispersion einen pH-Wert im Bereich von 2 bis 8, insbesondere 2,2 bis 7, vorzugsweise 2,5 bis 6,5, bevorzugt 2,8 bis 6, aufweist. Es ist möglich, Lösungen und Dispersionen von Arzneimitteln im sauren pH-Bereich langzeitstabil zu lagern und ohne Verlegung oder Verstopfung der Düsen auszubringen.

Es wird bevorzugt, wenn das Arzneimittel eine wässrige oder eine wässrig-ethanolische Lösung oder Dispersion von Budesonid ist.

Gemäß einer bevorzugten Ausführungsform ist das Arzneimittel das Arzneimittel eine Kombination aus

(a) mindestens einem Wirkstoff A ausgewählt aus der Gruppe von Budesonid, Fluticason, Ciclesonid und Beclometason, vorzugsweise Ciclesonid, sowie deren physiologisch verträglichen Salzen und Derivaten und
(b) mindestens einem Wirkstoff B ausgewählt aus der Gruppe von
6-Hydroxy-8-{1-hydroxy-2-[2-(4-methoxy-phenyl)-1,1-dimethyl-ethylamino]-ethyl}-4Hbenzo[1,4]oxazin-3-on;
6-Hydroxy-8-{1-hydroxy-2-[2-(4-phenoxy-essigsäureethylester)-1,1-dimethylethylamino]-ethyl}-4H-benzo[1,4]oxazin-3-on;
6-Hydroxy-8-{1-hydroxy-2-[2-(4-phenoxy-essigsäure)-1,1-dimethyl-ethylamino]-ethyl}-4H-benzo[1,4]oxazin-3-on;

8-{2-[1,1-Dimethyl-2-(2,4,6-trimethylphenyl)-ethylamino]-1-hydroxy-ethyl}-6-hydroxy-4H-benzo[1,4]oxazin-3-on;
6-Hydroxy-8-{1-hydroxy-2-[2-(4-hydroxy-phenyl)-1,1-dimethyl-ethylamino]-ethyl}-4H-benzo[1,4]oxazin-3-on;
6-Hydroxy-8-{1-hydroxy-2-[2-(4-isopropyl-phenyl)-1,1-dimethyl-ethylamino]-ethyl}-4H-benzo[1,4]oxazin-3-on;
8-{2-[2-(4-Ethyl-phenyl)-1,1-dimethyl-ethylamino]-1-hydroxy-ethyl}-6-hydroxy-4H-benzo[1,4]oxazin-3-on;
8-{2-[2-(4-Fluoro-3-methyl-phenyl)-1,1-dimethyl-ethylamino]-1-hydroxy-ethyl}-6- hydroxy-4H-benzo[1,4]oxazin-3-on;
8-{2-[2-(4-Fluor-2-methyl-phenyl)-1,1-dimethyl-ethylamino]-1-hydroxy-ethyl}-6-hydroxy-4H-benzo[1,4]oxazin-3-on;
8-{2-[2-(2,4-Difluor-phenyl)-1,1-dimethyl-ethylamino]-1-hydroxy-ethyl}-6-hydroxy-4Hbenzo[1,4]oxazin-3-on;
8-{2-[2-(3,5-Difluor-phenyl)-1,1-dimethyl-ethylamino]-1-hydroxy-ethyl}-6-hydroxy-4Hbenzo[1,4]oxazin-3-on;
8-{2-[2-(4-Ethoxy-phenyl)-1, 1-dimethyl-ethylamino]-1-hydroxy-ethyl}-6-hydroxy-4Hbenzo[1,4]oxazin-3-on;
8-{2-[2-(3,5-Dimethyl-phenyl)-1, 1-dimethyl-ethylamino]-1-hydroxy-ethyl}-6-hydroxy-4Hbenzo[1,4]oxazin-3-on;
4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure;
8-{2-[2-(3,4-Difluor-phenyl)-1,1-dimethyl-ethylamino]-1-hydroxy-ethyl}-6-hydroxy-4H-benzo[1,4]oxazin-3-on;
8-{2-[2-(2-Chlor-4-fluor-phenyl)-1, 1-dimethyl-ethylamino]-1-hydroxy-ethyl}-6-hydroxy-4H-benzo[1,4]oxazin-3-on;
8-{2-[2-(4-Chlor-phenyl)-1, 1-dimethyl-ethylamino]-1-hydroxy-ethyl}-6-hydroxy-4H-benzo[1,4]oxazin-3-on;
8-{2-[2-(4-Brom-phenyl)-1, 1-dimethyl-ethylamino]-1-hydroxy-ethyl}-6-hydroxy-4H-benzo[1,4]oxazin-3-on;
8-{2-[2-(4-Fluor-phenyl)-1, 1-dimethyl-ethylamino]-1-hydroxy-ethyl}-6-hydroxy-4H-benzo[1,4]oxazin-3-on;
8-{2-[2-(4-Fluor-3-methoxy-phenyl)-1, 1 -dimethyl-ethylamino]-1 -hydroxy-ethyl}-6-hydroxy-4H-benzo[1,4 ]oxazin-3-on;
8-{2-[2-(4-Fluor-2,6-dimethyl-phenyl)-1, 1-dimethyl-ethylamino]-1-hydroxy-ethyl}-6-hydroxy-4H-benzo[1,4]oxazin-3-on;
8-{2-[2-(4-Chlor-2-methyl-phenyl)-1,1-dimethyl-ethylamino]-1-hydroxy-ethyl}-6- hydroxy-4H-benzo[1,4]oxazin-3-on;
8-{2-[2-(4-Chlor-3-fluor-phenyl)-1, 1-dimethyl-ethylamino]-1-hydroxy-ethyl}-6-hydroxy-4H-benzo[1,4 ]oxazin-3-on;
8-{2-[2-(4-Chlor-2-fluor-phenyl)-1, 1-dimethyl-ethylamino]-1-hydroxy-ethyl}-6-hydroxy-4H-benzo[1,4]oxazin-3-on;
8-{2-[2-(3-Chlor-4-fluor-phenyl)-1, 1-dimethyl-ethylamino]-1-hydroxy-ethyl}-6-hydroxy-4H-benzo[1,4]oxazin-3-on;
8-{2-[2-(2,6-Difluor-4-methoxy-phenyl)-1, 1-dimethyl-ethylamino]-1-hydroxyethyl}-6-hydroxy-4H-benzo[1,4]oxazin-3-on;
8-{2-[2-(2,5-Difluor-4-methoxy-phenyl)-1,1-dimethyl-ethylamino]-1-hydroxyethyl}-6-hydroxy-4H-benzo[1,4]oxazin-3-on;
8-{2-[2-(4-Fluor-3,5-dimethyl-phenyl)-1,1-dimethyl-ethylamino]-1-hydroxyethyl}-6-hydroxy-4H-benzo[1,4]oxazin-3-on;
8-{2-[2-(3,5-Dichlor-phenyl)-1,1-dimethyl-ethylamino]-1-hydroxy-ethyl}-6-hydroxy-4Hbenzo[1,4]oxazin-3-on;
8-{2-[2-(4-Chlor-3-methyl-phenyl)-1,1-dimethyl-ethylamino]-1-hydroxy-ethyl}-6-hydroxy-4H-benzo[1,4]oxazin-3-on;
8-{2-[2-(3,4,5-Trifluor-phenyl)-1,1-dimethyl-ethylamino]-1-hydroxy-ethyl}-6-hydroxy-4H-benzo[1,4]oxazin-3-on;
8-{2-[2-(3-Methyl-phenyl)-1,1-dimethyl-ethylamino]-1-hydroxy-ethyl}-6-hydroxy-4H-benzo[1,4]oxazin-3-on und
8-{2-[2-(3,4-Dichlor-phenyl)-1,1-dimethyl-ethylamino]-1-hydroxy-ethyl}-6-hydroxy-4H-benzo[1,4]oxazin-3-on sowie deren physiologisch verträglichen Salzen und Derivaten.

Gemäß einer weiteren bevorzugten Ausführungsform ist das Arzneimittel eine Kombination aus

(a) mindestens einem Wirkstoff A, wie zuvor beschrieben,
(b) mindestens einem Wirkstoff B, wie zuvor beschrieben, und
(c) mindestens einem Wirkstoff C ausgewählt aus der Gruppe von Trospium, insbesondere Trospiumchlorid, Flutropium, insbesondere Flutropiumbromid, Tiotropium, insbesondere Tiotropiumbromid, Oxitropium, insbesondere Oxitropiumbromid, Iporatropium, insbesondere Ipratropiumbromid, Glycopyrronium, insbesondere Glycopyrroniumbromid, sowie deren physiologisch verträglichen Salzen und Derivaten.

Für weitergehende Einzelheiten kann auf die vorangehenden Ausführungen verwiesen werden, welche in Bezug auf die Austragsvorrichtung entsprechend gelten.

Schließlich wird beschrieben ein Verfahren zur Evaluation der Oberflächenmodifizierung eines mikrostrukturierten Bauteils, wobei eine Provokationslösung wiederholt durch das mikrostrukturierte Bauteil geleitet wird, insbesondere unter Hochdruck, und das Strömungsverhalten der Provokationslösung bei Austritt aus dem mikrostrukturierten Bauteil beobachtet wird.

Gemäß einer bevorzugten Ausführungsform wird durch den zeitlichen Verlauf des Strömungsverhalten, insbesondere die zeitliche Änderung des Strömungsverhaltens, der Provokationslösung bei Austritt aus dem mikrostrukturierten Bauteil die Güte der Oberflächenmodifizierung, insbesondere die Eignung des Modifizierungsreagenzes für die Oberflächenmodifizierung, bestimmt.

Es hat sich überraschenderweise herausgestellt, dass durch geeignete Provokationslösungen Ablagerungen in der Mikrostruktur des mikrostrukturierten Bauteils gezielt erzeugt werden können, welche das Strömungsverhalten der Lösung im Inneren des Bauteils und insbesondere bei Austritt aus den Austrittsöffnungen verändern, und zwar in sehr kurzen Zeiträumen. In der praktischen Anwendung des mikrostrukturierten Bauteils, beispielsweise in Inhalationsgeräten, treten derartige Störungen des Strömungsverhaltens nicht auf.

Die Ergebnisse aus den Provokationsversuchen können genutzt werden, um verbesserte Mikrostrukturen, insbesondere Düsengeometrien, zu bestimmen sowie beispielsweise Beschichtungsreagenzien auf ihre Eignung für eine Oberflächenmodifizierung zu überprüfen. Hierdurch kann der Einsatz des mikrostrukturierten Bauteils und der Austragsvorrichtung auf neue Anwendungsgebiete bzw. neue Werkstoffe erweitert werden.

Darüber hinaus dienen die Provokationsversuche auch zur Ermittlung von mikrostrukturellen Bauteilen mit verbesserter Langzeitperformence. Insbesondere im Fall von Modifizierungsreagenzien muss ein ausreichend großer Sicherheitsabstand zwischen den Bedingungen, unter welchen die Beschichtung nicht mehr ihren Zweck erfüllt, und den Anwendungsbedingungen vorliegen, damit insbesondere bei medizinischen Inhalationsgeräten der Anwender zuverlässig stets die therapeutische Dosis an Arzneimitteln zugeführt bekommt.

Unter einer Provokationslösung ist dabei verstehen, mit welcher Änderung des Strömungsverhaltens in mikrostrukturierten Bauteilen unter Bedingungen verursacht werden können, welche fernab der tatsächlichen Anwendungsbedingungen des mikrostrukturierten Bauteils liegen. So können den Provokationslösungen beispielsweise reaktive chemische Substanzen zugemischt werden oder Partikel, welche eine Verstopfung der Mikrostrukturen bzw. eine Änderung der Strömungsverhältnisse im Inneren des mikrostrukturierten Bauteils verursachen können.

Vorzugsweise wird die Provokationslösung 10- bis 1.000-mal, vorzugsweise 15- bis 500-mal, bevorzugt 25 bis 250-mal, durch die Mikrostruktur geleitet.

Es ist beschrieben, dass die Bestimmung des Strömungsverhaltens der Provokationslösung bei Austritt aus dem mikrostrukturierten Bauteil durch optische, insbesondere fotografische Methoden erfolgt. Insbesondere ist es in diesem Zusammenhang möglich, den Austritt der Provokationslösung aus den Auslassöffnungen fotografisch festzuhalten und die Bilddaten durch Vergleich mit einem vorher angelegten Katalog zu kategorisieren. Insbesondere ist es mit diesem Verfahren möglich, die zeitliche Änderung des Strömungsverhaltens zu untersuchen.

Beschrieben wird insbesondere eine DJI-Düse.

Üblicherweise ist es vorgesehen, dass die Provokationslösung wässrig und/oder alkoholisch basiert ist, insbesondere ein Wasser-Ethanol-Gemisch ist. In diesem Zusammenhang hat es sich bewährt, wenn die Provokationslösung ein volumenbezogenes Verhältnis von Alkohol zu Wasser im Bereich von 1 : 1 bis 20 : 1, insbesondere 3 : 1 bis 15 : 1, vorzugsweise 6 : 1 bis 12 : 1, bevorzugt von 9 : 1, aufweist.

Weiterhin kann es in diesem Zusammenhang vorgesehen sein, dass die Provokationslösung einen pH-Wert von höchstens 4, insbesondere höchstens 3, vorzugsweise höchstens 2, aufweist. Gleichfalls werden jedoch auch gute Ergebnisse erhalten, wenn die Provokationslösung einen pH-Wert im Bereich von 0 bis 4, insbesondere 0,1 bis 3, vorzugsweise 1 bis 2, aufweist.

Gemäß einer besonderen Ausführungsform weist die Provokationslösung mindestens einen weiteren Stoff ausgewählt aus Kieselsäuren und organischen chemischen Verbindungen, insbesondere Wirkstoffen, auf.

Wenn die die Provokationslösung organische chemische Verbindungen aufweist, so hat es sich bewährt, wenn die Provokationslösung die organische chemische Verbindung, insbesondere den Wirkstoff, in Mengen von 0,01 bis 5 mmol/l, insbesondere 0,1 bis 3 mmol/l, vorzugsweise 0,5 bis 2 mmol/l, bevorzugt 1 mmol/l, bezogen auf die Provokationslösung, aufweist.

Zur Ermittlung der Eignung von Beschichtungen bzw. zur Evaluation ihrer Beständigkeit kann eine Vielzahl von Wirkstoffen verwendet werden. Es hat sich in den bislang durchgeführten Experimenten gezeigt, dass beispielsweise mit Fenoterol besonders aussagekräftige Ergebnisse erhalten werden können.

Gleichfalls kann es auch vorgesehen, dass die Provokationslösung Kieselsäure in Mengen von 0,001 bis 2 Gew.-%, insbesondere 0,01 bis 1 Gew.-%, vorzugsweise 0,05 bis 0,5 Gew.-%, bevorzugt 0,1 Gew.-%, bezogen auf die Provokationslösung, aufweist.

Für weitergehende Einzelheiten kann auf die vorangehenden Ausführungen verwiesen werden, welche in Bezug auf das Verfahren zur Evaluation der Oberflächenmodifizierung eines mikrostrukturierten Bauteils entsprechend gelten.

Die einzelnen Merkmale der vorliegenden Erfindung können unabhängig voneinander genutzt oder miteinander kombiniert werden.

Weitere Vorteile, Merkmale, Eigenschaften und Aspekte der vorliegenden Erfindung ergeben sich aus den Ansprüchen, den Ausführungsbeispielen und der folgenden Beschreibung bevorzugter Ausführungsformen anhand der Zeichnungen. Die Zeichnungen zeigen in:

Fig. 1 eine schematische Darstellung eines mikrostrukturierten Bauteils mit einer Darstellung der Mikrostruktur einschließlich der Auslassöffnungen und der zu den Auslassöffnungen führenden Kanälen,

Fig. 2 einen Ausschnitt des in Fig. 1 dargestellten mikrostrukturierten Bauteils, bei welcher der Bereich des mikrostrukturierten Bauteils um die Auslassöffnungen detailliert dargestellt wird und die Beschichtung erkennbar ist,

Fig. 3 einen schematischen Schnitt einer Austragsvorrichtung, insbesondere eines Zerstäubers im ungespannten Zustand,

Fig. 4 einen schematischen, um 90° gegenüber Fig. 3 gedrehten Schnitt der Austragsvorrichtung, insbesondere des Zerstäubers, aus Fig. 1 im gespannten Zustand,

Fig. 5 Beispiel eines Spraybilds der Gruppe I (Abbildung in zwei verschiedenen Perspektiven) für die untersuchten Inhalatoren mit DJI-Düse,

Fig. 6 Beispiel eines Spraybilds der Gruppe II (Abbildung in zwei verschiedenen Perspektiven) für die untersuchten Inhalatoren mit DJI-Düse,

Fig. 7 die zeitliche Veränderung von Spraybildern der Gruppe III für Inhalatoren mit DJI-Düse mit unbeschichteten Düsenkörpern in Abhängigkeit vom pH-Wert,

Fig. 8 den 10 Tage-Duchschnitt der Rate von Spraybildern der Gruppe III für Inhalatoren mit DJI-Düse mit unbeschichteten Düsenkörpern in Abhängigkeit vom pH-Wert,

Fig. 9 die zeitliche Veränderung von Spraybildern der Gruppe I für Inhalatoren mit DJI-Düse mit beschichteten Düsenkörpern in Abhängigkeit vom Modifizierungsmittel,

Fig. 10 die zeitliche Veränderung von Spraybildern der Gruppe II für Inhalatoren mit DJI-Düse mit beschichteten Düsenkörpern in Abhängigkeit vom Modifizierungsmittel,

Fig. 11 die zeitliche Veränderung von Spraybildern der Gruppe III für Inhalatoren mit DJI-Düse mit beschichteten Düsenkörpern in Abhängigkeit vom Modifizierungsmittel,

Fig. 12 den 10 Tage-Duchschnitt der Rate von Spraybildern der Gruppe III für Inhalatoren mit DJI-Düse mit beschichteten Düsenkörpern in Abhängigkeit vom Modifizierungsmittel,

Fig. 13 die zeitliche Veränderung von Sprabildern der Gruppe I für Inhalatoren mit DJI-Düse mit mit 1H,1H,2H,2H-Tridecafluorooctyltriethoxysilan (F13C8-OET) beschichteten Düsenkörpern gegenüber Inhalatoren im Vergleich mit unbeschichteten Düsenkörpern im Langzeitversuch,

Fig. 14 die zeitliche Veränderung von Sprabildern der Gruppe II für Inhalatoren mit DJI-Düse mit mit 1H,1H,2H,2H-Tridecafluorooctyltriethoxysilan (F13C8-OET) beschichteten Düsenkörpern gegenüber Inhalatoren im Vergleich mit unbeschichteten Düsenkörpern im Langzeitversuch,

Fig. 15 die zeitliche Veränderung von Sprabildern der Gruppe III für Inhalatoren mit DJI-Düse mit mit 1H,1H,2H,2H-Tridecafluorooctyltriethoxysilan (F13C8-OET) beschichteten Düsenkörpern gegenüber Inhalatoren im Vergleich mit unbeschichteten Düsenkörpern im Langzeitversuch,

Fig. 16 den 10 Tage-Duchschnitt der Rate von Spraybildern der Gruppe III für Inhalatoren mit DJI-Düse mit mit 1H,1H,2H,2H-Tridecafluorooctyltriethoxysilan (F13C8-OET) beschichteten Düsenkörpern gegenüber Inhalatoren mit unbeschichteten Düsenkörpern im Langzeitversuch,

Fig. 17 die zeitliche Veränderung von Spraybildern der Gruppe I für Inhalatoren mit DJI-Düse im Vergleich mit beschichteten Düsenkörpern für weitere Modifizierungsmittel,

Fig. 18 die zeitliche Veränderung von Spraybildern der Gruppe II für Inhalatoren mit DJI-Düse mit beschichteten Düsenkörpern für weitere Modifizierungsmittel,

Fig. 19 die zeitliche Veränderung von Spraybildern der Gruppe III für Inhalatoren mit DJI-Düse mit beschichteten Düsenkörpern für weitere Modifizierungsmittel,

Fig. 20 den 10 Tage-Durchschnitt der Rate von Spraybildern der Gruppe III für Inhalatoren mit DJI-Düse mit beschichteten Düsenkörpern für weitere Modifizierungsmittel,

Fig. 21 die Ausbeute an Kategorie-I-Düsenkörpern in Abhängigkeit vom gewählten Modifizierungsmittel.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

Fig. 1 zeigt ein erfindungsgemäßes mikrostrukturiertes Bauteil 1, insbesondere einen Düsenkörper, in schematischer Draufsicht. Die Darstellung in Fig. 1 zeigt die Mikrostruktur eines Düsenkörpers mit einer Düse vom DJI-Typ, wie er bevorzugt in einem Inhalator des SMI-Typs verwendet wird. Der Düsenkörper ist vorzugsweise ein mikrofluides Sandwich-System und besteht aus einem quaderförmigen mikrostrukturierten Siliziumchip, der auf einer 0,5 mm dicken, vorzugsweise sehr glatten Glasplatte (z.B. ein Glas, das mit dem so genannten "Float"-Verfahren hergestellt wird, vorzugsweise auf Borosilikat-Basis) gebonded ist.

Das mikrostrukturierte Bauteil 1 besteht aus zwei fest miteinander verbundenen plattenförmigen Materialien, vorzugsweise einem Siliziumwafer und einem Glaswafer. Das Bauteil 1 besitzt Einlassöffnungen 2 und Auslassöffnungen 3 zur Aufnahme bzw. Abgabe von vorzugsweise unter Druck stehenden Fluiden, vorzugsweise Flüssigkeiten. An die Auslassöffnungen schließen sich Kanäle 4 an, welche vorzugsweise aufeinander gerichtet sind. Die Kanäle 4 und Auslassöffnungen 3 besitzen eine runde oder nicht runde Form, insbesondere vorzugsweise einen eckigen Querschnitt mit einem Durchmesser bzw. einer Tiefe von 2 bis 10 μm und einer Breite von 5 bis 15 μm, insbesondere in einer Tiefe von 4,5 bis 6,5 μm und einer Breite von 7 bis 9 μm.

Die Mikrostrukturen im Siliziumchip werden vorzugsweise durch Ätztechniken erzeugt. Die Ätztiefe auf dem Siliziumchip kann in Abhängigkeit des verwendeten Löse- oder Dispersionsmittels bzw. Verfahrens variieren. Vorzugsweise beträgt sie 5,6 μm für Düsenkörper für die Zerstäubung von wässrigen Formulierungen oder 7,0 μm für für die Zerstäubung von ethanolischen Formulierungen, bedingt durch die unterschiedlichen physikochemischen Eigenschaften der Lösungen bzw. Dispersionen. Die Gesamtquerschnittsfläche der Auslassöffnungen 3 liegt üblicherweise bei 30 bis 500 μm, wobei ein Querschnittsbereich von 30 bis 200 μm bevorzugt wird. Die Auslasskanäle 4 haben vorzugsweise eine Länge von 40 μm und eine Breite von 8μm.

Bei einem Bauteil 1 mit mindestens zwei Auslassöffnungen 3 können die Strahlrichtungen mit einem Winkel von 50 bis 130° gegeneinander geneigt sein, bevorzugt wird ein Winkel von 70 bis 110°, 85 bis 95°, ganz besonders bevorzugt 90°, erhalten. Die Auslassöffnungen 3 sind im Allgemeinen in einer Entfernung von 10 bis 200 μm angeordnet, insbesondere in einer Entfernung von 10 bis 100 μm, bevorzugt bei einer Entfernung von 30 bis 70 μm. Vorzugsweise beträgt die Entfernung zwischen den Auslassöffnungen 3 50 μm. Die Strahlrichtungen treffen sich nahe der Düsenöffnungen (vorzugsweise in einem Abstand kleiner 1mm, bevorzugt kleiner 100 μm, von der Oberfläche des Düsenkörpers) und durch den Aufprall der Fluidstrahlen wird das Fluid zerstäubt.

Im Folgenden wird eine bevorzugte Ausführungsform beschrieben, in welcher die Mikrostrukturen, welche durch die Einlassöffnungen 2, die Auslassöffnungen 3 und die Kanäle 4 definiert sind, in die Oberfläche eines Siliziumwafers eingebracht sind. Die Mikrostrukturen können durch sämtliche geeigneten Verfahren in den Siliziumwafer eingebracht werden, vorzugsweise erfolgt die Mikrostrukturierung jedoch durch Ätzverfahren, wie sie beispielsweise aus der Halbleitertechnik bekannt sind. Um Oberflächen im Inneren des mikrostrukturierten Bauteils 1 zu schaffen, welche geeignet sind, unter Druck stehende Fluide aufzunehmen und wieder abzugeben, werden die Mikrostrukturen in einem der Materialien, vorzugsweise dem Siliziumwafer, des Bauteils eingeführt und mit dem zweiten Bauteil, vorzugsweise einem Glaswafer, verbunden. Auf diese Weise können Hohlräume in Form von Mikrostrukturen in dem mikrostrukturierten Bauteil erhalten werden.

Zwischen den Auslassöffnungen 3 bzw. den Kanälen 4 kann ein Feinfilter 5 angeordnet sein, welcher aus einer Vielzahl von Filterkanälen, besteht. Die Durchgangswege bzw. Filterkanäle in der Filterstruktur im Feinfilter 5 sind derart gewählt, dass auch kleinste Verunreinigungen nach Möglichkeit nicht in den Bereich der Auslassöffnungen, d. h. den Düsenbereich, gelangen können und diesen verstopfen bzw. die Geometrie ändern. Der Durchmesser der Filterkanäle bzw. Filterdurchgangswege beträgt üblicherweise 0,5 bis 20 μm, vorzugsweise 2 bis 5 μm. der Feinfilter 5 weist üblicherweise zur Vergrößerung der Oberfläche eine Zick-Zack-Anordnung auf. Im Feinfilter 5 können Partikel, die größer sind als die Querschnitte der Filterkanäle, zurückgehalten werden um ein Verstopfen der Auslasskanäle zu verhindern. Die Filterkanäle der Filter des Feinfilters 5 werden durch Vorsprünge gebildet, die zur Erhöhung der Filteroberfläche vorzugsweise zickzackförmig angeordnet sind. So bilden beispielweise mindestens zwei Reihen der Vorsprünge eine Zick-Zack-Konfiguration. Auch können mehrere Reihen von Vorsprüngen ausgebildet sein, wobei die Reihen vorzugsweise in spitzen Winkeln an einander angrenzen und die Zick-Zack-Konfiguration bilden. In solchen Ausführungsformen

kann der Einlass und der Auslass jeweils einen Einströmbereich für unfiltriertes bzw. einen Ausströmbereich für filtriertes Fluid aufweisen, wobei der Einströmbereich bzw. Ausströmbereich im Wesentlichen genauso bereit ist wie der Filter 5 und im Wesentlichen genauso hoch ist wie die Vorsprünge für den Einlass- bzw. Auslassseiten des Filters 5. Die Zick-Zack-Konfiguration, die von wenigstens zwei Reihen von Vorsprüngen gebildet wird, weist einen Neigungswinkel von bevorzugt 20 bis 250 ° auf. Weitere Einzelheiten dieser Bauteilkonstruktion können der WO 94/07607 A1 entnommen werden.

Das mikrostrukturierte Bauteil 1 kann einen Ausströmbereich oder eine Plenumkammer 6 aufweisen. Insbesondere ist die Plenumkammer 6 zwischen den Auslassöffnungen 3 bzw. den Kanälen 4 und dem Feinfilter 5 angeordnet. Die Plenumkammer kann anwendungsbedingt Säulenstrukturen 7 aufweisen. Durch die Säulenstrukturen 7 in der Plenumkammer 6 wird eine Vielzahl von Kanälen geschaffen, welche vorzugsweise in den Kanälen 4 der Auslassöffnungen 3 zusammenlaufen.

Das mikrostrukturierten Bauteils 1 bzw. der Düsenkörper stellt ein rigides System dar, das dazu gedacht ist, die zwei Flüssigkeitsstrahlen nach Austritt aus den Auslassöffnungen 3 miteinander impaktieren zu lassen. Bei korrekter Impaktion bildet sich eine sogenannte Impaktionsscheibe, an deren Grenzfläche das feine Aerosol entsteht. Zu den kritischen Parametern der Aerosolbildung zählen u.a. die Strömungsgeschwindigkeit (ca. 100 m/s) und der Impaktionswinkel. Deponiertes Material in den Düsenkanälen kann daher die Aerosolbildung, beispielsweise durch Strahlablenkung, empfindlich beeinflussen und sogenannte Sprayanomalien bis hin zum Ausbleiben der Spraywolke durch Nicht-Treffen der Strahlen (Jet-Divergency) hervorrufen.

Die Oberflächen des mikrostrukturierten Bauteils 1 weisen eine Beschichtung 8 auf, welche die Oberflächeneigenschaften des mikrostrukturierten Bauteils bestimmt. Hierbei kann es vorgesehen sein, dass sowohl die inneren als auch die äußeren Oberflächen des mikrostrukturierten Bauteils 1 beschichtet sind. Bevorzugt sind zumindest die inneren Oberflächen des mikrostrukturierten Bauteils beschichtet, um eine Anhaftung von Partikeln und somit eine Düsenverlegung bzw. Düsenverstopfung zu vermeiden.

Gemäß einer bevorzugten Ausführungsform der vorliegenden Erfindung ist auch die äußere Oberfläche des mikrostrukturierten Bauteils 1 zumindest im Bereich der Auslassöffnungen modifiziert bzw. beschichtet.

Fig. 3 und 4 zeigen eine schematische Darstellung einer erfindungsgemäßen Austragsvorrichtung in Form eines handbetätigten medizinischen Gerätes. Bei der Austragsvorrichtung gemäß Fig. 3 und 4 handelt es sich vorzugsweise um einen treibgasfreien Zerstäuber 9, der pro Betätigungszyklus jeweils vorbestimmte Menge einer Flüssigkeit bzw. einer flüssigen medizinischen Formulierung als vorzugsweise lungengängiges bzw. inhalierfähiges Aerosol 11 ausgibt. Aerosol-Tröpfchen mit einem aerodynamischen Durchmesser von 0,5 bis 5 $\mu$m können von einem Benutzer eingeatmet werden. Die durchschnittliche aerodynamischen Tröpfchengröße des Aerosols 11 liegt vorzugsweise in einem Durchmesserbereich von 0,5 bis 10 $\mu$m, insbesondere im Bereich von 0,5 bis 5$\mu$m. Zur Zerstäubung wird hierbei eine geeignete Düse in Form des erfindungsgemäßen mikrostrukturierten Bauteils 1 verwendet. Beim Betrieb des Zerstäubers 9 wird unterschieden zwischen dem so genannten "ungespannten Zustand" mit unbefülltem Dosiervolumen in der Drucckammer 12 (Fig. 3) und dem so genannten "gespannten Zustand" mit befüllter Druckkammer 12 (Fig. 4). Beim Spannen des Zerstäubers 9 wird das Gehäuseoberteil 13 relativ zum Gehäuseinnenteil 14 und Gehäuseunterteil 15 um einen festen Drehwinkel wie z. B. 180° gedreht. Mittels einen innen angeordneten Schraubgetriebes wird durch die Relativdrehung eine Kolbenpumpe angetrieben, so dass eine vorbestimmte, gegebenenfalls einstellbare der Menge der Flüssigkeit 10 aus dem Behälter 16 in die Druckkammer 12 gefördert und gleichzeitig die Antriebsfeder 17 des Druckerzeugers 18 gespannt wird (der Endzustand des Spannvorgangs ist in Fig. 4 dargestellt). Beim Auslösen des Zerstäubers 9, d. h. bei Betätigung eines Sperrringes 19 über eine Taste 20 wird die in der Antriebsfeder 17 gespeicherte Energie des Druckerzeugers 18 freigesetzt: Der zuvor zur Flüssigkeitsförderung benutzte Hohlkolben 21 drückt dann mit geschlossenem Rückschlagventil 22 in die Druckkammer 12, so dass die durch die Hubbewegung des Hohlkolbens 21 vorbestimmte Flüssigkeitsmenge von dort durch die Auslassöffnung 3 ausgebracht wird. Das Gerät befindet sich nun wieder im entspannten Zustand, wie er in Fig. 3 dargestellt ist.

Im Darstellungsbeispiel ist der Hohlkolben 21 fest mit einer zum Druckerzeuger 18 gehörenden Halterung 23 über den Behälter 16 verbunden - beispielsweise angespritzt, verklebt oder verschnappt. Der Behälter 16 wird über die Halterung 23 insbesondere klemmend oder rastend so in dem Zerstäuber 9 fixiert, dass der Hohlkolben 21 im Fluidraum des Behälters 16 eintaucht und/oder fluidisch mit der Flüssigkeit 10 im Behälter 16 verbunden wird und sie über den Hohlkolben angesaugt werden kann. Der Behälter kann wahlweise austauschbar sein. Das Gerätegehäuse kann zu diesem Zweck so gestaltet sein, dass es aufgemacht oder teilweise abgenommen werden kann (z. B. in Form eines kappenartigen Gehäuseunterteils wie in WO 07/123381 A1 offenbart).

Der Behälter 16, der in dem mit einem Dosierindikator oder einem Zählwerk 24 ausgestatteten Zerstäuber 9 eingesetzt wird, ist für die Entnahme mehrerer Dosiseinheiten ausgelegt. Dazu muss er so beschaffen sein, dass der Innendruck auch Flüssigkeitsentnahme im Wesentlichen unverändert bleibt, so dass beim Ansaugen immer die gleiche Menge Flüssigkeit 10 entnommen wird. Hierzu kann prinzipiell sowohl ein Behälter 16 mit starrer Behälterwand, dessen Innendruck über Belüftung konstant gehalten wird und wieder beispielsweise in der WO 06/136426 A1 beschrieben wird, als auch ein Behälter 16 mit einer flexiblen Wand verwendet werden, die sich bei Flüssigkeitsentnahme zumindest

teilweise derart ins Behälterinnere verschiebt, dass die Verkleinerung des Innenvolumens der Innendruck konstant gehalten wird. Bevorzugt werden dabei Behälter 16, in denen die flexible Wand durch einen Beutel gebildet wird, der im Wesentlichen verformbar, zusammendrückbar und/oder zusammenziehbar ausgebildet ist. Solche Behälter werden in verschiedenen Ausführungsformen in den Schriften WO 00/49988 A2, WO 01/076849 A1, WO 99/43571 A1, WO 09/115200 A1 und WO 09/103510 A1 beschrieben. Besonders bevorzugt besteht der Behälter 16 aus einem flexiblen, mehrschichtigen, unten geschlossenen Folienbeutel, der im oberen Bereich direkt mit einem haltgebenden Flansch, vorzugsweise aus Kunststoff, verbunden ist, eine daran angeschweißten Behälterkappe zur Anbindung an die Halterung 23 des Zerstäubers 9, einer äußeren Schutzhülse und einem Kopfsiegel (Details siehe WO 99/43571 A1 und WO 09/115200 A1). Das typische Füllvolumen eines Behälters 16 besteht aus 3,0 bis 3,6 ml Inhalationslösung.

Im Flüssigkeitsauslassbereich der Druckkammer 12 kann sich ein Filtersystem 25 befinden, dass dem mikrostrukturierten Bauteil 1 vorangeht. Dieses Filtersystem 25 besteht vorzugsweise aus mehreren, hintereinander angeordneten Filterbauteilen, die sich insbesondere durch die angewendete Filtertechnik unterscheiden. Die Filterschwellen der einzelnen Filterbauteile sind dabei derart bemessen, dass jeder Filter nach dem größten Austauschprinzip kleinere Partikel durchlässt als sein Vorgänger. Über die Kombination verschiedener Filtertechniken und die Anordnung von Filtern mit sukzessivem wachsendem Trenngrad bzw. sukzessiv kleiner werdenden Porengrößen wird insgesamt eine hohe Filterkapazität, d. h. die Abscheidung von größeren Partikelmengen ohne komplette Filterverlegung, und eine gründliche Filterung erreicht. Zusätzlich zum Auffang fester Partikel mit einer bestimmten Größe kann der Filter gegebenenfalls zusätzliches Material über Adsorption auffangen. Hierzu können Filter unterschiedlicher Beschaffenheit und unterschiedlicher Materialien eingesetzt werden, so dass sich die Adsorptionseigenschaften von Filter zu Filter unterscheiden. Durch die Kombination von verschiedenen Filtern können dementsprechend auch mehr Partikel und insbesondere auch unter Druck verformbare Partikel dank der verschiedenen Adsorptionseffekte abgefangen werden. Für weitergehende Einzelheiten zu bevorzugten Filtersystemen wird insbesondere auf die WO 2012/007315 verwiesen.

Das Gesamtsystem aus Druckerzeuger 18 mit Antriebsfeder 17 und dem mikrostrukturierten Bauteil 1 ist vorzugsweise so aufgebaut, dass bei der Sprühnebelerzeugung nicht nur an die Lungengängigkeit angepasste Tröpfchengrößen gebildet werden, sondern dass die Sprühnebelwolke selbst so lange anhält, dass der Patient seine Einatmung leicht an sie anpassen kann. Bevorzugt werden hierbei Sprühzeiten von 0,5 bis 2 s, insbesondere von 1 bis 2 s. Der Druck, mit dem das Fluid, insbesondere das flüssige Medikament, die Auslassöffnungen verlässt beträgt 50 bis 1000 bar, insbesondere 200 bis 600 bar.

Das Aerosol 11 enthält (in Abhängigkeit von der Dimensionierung der Bauteile und von der zu zerstäubenden flüssigen Formulierung) einen ausgesprochen hohen Feinpartikelanteil (hier: Anteil, den die Partikel mit Durchmessern $\leq 5\ \mu m$ im Spray ausmachen) von beispielsweise $\geq 50\%$, vorzugsweise $\geq 65\ \%$, besonders bevorzugt $\geq 80\%$ insbesondere für ethanolische Formulierungen, und die erzeugte Sprühwolke ist vorzugsweise langsamer als in andersartigen tragbaren Inhalatoren wie beispielsweise des MDI-Typs. Dies führt zu einer wesentlich höheren Lungendeposition als bei anderen herkömmlichen Inhalatoren, wie z.B. pMDIs und DPIs. Darüber hinaus zeichnet sich der erfindungsgemäße Zerstäuber 9 durch eine ausgesprochen lange Sprühdauer aus, was eine gute Patienten-Koordination hinsichtlich der Auslösung des Zerstäubers 9 bzw. des Inhalalators ermöglicht.

In Abhängigkeit der benötigten Tagesdosis und des vorgesehenen Anwendungszeitraums kann der Zerstäuber 9 für die Abgabe von 10 bis 200, insbesondere 20 bis 150, vorzugsweise 60 bis 130, Sprühstößen ausgelegt sein. Ein Reiter am Zählwerk 24 zeigt an wie viele Hübe bereits verbraucht wurden beziehungsweise noch vorhanden sind. Nachdem die spezifische Hubanzahl erreicht wird, verriegelt sich die Austragsvorrichtung vorzugsweise automatisch und ist für weitere Anwendungen gesperrt. Ein "tailoff", wie es in druckgasbetriebenen Dosieraerosolen zu beobachten ist, wird dadurch vermieden.

Um den Zerstäuber 9 für die Anwendung vorzubereiten muss zunächst ein Behälter 16, insbesondere in Form einer Kartusche, eingesetzt werden. Hierfür muss das Gehäuseunterteil 15 abgenommen werden. Nach dem Einsetzen des Behälters wird das entfernte Gehäuseunterteil 15 wieder aufgesetzt und das Gerät durch mehrmaliges Betätigen geprimt (= Ausbringen der Luft aus dem System). Erst ab diesem Zeitpunkt ist der Zerstäuber 9 einsatzbereit und garantiert eine konstante Dosisabgabe.

Das Priming dient der vollständigen Befüllung der Dosierkammer bzw. Druckkammer 12. Bei der Betätigung des Geräts wird bei senkrecht nach oben gerichtetem Mundstück das Gehäuseunterteil 15 gegen das Gehäuseoberteil 18 um 180° bis zum hörbaren Klicken und Einrasten gedreht. Dabei wird die Antriebsfeder 17 gespannt, die Taste 20 springt beim Einrasten nach vorne und zeigt durch bündigen Sitz mit den Seitenflanken den gespannten Zustand des Zerstäubers 9 an. Durch Drücken der Taste 20 wird das Aerosol generiert, wobei die Lage des Zerstäubers 9 frei wählbar ist.

## Ausführungsbeispiele

### 1. Verwendete Methoden und Experimentaufbau

Für die nachfolgenden Untersuchungen wurden die Oberflächen von Flachsubstrate aus Silizium und Glas sowie eines mikrostrukturierte Düsensystems modifiziert und auf ihre Eigenschaften untersucht. Als Flachsubstrate für die im folgenden erläuterten Versuche werden in erster Linie handelsübliche Glas-Flachsubstrate verwendet, die im sogenannten "Float"-Verfahren aus Borosilikatglas hergestellt wurden. Als mikrostrukturiertes Düsensystem werden Düsenkörper mit Mikrostrukturen entsprechend der Abbildung in Fig. 1 eingesetzt.

Si/Glas-Flachsubstrate bestehen aus den identischen Materialien wie der Düsenkörper und werden aus den Ausgangsmaterialien des Düsenkörpers, sprich einem Glas-Wafer (Borosilikatglas mit glatter Oberfläche gemäß dem "Float"-Verfahren) und einem Silizium-Wafer (111), zugeschnitten. Sie haben die Abmessungen eines herkömmlichen Objektträgers (26mm x 75mm) und dienen als Bezugsmaterial, da der verwendete Düsenkörper für viele Charakterisierungsversuche schwer zugänglich ist.

Der für die nachfolgenden Untersuchungen verwendete Düsenkörper ist ein mikrofluides Sandwichsystem, bestehend aus einem 2,05 x 2,55 $mm^2$ mikrostrukturierten Siliziumchip, der auf einer 0,5 mm dicken Glasplatte (hier ein Borosilikat-Glas, das mit dem so genannten "Float"-Verfahren hergestellt wird) gebondet ist. Die innere Mikrostruktur des verwendeten Düsenkörpers besteht aus einem Einlassbereich mit einer Einlassöffnung, einem Zick-Zack-Feinfilter, einer säulenartigen Mikrostruktur 7 und dem vorderen Düsenbereich. Die Ätztiefe auf dem Silizium-Chip beträgt 7,0 $\mu$m. Der Abstand der beiden Auslassöffnungen 3 beträgt 50 $\mu$m.

Während des Auslösens strömt eine flüssige Lösung oder Dispersion mit sehr hohem Druck durch den Einlassbereich in die Mikrostruktur ein. Im Zick-Zack-Feinfilter können die Feinfilterstrukturen mit einer Durchlassbreite von 3 $\mu$m größere Partikel zurückhalten um ein Verstopfen der Auslasskanäle durch solche größeren Partikel zu verhindern. Die Auslasskanäle haben eine Länge von 40 $\mu$m und eine Breite von 8 $\mu$m. Die Inhalationslösung verlässt den Düsenkörper durch die beiden vorderen Düsenauslasskanäle. Die Aerosolgenerierung geschieht außerhalb des Düsenkörpers, durch Impaktion der zwei generierten Flüssigkeitsstrahlen.

Die Struktur des Düsenkörpers stellt ein rigides System dar, das die korrekte Impaktion der beiden Flüssigkeitsstrahlen gewährleistet. Die beiden Flüssigkeitsstrahlen impaktieren nach Austritt aus den Düsenkanälen in einem Winkel von 90° miteinander. Bei korrekter Impaktion bildet sich eine sogenannte Impaktionsscheibe, an deren Grenzfläche das feine Aerosol entsteht. Zu den kritischen Parametern der Aerosolbildung zählen u.a. die Strömungsgeschwindigkeit (ca. 100 m/s) und der Impaktionswinkel.

Die Funktion der im Rahmen der hier dargestellten Versuche Oberflächenmodifizierten Düsenkörper wird in Einbausituation anhand von SMI-Zerstäubern in Ausgestaltung gemäß Fig. 3 und 4 geprüft.

### 1.1. Präparation von oberflächenfunktionalisierten Düsenkörpern und Si/Glas-Flachsubstraten durch Flüssigbeschichtung ("Solution-Coating")

Der Beschichtungsprozess startet mit der Aktivierung der Silizium bzw. GlasOberflächen. Die Aktivierung reinigt die Oberfläche von anhaftendem Schmutz und erhöht durch Oxidation die Anzahl an freien reaktiven Silanol-Gruppen auf der Oberfläche. Die reaktiven Silanol-Gruppen auf der Oberfläche sind in der Lage mit funktionellen Silanen zu reagieren. Untersucht werden Modifizierungsreagenzien auf Basis verschiedener Chloralkylsilane und Alkylalkoxysilane, die nach der Reaktion ein stabiles Siloxangerüst auf der Oberfläche ausbilden.

Die Qualität der Oberflächenbeschichtung auf Si/Glas-Flachsubstraten wird mittels statischer Kontaktwinkelmessung und über Schichtdickenmessung via spektroskopischer Ellipsometrie bestimmt. Für die Mikrostruktur, sprich den Düsenkörper, kann der sogenannte Kapillaritätstest durchgeführt werden.

#### 1.1.1 Probenvorbereitung Düsenkörper

Die Funktionalisierung von Düsenkörpern wird in PEEK-Trays durchgeführt. PEEK (Polyetheretherketon) ist ein sehr inertes Material und eignet sich hervorragend für diese Anwendung. Eine Funktionalisierung in Trays ist für Düsenkörper notwendig, da die sehr kleinen Düsenkörper andernfalls nicht in den Prozessbecken zu handhaben wären. Das Tray besitzt eine Reaktionskammer, die Platz für ca. 50 bis 75 Düsenkörper bietet und mittels eines Deckels verschlossen werden kann. Unterhalb der Reaktionskammer kann zusätzlich ein Rührfisch positioniert werden, der eine Durchmischung der Kammer gewährleistet. Sowohl der Deckel als auch die Reaktionskammer sind mit kleinen Poren versehen, so dass ein Durchströmen der Kammer mit Lösung ermöglicht wird.

Vor der Beschichtung werden die Düsenkörper mittels eines Vakuumsaugers in die Reaktionskammer befördert. Da die Düsenkörper bereits vorgereinigt sind, müssen vor der Aktivierung keine speziellen Reinigungsschritte an dem Bauteil vorgenommen werden, wie es für die Silizium- oder Glasflachsubstrate der Fall ist.

### 1.1.2 Probenvorbereitung Flachsubstrate

Die Silizium- und Glasflachsubstrate haben nicht die Reinigungsschritte wie der Düsenkörper durchlaufen, weswegen diese vor der Aktivierung noch einmal gründlich mit Wasser und Isopropanol gespült werden müssen. Die Substrate sind auf die herkömmlichen Abmessungen eines Objektträgers zugeschnitten und werden ebenfalls in einem inerten Tray, das ca. 20 Substraten Platz bietet, beschichtet. Die Si-Substrate sollten zeitnah vor der Funktionalisierung gestrippt, d. h. einer HF-Behandlung unterzogen, worden sein, was einen einheitlichen $SiO_2$-Film auf der Oberfläche gewährleistet. Beim sogenannten "strippen" der Silizium-Substrate wird die natürliche Oxid-Schicht auf der Oberfläche entfernt. Nach der Entfernung wächst der Oxidfilm langsam wieder auf und generiert einen einheitlichen dünnen Oxidfilm.

Für das "strippen" wird eine 6%ige Fluorwasserstofflösung angesetzt, in welcher die Silizium-Substrate mindestens 20 Minuten verweilen sollten. Nach der Entnahme werden die Si-Substrate gründlich mit Wasser abgespült und für die Beschichtung in die Substrat-Trays sortiert.

Die verwendeten Glas-Substrate weisen eine durch das sogenannte "flammpolieren" geglättete Seite ("fire-polished side") und eine im "Float"-Verfahren erzeugte, extra-glatte Seite auf. Zur besseren reproduzierung der Messergebnisse wird bei den hier dargestellten Versuchen bei den Glas-Substraten vor dem Prozess-Start die "Fire-polished side" mit einem Diamant-Ritzer markiert. Dies ist die Seite, auf der im späteren Verlauf die ellipsometrischen Messungen zu erfolgen haben.

### 1.1.3 Übersicht über die Prozessschritte der Oberflächenfunktionalisierung

Der Prozess startet mit der Reinigung der Oberflächen von anhaftendem Schmutz. Anschließend wird die gereinigte Oberfläche oxidativ aktiviert. Nach einem Spülschritt, in welchem die Aktivierungslösung von den Düsenkörpern bzw. Substraten abgewaschen wird, kann der eigentliche Beschichtungsprozess starten. Wird die Beschichtung mit einem Alkyltrialkoxysilan durchgeführt, muss die Beschichtungsreagenz über einer Precursorspaltung in die aktive Silanol-Komponente überführt werden. Nach der Beschichtung werden die Proben getrocknet und anschließend in einem Ofen bei 120 °C getrocknet.

#### 1.1.3.1 Reinigung und Aktivierung von Düsenkörpern und Si/Glas-Flachsubstraten

Der Beschichtungsprozess beginnt üblicherweise mit der Reinigung und Aktivierung der Silizium- und Glas-Oberflächen. Die Reinigung und Aktivierung der Proben erfolgt in einem Arbeitsschritt. Die folgenden Reinigungs- bzw. Aktivierungslösungen werden für Düsenkörper und Si/Glas-Flachsubstrate in den folgenden Untersuchungen verwendet.

##### 1.1.3.1.1 NaOH Lösung (stark alkalische Aktivierung)

Die Proben werden in eine 25%ige Natronlauge gegeben und gerührt. Anschließend werden diese gründlich mit viel Wasser abgespült. Die Methode ist in den meisten Fällen sehr gut anwendbar, führt aber relativ oft zu Trübungen auf der Glas bzw. Silizium-Oberfläche, da die Oberfläche bereits angeätzt wird (Glaskorrosion). Im Folgenden wird diese Methode daher nur für Vorversuche verwendet, da eine Trübung auf dem Düsenkörper aufgrund der optischen Qualitätskontrolle nicht akzeptabel ist.

##### 1.1.3.1.2 RCA-Lösung (RCA = Radio Corporation of America)

Bei der nachfolgenden RCA-Lösung genannten Aktivierungslösung handelt es sich um die Standard Clean 1 -Lösung (SC-1-Lösung), welche für das RCA-Verfahren zur Reinigung von Silizium-Wafern entwickelt wurde. Die Proben werden in eine Lösung aus $H_20$:$NH_3$ (aq. 25%):$H_20_2$ (aq. 30%) in einem Volumenverhältnis von 5:1:1 bei 70 °C gegeben und gerührt. Die Si/Glas-Substrate werden dabei konstant 20 Minuten gerührt, während Düsenkörper 1 Stunde lang abwechselnd mit Ultraschall behandelt (je 20 Minuten) und gerührt (je 10 Minuten) werden. Anschließend werden die Substrate oder Düsenkörper gründlich mit viel Wasser gespült. Bis zum Prozessbeginn werden die Substrate oder Düsenkörper in Wasser gelagert.

##### 1.1.3.1.3 Piranha Lösung (saure oxidative Aktivierung)

Die Proben werden in eine Lösung aus $H_2SO_4$ (conc.):$H_20_2$ (aq. 30%) in einem Volumenverhältnis von 7:3 gegeben und bei 70 °C gerührt. Die Si/Glas-Substrate werden dabei konstant 20 Minuten gerührt, während Düsenkörper 1 Stunde lang abwechselnd beschallt (je 20 Minuten) und gerührt (je 10 Minuten) werden. Anschließend werden die Proben gründlich mit Wasser abgespült und können bis zur Beschichtung in Wasser gelagert werden.

**1.1.3.2 Oberflächenfunktionalisierung von Silizium- und Glasoberflächen mit Alkyltrialkoxysilanen**

Die Trialkoxysilane werden in einer alkoholischen Lösung angesetzt und müssen als Precursor-Verbindung zunächst proteolytisch in die aktive Silanol-Komponente gespalten werden. Exemplarisch kann diese Reaktion der folgenden Gleichung 1 entnommen werden. Die Silanol-Verbindung entsteht üblicherweise innerhalb von fünf Stunden, wie in Gleichung 1 für das Beispiel 1H,1H,2H,2H-Tridecafluorooctyltriethoxysilan (Beispiel für verwendete Substanz: Dynasylan® F8261 von der Firma Evonik) aus der Ausgangsverbindung angegeben.

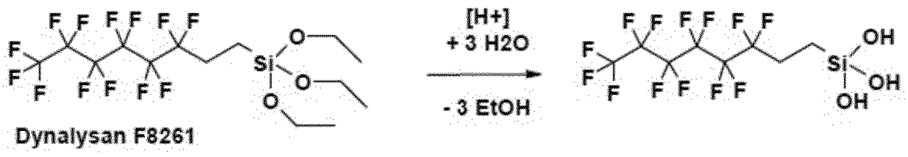


(1)

Die aktive Silanol-Komponente assoziiert nun an die aktivierte Oberfläche und bindet nach dem Tempern kovalent.

Während der Funktionalisierung wird die Lösung ständig gerührt. Im Gegensatz zu den Substraten werden Düsenkörper während der Funktionalisierung in festgelegten Zeit-Intervallen mit Ultraschall behandelt. Nach der Funktionalisierung werden die Proben aus der Lösung entnommen und für 1 Stunde an der Luft getrocknet. Nach der Trocknung werden die Proben für eine Stunde bei 120 °C im Ofen getempert. Nach dem Tempern werden die Substrate vorsichtig mit wenig Isopropanol abgespült. Düsenkörper können ohne einen weiteren Reinigungsschritt verwendet werden.

**1.1.3.2.1 Ablauf der Oberflächenbeschichtung mit Alkyltrialkoxysilanen in einer Isopropanol-Wasser Mischung**

**1.1.3.2.1.1 Hydrolyse der Ausgangsverbindung**

Es wird eine 0,1 - 1,0 %ige (v/v) Lösung des Silans in einer sauren Alkohol/Wasser-Mischung (2-Propanol/ $H_2O/HCl_{conc}$ 89,8:10:0,2) angesetzt und für mindestens fünf Stunden bei Raumtemperatur gerührt. Die Verbindung ist nach fünf Stunden verwendbar und muss innerhalb 24 Stunden verbraucht werden.

**1.1.3.2.1.1 Oberflächenfunktionalisierung**

Die Proben werden in die Funktionalisierungslösung gegeben und während der Beschichtung ständig gerührt. Düsenkörper werden in definierten Zeitintervallen während der Beschichtung wiederholt mit Ultraschall behandelt. Nach der Funktionalisierung werden die Proben aus der Flüssigkeit entnommen und das Tray für die Substrate bzw. das Tray für die Düsenkörper zum Trocknen auf ein Zellstofftuch gegeben. Die Proben werden für eine Stunde an der Luft getrocknet.

**1.1.3.2.1.2 Kondensation**

Die Proben werden in einem Ofen bei 120 °C getempert. Anschließend werden die Substrate mit Isopropanol vorsichtig gewaschen. Düsenkörper können nach dem Tempern ohne einen weiteren Reinigungsschritt verwendet werden. Die fertigen Düsenkörper und Substrate können unter Laborbedingungen gelagert werden.

**1.1.3.3 Oberflächenfunktionalisierung von Silizium- und Glasoberflächen mit Chloralkylsilanen**

Die Oberflächenfunktionalsierung mittels Chloralkylsilanen basiert auf der Kondensationsreaktion zwischen der freien Silanolgruppe auf der Oberfläche mit der Chlor-Funktion des funktionellen Alkylsilans.

Chlorsilane sind sehr feuchtigkeitsempfindlich und ihre Reaktionen müssen in trockenen, unpolaren Lösungsmiteln wie z.B. Toluol, Tetrachlormethan oder Alkanen, wie z.B. Hexan, durchgeführt werden.

**1.1.3.3.1 Schematischer Ablauf der Oberflächenbeschichtung mit Alkylchlorsilanen in Toluol**

Die Aktivierung der Oberfläche von Substraten und Düsenkörpern wird wie zuvor beschreiben durchgeführt.

#### 1.1.3.3.1.1 Trocknung des Lösungsmittels

Das Toluol wird über Molekularsieb (Typ 4 A) für mindestens 12 Stunden getrocknet. Das Verhältnis ist 10 g Molekularsieb auf 1 Liter Lösungsmittel.

#### 1.1.3.3.1.2 Oberflächenfunktionalisierung

Die Proben werden in 0,07 bis 0,7 molaren Lösungen des Chlorsilans unter ständigem Rühren beschichtet. Düsenkörper werden in definierten Zeitintervallen während der Beschichtung wiederholt mit Ultraschall behandelt. Nach der Funktionalisierung werden die Proben aus der Flüssigkeit entnommen und das Tray für die Substrate und das Tray für die Düsenkörper werden zum Trocknen auf ein Zellstofftuch gegeben. Die Proben werden für eine Stunde an der Luft getrocknet.

#### 1.1.3.3.1.3 Kondensation

Die Proben werden nach der Trocknung in einem Ofen bei 120 °C getempert. Anschließend werden die Substrate mit Isopropanol vorsichtig gewaschen. Düsenkörper können nach dem Tempern ohne einen weiteren Reinigungsschritt verwendet werden. Die fertigen Düsenkörper und Substrate können unter Laborbedingungen gelagert werden.

## 1.2 Kategorisierung von Düsenkörpern nach der Beschichtung

Die beschichteten Düsenkörper werden in die folgenden Düsenkategorien I bis IV eingeteilt.

#### Kategorie I

Der Düsenbereich, der Zick-Zack-Filter und die Stützstruktur des Düsenkörpers sind weitestgehend frei von Rückständen.

#### Kategorie II

Der Düsenbereich ist frei, Zick-Zack-Filter und Säulenstruktur besitzen allerdings Rückstände.

#### Kategorie III

Ein Düsenkanal besitzt Rückstände bzw. ist komplett verstopft.

#### Kategorie IV

Zwei Düsenkanäle besitzen Rückstände bzw. sind komplett verlegt.

## 1.3. Provozierung von Düsenverlegungen am Düsenkörper

### 1.3.1 Provokationslösungen

Sogenannte Provokationslösungen sollen das Phänomen der Düsenverlegung oder den Effekt des Nicht-Treffens der Strahlen ("Jet-Divergency") möglichst oft erzeugen. Sie sind daher hinsichtlich ihrer Parameter so gewählt, dass sie während eines Versuches sehr viele Düsenverstopfungen hervorrufen werden. Dies ist notwendig, um eine ausreichende Anzahl an Düsenverstopfungen mit möglichst kleinen Mustermengen zu generieren, so dass sehr gut beurteilt werden kann, ob eine Modifikation einen Einfluss auf das Auftreten von Düsenverstopfungen hat.

### 1.3.2 Provozierung von Düsenverlegungen im In-Use Betrieb eines Zerstäubers mit DJI-Düse (=Provokationsversuche)

#### 1.3.2.1 Das Phänomen der Düsenverlegung

In einem SMI-Inhalator mit DJI-Düse basiert die Aerosolerzeugung auf der Impaktion von zwei Mikroflüssigkeitsstrahlen. Diese werden durch zwei rechteckige, im 90° Winkel zueinander ausgerichtete Düsenauslasskanäle im Düsenkörper generiert. Für die Sprayperformance ist die korrekte Impaktion der beiden Flüssigkeitsstrahlen von entscheidender Bedeutung. Provozierte, partikuläre Ablagerungen in einem oder auch in beiden Düsenkanälen können die korrekte Impaktion durch Strahlablenkung stören und zu Spraybildveränderungen und Abweichungen in der Feinpartikelfraktion führen. Unter der Bezeichnung "Jet-Divergency" werden in dieser Arbeit Spraybilder der Gruppe III verstanden, die einen veränderten Feinpartikelanteil aufweisen bzw. bei denen allenfalls nur ein kleiner Teil der mit der Betätigung des Zerstäubers auszubringenden Flüssigkeitsmenge tatsächlich als Aerosol ausgebracht wird. Eine umfassendere Definition des Begriffs lautet wie folgt: Das Phänomen der "Jet-Divergency" (Strahlablenkung durch Düsenverlegung) ist eine reversible oder irreversible, teilweise oder komplette Verlegung / Verstopfung von einem oder beider Düsenkanäle des Düsenkörpers, die durch Partikel in der Inhalationslösung verursacht wurde und zu Sprays mit verändertem Feinpartikelanteil führt.

**1.3.2.2 Zuordnung von Spraybildern in Provokationsversuchen**

Provokationsversuche ermöglichen die Bewertung verschiedener Einflussfaktoren auf das Phänomen der Düsenverlegung. Das Versuchsdesign ist so ausgelegt, dass es zu einer möglichst hohen Anzahl verlegter Inhalatordüsen über die Versuchsdauer kommt. Hierfür werden die Zerstäuber einmal täglich abgehubt und das Spraybild visuell bestimmt. Ein solches Versuchsszenario wird im Folgenden als "In-Use-Versuch" bezeichnet, da es hierbei die tägliche Verwendung eines Zerstäubers durch einen Anwender - zumindest in Bezug auf die Anwendungshäufigkeit - nachgestellt wird.

Über die gesamte Versuchszeit wird das Spraybild (SB) des Zerstäubers gemäß zuvor festgelegter Kategorisierung dokumentiert und anschließend ausgewertet. Die einzelnen Spraybilder werden hierfür in den drei folgenden übergeordneten Gruppen zusammengefasst: Normale ("Gut") Sprays (Gruppe I), Spraybildanomalien (Gruppe II) und Sprays mit verändertem Feinpartikelanteil (Gruppe III = "Jet-Divergency"). Der Einfluss eines Versuchsparameters auf die Inzidenz wird durch den Vergleich mit einer Referenz ersichtlich.

Fig. 5 zeigt Beispiele für ein Spraybild der Gruppe I. Solche Sprays weisen eine dominante, ungeteilte Sprühwolke (allenfalls mit nur kleinem Seitenspray) auf. Normalerweise ist die Sprühwolke symmetrisch.

Fig. 6 zeigt Beispiele für ein Spraybild der Gruppe II. Die Spraybilder dieser Gruppe zeigen geteilte Sprühwolken. In Fig. 6 ist eine symmetrisch geteilte Sprühwolke zu sehen, asymmetrisch geteilte Sprühwolken oder mehrfach geteilte Sprays gehören aber ebenfalls zu dieser Spraybild-Gruppe. Hierbei ist zu beachten, das die Erkennung der Aufteilung der Sprühwolke abhängig vom Blickpunkt des Betrachters sein kann. Bei einer (automatischen) Bilderfassung der Sprühwolke wird daher vorzugsweise mit zwei Kameras gearbeitet, die in einem 90° Winkel zur Achse der Aerosolwolke ausgerichtet und simultan mit dem Auslösen des Gerätes die Bilder der Aerosolwolke aus zwei verschiedenen Blickpunkten aufnehmen.

Spraybilder der Gruppe III, für die (aufgrund der verminderten Aerosolbildung) der Feinpartikelanteil gegenüber den Sprays der Gruppen I und II deutlich verändert bzw. reduziert ist, sind nicht mit einfachen Bildaufnahmesystemen erkennbar. Bei spezieller Ausleuchtung ist es jedoch visuell möglich, die aus der Düse z.B. in ein oder zwei sehr dünnen Strahlen austretende Flüssigkeit zu erkennen. Bei Spraybildern dieser Gruppe kommt es durch die Strahlablenkung nicht zur Impaktion der Flüssigkeitsstrahlen (und somit nicht zur Ausbildung der DJI-Düsen-typischen Sprühwolke), es liegt also eine sogenannte "Jet-Divergency" vor.

**1.3.2.3 Spraybildbeurteilung**

Die Spraybildbeurteilung erfolgt visuell (in einer händisch durchgeführten Versuchsreihe) oder mit Hilfe von Kamera-Techniken (in Versuchsreihen, die mit Hilfe eines Abhubroboters durchgeführt werden) und wird zu jedem Hub des Gerätes durchgeführt. Das am Versuchstag ermittelte Spraybild wird zusammen mit der Hubnummer dokumentiert. In der Auswertung werden die dokumentierten Spraybilder den einzelnen Gruppen zugeordnet und der Verlauf des Spraybildes gegen die Zeit betrachtet.

Die Lagerung der Zerstäuber erfolgt während eines Provokationsversuches im Labor unter konstanter Temperatur und Luftfeuchte. Die Spraybildbeurteilung wird für alle Geräte innerhalb eines Versuches jeweils am selben Tag durchgeführt.

Erstmalig in Betrieb genommene Geräte müssen zum Versuchsstart geprimt werden.

Für die anschließende Spraybildbeurteilung wird das geprimte Gerät bei einem 45° Winkel in eine Absaugvorrichtung abgehubt. Die Distanz zur Absaugapparatur beträgt dabei ca. 20 - 30 cm, so dass die Aerosolwolke deutlich sichtbar bleibt. Eine Kontrasterhöhung kann durch Benutzung einer schwarzen Kartonage als Untergrund und einer Kaltlichtquelle erzielt werden. Die Einteilung der Spraybilder erfolgt gemäß der zuvor genannten Gruppen.

Die Spraybildbeurteilung kann auch automatisiert mit Hilfe eines Abhubroboters durchgeführt werden. Der Roboter wird dazu mit vorgeprimten Zerstäuber bestückt und ein Roboterarm koordiniert anschließend das Spannen und das Auslösen des Inhales. Zwei CCD-Kameras sind in einem 90° Winkel zur Achse der Aerosolwolke ausgerichtet und nehmen simultan mit dem Auslösen des Gerätes die Bilder der Aerosolwolke auf. Die Spraybildbeurteilung erfolgt

anhand der aufgezeichneten Bilder manuell durch einen Bearbeiter.

### 1.3.3 Versuchslayout eines Standard-Provokationsversuches

Das Standard-Versuchslayout für einen Provokationsversuch ist wie folgt:

**Inhalatoranzahl:** 30-150 Stück (abhängig von getesteten Einflussparametern)
**In-Use Modus:** 1x1 (1 Hub/Tag) oder 1x2 (2 Hübe/Tag)
**Prüfpunkt:** Spraybild (Spezifikation gemäß Spraybild-Katalog)
**Versuchsdauer:** 28-120 Tage; analog zu einer 1-monatigen, bzw. 4-monatigen Patientenanwendung
**Formulierung:** Ethanol/Wasser 90/10 (v/v), pH-Wert ≤ 2,0
**Referenz:** 30-75 Stk.

### 1.3.3.1 10-Tage-Durchschnitt und Provokationsreferenz

Die einzelnen Spraybildverläufe in einem Provokationsversuch können über die Versuchszeit großen Fluktuationen unterworfen sein. Die Anzahl an Geräten mit verstopften Düsenist zeitabhängig und nimmt nach einiger Zeit mit linearem Charakter zu und erreicht häufig anschließend einen "Steady State" um den das System streut. Eine exakte, endgültige Rate zu bestimmen, ist daher nicht immer möglich. Aus diesem Grund wird, zusätzlich zu den Spraybildverläufen, die mittlere Rate an Gruppe III Sprays für jeden Versuchsarm über die letzten zehn Versuchstage ermittelt (d.h. Bestimmung der "10-Tage-Durchschnittsrate"). Wie stark der Einfluss eines Untersuchungsparameters auf die Inzidenz an Gruppe III Sprays tatsächlich ist, lässt sich nur durch den Vergleich mit einer internen Provokationsreferenz herausfinden. Eine Provokationsreferenz ist dabei eine Gruppe an Inhalatoren, denen das zu untersuchende Merkmal, z.B. eine Beschichtung der Düse, fehlt. Sie stellen den Ursprungszustand des Gerätes dar und erlauben damit eine bewertende Aussage über eine Modifikation.

## 1.4. Instrumentelle Analytik

### 1.4.1 Kontaktwinkelmessung

Die Qualität der Beschichtung auf Si/Glas-Flachsubstraten kann anhand statischer Kontaktwinkelmessung beschrieben werden. Die Ergebnisse erlauben eine Aussage über das Vorhandensein der Beschichtung und deren Homogenität. Die Kontaktwinkelmessungen wurden mit einem handelsüblichen Messgerät (hier: Typ DSA 10 MKR der Firma Krüss GmbH) mit zugehöriger Steuerungstechnik und Steuerungssoftware (mit Auswertung nach dem Young-Laplace-Modell) durchgeführt.

### 1.4.1.1 Grundlagen

Für die Bewertung, inwieweit eine Flüssigkeit eine Oberfläche benetzen kann, kann der Kontaktwinkel herangezogen werden. Der Kontaktwinkel wird an der Kontaktlinie Festkörper/Flüssigkeit/Gasphase zwischen Festkörper und Flüssigkeit gebildet und gemessen. Inwieweit hierbei eine Flüssigkeit eine Oberfläche benetzen kann, hängt vom Verhältnis der Oberflächenspannungen Festkörper/Flüssigkeit ($\gamma_{f/fl}$) Festkörper/Luft ($\sigma_{f/g}$ sowie Flüssigjkeit/Luft ($\sigma_{fl/g}$) ab. Die Grenzflächenspannungen befinden sich am Tropfen in einem Gleichgewicht, welches durch die Young-Gleichung 2 ausgedrückt werden kann.

$$\sigma_{f/g} = \gamma_{f/fl} + \sigma_{fl/g} \times \cos\theta \qquad (2)$$

Je kleiner der Kontaktwinkel ist, desto besser ist die Benetzung. Bei einem Kontaktwinkel von 0° spricht man von einer vollständigen Benetzung, ein Kontaktwinkel von 180° wiederum steht für eine ausbleibende Benetzung. Die Einstellung des durch die Young Gleichung beschriebenen Gleichgewichtes ist dabei zeit- und temperaturabhängig.

### 1.4.1.2 Statische vs. dynamische Kontaktwinkelmessung

Bei der statischen Kontaktwinkelmessung wird im Gegensatz zur dynamischen Kontaktwinkelmessung die Kontaktfläche zwischen Festkörper und Flüssigkeit während der Messung nicht verändert. Auf einer idealen, chemisch und topologisch homogenen Festkörperoberfläche würde eine reine Flüssigkeit in einer gesättigten Dampfphase einen

gleichen dynamischen und statischen Kontaktwinkel besitzen. Dieser Zustand wird in der Young-Gleichung beschrieben. Der Kontaktwinkel kann sich jedoch zeit- und ortsabhängig ändern. Diesem Phänomen wird begegnet, indem stets sofort nach der Tropfenaufgabe der Kontaktwinkel gemessen wird.

**1.4.1.3 Beschreibung der Kontaktwinkelmessung**

Die beschichteten Silizium- bzw. Glassubstrate werden vor jeder Messung mittels ölfreier Luft von anhaftendem Staub befreit und auf dem Probentisch für die Messung bereitgelegt. Die Siliziumsubstrate werden hierfür mit der matten Seite nach unten positioniert. Mittels einer automatisiertem Mikroliterspritze wird ein Tropfen mit einem Volumen von 8 µl auf dem Substrat abgelegt und sofort mittels der Steuerungssoftware bzw. des Steuerungsprogramms vermessen. Für alle Kontaktwinkelmessungen wird doppelt-entionisiertes Wasser und/oder n-Dodecan verwendet. Alle in dieser Arbeit angegebenen Kontaktwinkelwerte sind Durchschnittswerte, die aus der Messung von in der Regel fünf Tropfen resultieren. Jeder Tropfen wird dabei zehnmal durch das Steuerungsprogramm analog dem Young-Laplace Model gefittet und vermessen. In der Regel wird jede Beschichtung mit 3 - 5 Substraten durchgeführt, wobei jedes Substrat auch für Kontaktwinkelmessung herangezogen wird.

**1.4.2 Überprüfung des Dosierungsverhalten anhand der Delivered Mass (DM) und Metered Mass (MM)**

Die dosierte Formulierungsabgabe durch einen Zerstäubers, d.h. die abgemessene Masse an Formulierung (im Folgenden "Metered Mass" (MM)) wird durch die baulichen Abmessungen des Inhalators und die Dichte der Formulierungslösung bestimmt. Es handelt sich hier um die Masse der Formulierungslösung, welches auf eine Betätigung des Zerstäubers hindurch die freigegeben wird. Die Metered Mass (MM) wird gravimetrisch bestimmt.

In der Regel bleibt ein kleiner Teil der abgemessenen Masse nach der Auslösung als Rest an der Düse zurück. Es ist nicht möglich dieses gänzlich zu verhindern, da es bei dem verwendeten Zerstäubungsprinzip basierend auf einer von zwei Flüssigkeitsstrahlen gebildeten Impaktionsscheibe immer einen Rückstreubereich gibt. Daher ist die tatsächlich als Sprühwolke vom Gerät abgegebene Masse (im Folgenden "Delivered Mass" (DM)) gewöhnlich kleiner als die Metered Mass (MM). Die Delivered Mass wird ebenfalls gravimetrisch bestimmt.

**1.4.3 Ellipsometrie**

**1.4.3.1 Instrumenteller Aufbau**

Die Ellipsometrie ist eine Messmethode, die bereits im 19. Jahrhundert angewendet wurde. Einige essentielle Komponenten eines Ellipsometers stehen bereits seit sehr langer Zeit zur Verfügung, während wiederum andere Komponenten aus modernen Ellipsometern erst kürzlich entwickelt wurden.

In der spektroskopischen Ellipsometrie (SE) ermöglicht eine Weißlichtquelle mit einem Monochromator die ellipsometrischen Messungen bei verschiedenen Wellenlängen. Der Monochromator kann dabei vor dem Polarisator oder aber nach dem Analysator lokalisiert sein. Darüber hinaus gibt es spektrokopische Ellipsometer mit einem rotierenden Kompensator (vgl. Irene, E.A. and H.G. Tompkins, Handbook of Ellipsometry. 2005: William Andrew Pub).

Die ellipsometrischen Messungen in dieser Arbeit wurden mit einem spektroskopischen Ellipsometer mit rotierendem Kompensator durchgeführt. Nähere Informationen zu den verwendeten Geräten können Tabelle 1 entnommen werden

**Tabelle1: Verwendetes Gerät bei der Ellipsometrie.**

| | |
|---|---|
| Ellipsometer | Alpha-SE® mit rotierendem Kompensator der Fa. J.A Woollam Co., Inc. (USA) |
| Spektral bereich | 390-900 nm |
| Einfallswinkel | 65°, 70° und 75° sowie Transmission bei 90° |
| Steuerungssoftware | Complete EASE von der Fa. J.A Woollam Co., Inc. (USA) |

**1.4.3.2 Beschreibung der ellipsometrischen Messungen**

Vor jeder Messung werden die Silizium- bzw. Glassubstrate zunächst mit ölfreier Luft von anhaftendem Staub befreit. Anschließend werden die Substrate mit einer Wafer-Pinzette vorsichtig auf dem Probentisch des Ellipsometers abgelegt. Dabei ist darauf zu achten, dass die Siliziumsubstrate mit der matten Seite nach unten positioniert werden. Bei den transparenten Glas-Substraten (Borofloat® 33, Nexterion) wird vor der Positionierung auf dem Probentisch die

"tin-side" mit Scotch-Tape abgeklebt. Zu identifizieren ist diese an einer im Vorfeld eingeritzten Markierung auf dem Glas, die vor dem Beschichtungsprozess mittels eines Diamantritzers angebracht wird.

Das Abkleben der "tin-side" bedingt sich aufgrund von zwei Aspekten. Zum einen soll das Aufkommen von Rückseitenreflexion bei dem transparenten Glassubstrat verhindert werden und die Schichtdickenmessung sollte aufgrund der Zinnrückstände nicht auf der "Float-Seite" des Glases durchgeführt werden, da es die Auswertung verkompliziert. Bei dem verwendeten Glas handelt es sich um Float-Glas, dass während seiner Herstellung auf ein Zinnbad gegossen wird, woraus sich für eine Glas-Seite eine starke Zinnkontamination ergibt.

Nach der Positionierung der Flachsubstrate auf dem Probentisch kann das Tisch-Vakuum eingeschaltet werden (verbessert planparallele Orientierung) und die Messung durchgeführt werden. Gemessen wird bei den Winkeln 65°, 70° und 75°. Nach der Messung werden die Rohdaten mit der entsprechenden Methode für Silizium bzw. Glas mittels der Auswertefunktion der Software Complete EASE ausgewertet.

### 1.4.3.3 Entwicklung einer Auswertemethode für die Schichtdickenmessung auf Silizium- und Glasflachsubstraten

#### 1.4.3.3.1 Auswertemodell für Silizium-Substrate

Für die ellipsometrischen Messung der Schichtdicke auf Siliziumsubstraten wird folgendes Schicht-Modell angenommen: Siliziumsubstrat/Natives Oxid/Self-Assembled Monolayer

Silizium bildet an seiner Oberfläche eine native Oxidschicht, die sich auch nach dem Entfernen (strippen) mit HF in relativ kurzer Zeit wieder aufbaut. Auf diese Oxidschicht orientiert sich während der Beschichtung der "Self-Assembled Monolayer" der reaktiven Silanverbindung.

Dieses Schichtmodell kann mit der zum verwendeten Messgerät gehörenden Software (Compleate EASE) modelliert werden, sofern die notwendigen optischen Konstanten und Schichtdicken vorhanden sind. Die optischen Konstanten für reines Silizium und für die native Oxidschicht sind bekannt und sind in der Datenbank der Auswertesoftware hinterlegt. Die optischen Konstanten für den zu generierenden SAM-Layer können, wenn vorhanden, der Literatur entnommen werden oder müssen selbst bestimmt werden. Für die Schichtdickenbestimmung des "Self-Assembled Monolayers" wird die Cauchy-Dispersionsgleichung in der Auswertesoftware herangezogen. Sie beschreibt den Brechungsindex n als Funktion der Wellenlänge $\lambda$ und ist geeignet für die Auswertung ultra-dünner, transparenter, nicht absorbierender Filme. Die Schichtdicke der nativen Oxidschicht nach RCA-Behandlung wurde experimentell bestimmt und strebt einem Maximum von 1,5 nm zu.

Das Versuchslayout war wie folgt:

**Substrat:** je Testbedingung zwei Silizium-Flachsubstrate
**HF-Behandlung:** 20 Minuten vor der RCA-Behandlung
**RCA-Behandlung [min]:** 0, 5, 10, 20, 60, 90

Die RCA-Reinigung beläuft sich im Standard-Beschichtungs-Verfahren auf 60 - 90 Minuten. Für die Schichtdickenmessungen der SAMs auf Si-Substraten muss daher eine Siliziumoxidschicht von 1,5 nm herangezogen werden.

Die Kennzeichnung der Schichtdicke der nativen Oxidschicht ist wichtig um später die Schichtdicke der "Self-Assembled Monolayer" auf Si-Substraten zu ermitteln.

#### 1.4.3.3.2 Auswertemodell für Glas-Substrate

Für die Messung der Schichtdicke auf Glas-Substraten wird folgendes Schicht-Modell angenommen: Glas-Subtstrat/Self-Assembled Monolayer

Das Model arbeitet ohne eine Oxidschicht, wie es für die Siliziumsubstrate der Fall ist. Es wird angenommen, dass der Self-Assembled Monolayer direkt auf dem Glas-Substrat bindet. Neben den konkreten optischen Konstanten für das hier verwendete Glas wurde zusätzlich berücksichtigt, wie sich das Glas nach einer RCA-Behandlung verhält und die Materialkonstanten dementsprechend angepasst. Das Glas wurde mit seinen Konstanten als Material in der Software angelegt, so dass mit bekanntem Brechnungsindex für den SAM-Layer auch hier die Schichtdicke gemäß Cauchy-Dispersionsgleichung ermittelt werden kann.

### 1.4.4 Kapillaritätstest an Düsenkörpern

Weil das Innere der Mikrostruktur schwer für analytische Methoden zugänglich ist, wird der Beweis, ob ein Düsenkörper beschichtet ist anhand eines sogenannten Kapillartitätstest durchgeführt. Der Test basiert auf der spontanen Befüllung einer Kapillare mit Lösungsmitteln unterschiedlicher Polarität (in der Regel Wasser). Der Kapillaritätstest

ermöglicht es in wenigen Sekunden, auf Anwesenheit einer Beschichtung zu prüfen. Wasser benetzt als polare Flüssigkeit die Düsenkörperoberfläche eines nicht-beschichteten Düsenkörpers und dringt bei Kontakt mit dem Düsenkörper aufgrund positiver Kapillarkräfte sofort in die Mikrostruktur ein (Effekt der Kapillaraszension). Dieser Effekt lässt sich unter dem Mikroskop verfolgen und unterbleibt beispielsweise bei einem hydrophob beschichteten Düsenkörper.

Bei einer erfolgreichen hydrophoben Beschichtung setzt der Effekt der Kapillaraszension aus und eine so genannte Kapillardepression wird beobachtet. Im Falle der Kapillardepression sind die Kohäsionskräfte zwischen den Molekülen größer als die Adhäsionskräfte zur Oberfläche. Die Flüssigkeit kugelt sich ab und eine Benetzung der Oberfläche unterbleibt. Eine Flüssigkeit, welche die Kapillaroberfläche nicht benetzt, wird beim Eintauchen der Kapillare aus dieser heraus getrieben.

#### 1.4.4.1 Durchführung des Kapillaritätstest

Ein beschichteter Düsenkörper wird mit Hilfe einer PTFE-Pinzette unter dem Mikroskop so ausgerichtet, dass die innenliegende Mikrostruktur gut sichtbar ist. Anschließend wird ein mit Testflüssigkeit getränktes Wattestäbchen (Q-Tip) vorsichtig an den Eingangsbereich des Düsenkörpers heran geführt. Bei Kontakt des Q-Tips mit der Eingangsstruktur unterbleibt bei einem hydrophobisierten Düsenkörper die Befüllung der Mikrostruktur mit einer polaren Flüssigkeit wie z.B. Wasser. Als positiv-Kontrolle kann in diesem Falle der Test mit einer unpolaren Substanz wie z.B. n-Dodecan wiederholt werden. Aufgrund guter Benetzung gelingt in diesem Falle die Befüllung. Ein Düsenkörper, der mit einem perfluorierten Alkylsilan beschichtet wurde, sollte sich wiederum in diesem Falle mit einer perfluorierten Testflüssigkeit, wie z.B. Perfluoroctan, befüllen lassen.

## 2. Ergebnisse

### 2.1 Einflussparameter auf die Sprayperformance unbeschichteter Düsenkörper

Bei der Bestimmung einer geeigneten Provokationslösung wurde im Vorfeld untersucht, inwieweit sich unterschiedliche äußere Einflüsse, insbesondere der pH-Wert, auf die Sprayperformance der hier betrachteten Zerstäuber auswirken. Hierbei wurden beispielsweise ethanolische Formulierungen (90/10) mit unterschiedlichen Ansäuerungen (pH 2.0, 2.4, 2.8, 3.2, 3.5, 5.0) auf ihr Risiko, Düsenverlegungen hervorzurufen getestet.

Exemplarisch aus diesen Voruntersuchungen wird hier in Fig. 7 gezeigt, wie die Rate der auftretenden Jet-Divergencies von dem pH-Wert der Provokationslösung abhängt. Wie Fig. 7 entnommen werden kann wird ein früher und sehr starker Anstieg für die pH-Werte 2,0 und 2,4 an Gruppe III Sprays registriert. Für den pH-Wert 2,0 wird zum Versuchsende ein Wert von annähernd 70 % erzielt und für den pH-Wert 2,4 ein Wert von ca. 50 %. Beide Verläufe zeigen bereits sehr früh ab Tag 2 die ersten Gruppe III Sprays. Demgegenüber steht der Spraybildverlauf für die pH Werte 3,5 und 5,0. Hier liegt der Anteil sogar noch zum Versuchsende bei annähernd 0 %. Erst ab Tag 21 treten vereinzelt einige wenige Gruppe III Sprays auf.

Es ist anhand der Spraybildverläufe sehr deutlich erkennbar, dass die Häufigkeit eines Gruppe III Sprays mit abnehmendem pH-Wert (von 5,0 bis 2,0) stark zunimmt.

Fig. 8 zeigt die 10-Tage-Durchschnittsrate (in Versuchen über 28 Tagen)an Gruppe III Sprays ("Jet-Divergency") zum Versuchsende. Mit steigendem pH-Wert nimmt die Anzahl an Gruppe III Sprays ab. Der pH-Wert der Inhalationslösung hat daher einen maßgeblichen Anteil am Auftreten von Düsenverlegungen/Düsenverstopfungen. Je niedriger der pH-Wert der Inhalationslösung im hier getesteten Bereich ist, desto häufiger kommt es zu Abweichungen von einem Gruppe I ("Gutspray").

Insgesamt wurde aus den Vorversuchen eine geeignete Provokationslösung bestimmt: Ethanol/Wasser-Lösung im Verhältnis 90/10 (v/v) angesäuert mit HCl mit pH≤2,0 (ohne Zusatz von Wirkstoff).

### 2.2 Funktionalisierung von Flachsubstraten und Düsenkörpern

#### 2.2.1 Funktionalisierung von Silizium- und Glassubstraten

##### 2.2.1.1. Einfluss der Aktivierung

Um einen geeigneten Beschichtungsprozess für die simultane Beschichtung von Silizium und Glas festzulegen, müssen verschiedene Einflussfaktoren untersucht werden. Gerade der Aktivierung der Silizium- und Glasoberflächen kommt dabei eine zentrale Rolle zu, denn sie gewährleistet erst eine ausreichende Stabilität der Beschichtung.

In einem Versuch wird der Einfluss unterschiedlicher Aktivierungen auf die Eigenschaften der resultierenden Beschichtung untersucht. Mit den vier unterschiedlichen Aktivierungsmethoden (Piranha-Lösung, RCA-Lösungund NaOH-Lösung) werden Silizium- und Glasflachsubstrate simultan unter den gleichen Bedingungen beschichtet. Die Funktionalisierung wird anschließend mit 1H,1H,2H,2H-Tridecafluorooctyltriethoxysilan (c = 0,003 mol/Liter) durchgeführt. Geeignetes 1H,1H,2H,2H-Tridecafluorooctyltriethoxysilan wird beispielsweise von der Fa. Evonik unter der Bezeichnung Dynasylan® F8216 vertrieben. Nach der Beschichtung werden die Substrate mit 2-Propanol gewaschen und anschließend der statische Wasser-Kontaktwinkel gemessen. Ziel ist es, einen Parametersatz für jede Aktivierung zu erhalten, mit dem Wasser-Kontaktwinkel für jede Aktivierungslösung von über 100° nach der Beschichtung erhalten werden können. Ein Wasser-Kontaktwinkel oberhalb 100° indiziert eine homogene, flächendeckende Beschichtung und eine gute Hydrophobizität.

Es zeigt sich, dass mit allen untersuchten Aktivierungen Silizium/Glas-Flachsubstrate erfolgreich zu beschichten und Kontaktwinkel über 100° zu erhalten.

Es hat sich jedoch auch gezeigt, dass die Methode der NaOH-Aktivierung für den Beschichtungsprozess nur eingeschränkt geeignet ist, da sie in nicht seltenen Fällen zu Trübungen auf dem Glas führt. Dieses Phänomen ist auch unter dem Begriff Glaskorrosion bekannt. Aus diesem Grund wurde die NaOH-Aktivierung nur für Vorversuche, nicht aber für die späteren Versuche am Düsenkörper vorgesehen. Für die Qualitätskontrolle der hergestellten DJI-Düsen ist es wünschenswert, dass diese stets mikroskopierbar sind. Dies wäre bei einer Eintrübung des Glas-Anteiles des Düsenkörpers nicht mehr möglich.

### 2.2.1.1. Konzentrationseinfluss des Beschichtungsreagenzes

Der Einfluss der Beschichtungskonzentration auf den Wasser-Kontaktwinkel auf Silizium/Glas-Flachsubstraten wird analysiert. Die Silizium/Glas-Flachsubstrate werden hierfür mit einem kurz-kettigen Alkyltrialkoxysilan (Methyltrimethoxysilan = MTMS) beschichtet. Um den Einfluss der Beschichtungskonzentration zu analysieren, wird diese sukzessive erhöht. Die Charakterisierung der Substrate erfolgt im Anschluss anhand statischer Wasser-Kontaktwinkelmessung.

Die Silizium- und Glas-Flachsubstrate werden analog des bereits beschriebenen Ablaufes gemäß Kapitel 1.1 beschichtet. Die Aktivierung erfolgt anhand einer RCA-Aktivierung für 20 Minuten bei 75 °C. Beschichtet werden die Substrate für zwei Stunden ohne Ultraschall bei Raumtemperatur mit dem Funktionalisierungsreagenz Methyltrimethoxysilan (Fa. abcr GmbH, Karlsruhe, Germany) bei verschiedenen von Stoffmengenkonzentration von 0,7 mmol/Liter bis 70 mmol/Liter. Anschließend werden die Substrate für eine Stunde an Raumluft getrocknet und schließlich im Ofen für eine Stunde bei 120°C getempert. Die Ergebnisse der Untersuchungen sind in den nachfolgenden Tabellen 2 und 3 dargestellt.

**Tabelle 2: Wasser-Kontaktwinkel (mit Standardabweichung d) von mit MTMS beschichteten Glas-Flachsubstraten in Abhängigkeit von der MTMS-Konzentration**

| MTMS-Konzentration [mmol/l] | Kontaktwinkel [°] | d [°] |
|---|---|---|
| 0,7 | 67,11 | 12,45 |
| 3,5 | 87,45 | 6,03 |
| 7 | 78,32 | 4,47 |
| 14 | 83,60 | 8,13 |
| 35 | 97,39 | 8,46 |
| 70 | 103,22 | 2,68 |

**Tabelle 3: Wasser-Kontaktwinkel (mit Standardabweichung d) von mit MTMS beschichteten Silizium-Flachsubstraten in Abhängigkeit von der MTMS-Konzentration**

| MTMS-Konzentration [mmol/l] | Kontaktwinkel [°] | d [°] |
|---|---|---|
| 0,7 | 51,79 | 7,18 |
| 3,5 | 79,82 | 12,61 |
| 7 | 79,00 | 3,66 |
| 14 | 79,50 | 4,45 |

(fortgesetzt)

| MTMS-Konzentration [mmol/l] | Kontaktwinkel [°] | d [°] |
|---|---|---|
| 35 | 105,13 | 9,13 |
| 70 | 112,26 | 7,23 |

Es zeigt sich, dass der Wasser-Kontaktwinkel sowohl auf Glas als auch auf Silizium mit zunehmender Konzentration an MTMS ansteigt. Eine Konzentrationserhöhung im Bereich der niedrigen Konzentrationen hat dabei einen etwas größeren Effekt als im Bereich der höheren Konzentrationen. Das System nähert sich einem Maximum im Bereich von 110-120°. Eine weitere Erhöhung der Beschichtungskonzentration hat daher einen immer kleiner werdenden Effekt auf den Kontaktwinkel. Insgesamt lässt sich auch feststellen, dass sich ab einer Konzentration von ca. 7 mmol/l Werte mit einer moderateren Streuung ermitteln lassen. Dies spricht insgesamt für eine homogenere Oberflächenbelegung mit Beschichtungsmolekülen.

Die Daten zeigen, dass es möglich ist mit einem Beschichtungsprozess beide Substratmaterialien erfolgreich zu beschichten.

Wie sich herausstellt, erhöht sich der Kontaktwinkel mit zunehmender Konzentrationserhöhung an Beschichtungsreagenz und strebt mit höheren Konzentrationen asymptotisch einem scheinbaren Maximum entgegen. Dies erscheint plausibel, da die Oberfläche ab einem gewissen Punkt gesättigt ist, d.h. sich die Schicht durchgehend auf der Oberfläche ausgebildet hat. Dieser Effekt lässt sich gut an den Ergebnissen für Silizium und Glas ableiten. Im Bereich der niedrigen Beschichtungskonzentrationen wird bei einer geringfügigen Konzentrationserhöhung ein hoher Effekt bezüglich des Kontaktwinkels erzielt, während dieser Effekt im Bereich der höheren Konzentrationen immer geringer wird. Eine Verdopplung der Konzentration von 35 mmol/Liter auf 70 mmol/Liter erzielt hier nur noch eine weitere Erhöhung von 10° im Wasser-Kontaktwinkel. Generelle Unterschiede für Silizium oder Glas sind nicht auszumachen (die hier gestellte Anforderung der simulatn möglichen Beschichtung von Glas und Silizium wird somit erfüllt).

Anhand des Ergebnisses lässt sich auch eine optimale Mindestbeschichtungskonzentration für den Prozess ableiten. Eine optimale Mindest-Beschichtungskonzentration sollte über 30 mmol/Liter liegen, da es sich gezeigt hat, dass ab dieser Konzentration verlässliche und reproduzierbare Beschichtungen erzielt werden.

### 2.2.2 Funktionalisierung von Düsenkörpern

Die Funktionalisierung von Düsenkörpern folgt im Allgemeinen dem Protokoll gemäß Kapitel 1.1. Das äußere Erscheinungsbild von Düsenkörpern nach der Beschichtung wurde untersucht.

#### 2.2.2.1 Einfluss der Aktivierungslösung

In einem Versuch werden Düsenkörper mittels der verschiedenen Aktivierungslösungen aktiviert und anschließend mit 1H,1H,2H,2H-Tridecafluorooctyltriethoxysilan beschichtet. Anschließend werden diese über Lichtmikroskopie visuell auf Auffälligkeiten untersucht. Im Allgemeinen zeigen RCA- und Piranhaaktivierte Düsenkörper ein vergleichbares Aussehen.

Im Allgemeinen zeigen beschichtete Düsenkörper auch im Rasterelektronenmikroskop eine vergleichbare Oberfläche gegenüber nicht beschichteten Düsenkörpern und sind daher als optisch identisch anzusehen. Eine monomolekulare Schicht eines Alkylsilans kann nicht direkt durch Rasterelektronenmikroskopie nachgewiesen werden, weswegen beschichtete und unbeschichtete Düsenkörper auch nicht direkt zu unterscheiden sind. Alle beschichteten Düsenkörper zeigen einen positiven Kapillaritätstest und wurden somit erfolgreich beschichtet. Nur in äußerst wenigen Fällen kommt es zu morphologischen Artefakten während der Oberflächenfunktionalisierung. Diese Artefakte treten nur in äußerst wenigen Fällen auf, so dass typischerweise durch das Beschichten die Oberflächenmorphologie der Düsen unverändert bleibt.

#### 2.2.2.2 Kapillaritätstest an beschichteten Düsenkörpern

Tabelle 4 zeigt illustrativ das Ergebnis des Kapillaritätstests an zehn hydrophobisierten, perfluorierten Düsenkörpern, die mit 0,03 mol/l 1H,1H,2H,2H-Tridecafluorooctyltriethoxysilan beschichtet wurden. Das Verfahren folgt der Beschreibung in Kapitel 1.1 und wird nach jeder Beschichtung anhand zufällig ausgewählter Düsenkörper-Proben durchgeführt.

**Tabelle 4: Kapillaritätstest an beschichteten, perfluorierten Düsenkörpern (N = 10)**

| Lösungsmittel / Formel (Eigenschaft) | Spontane Befüllung eines unbeschichteten Düsenkörpers | Spontane Befüllung eines beschichteten (perfluorierten) Düsenkörpers |
|---|---|---|
| Wasser / H20 (hydrophil) | Ja | Nein |
| Perfluorooctane / C8F18 (hydrophob, oleophob) | Nein | Ja |

Der direkte Nachweis der Beschichtung in beschichteten Düsenkörpern ist schwierig, da dies nur über komplizierte, aufwendige Messverfahren wie z.B. TOF-SIMS und ESCA nach einer Spaltung des Sandwich-Systems gelingt. Die Begründung hierfür liegt in den geringen Probenvolumina, die sich durch die dünne Schichtdicke von wenigen Nanometern ergeben.

Am einfachsten ist daher der indirekte Nachweis der Beschichtung, indem man sich des veränderteten Kapillareffektes der Mikrostruktur bedient. Das Verfahren stellt eine Identitätsprüfung für die Anwesenheit der Beschichtung dar. Dieser Test eignet sich auch für die Qualitätsprüfung im großtechnischen Maßstab.

### 2.2.2.3 Einfluss von Rückständen der Funktionalisierungslösung in Düsenkörpern auf die Sprayperformance

In den hier verwendeten Laborsituationen kamen nicht alle Proben sauber aus dem Beschichtungsprozess. Um den Einfluss von Beschichtungsrückständen auf die Spraybildperformance analysieren zu können wurde ein Fehlerbildkatalog mit charakteristischen Fehlerbildern der Düsen aufgesetzt. Die Düsen werden dabei je nach Lokalisation des Rückstandes einer zutreffenden Düsenkategorie zugeordnet.

### 2.2.2.4 Auswirkung der Düsenkörper-Kategorie auf die Sprayperformance

Um den Einfluss der Düsenkörper-Kategorie auf die Sprayperformance der Zerstäuber zu untersuchen, werden Düsen, die für den Langzeit-Provokationsversuch beschichtet wurden (vgl. 0), mikroskopisch analysiert und der entsprechenden Düsenkörper-Kategorie zugeordnet. Tabelle 5 zeigt das Ergebnis der mikroskopischen Analyse dreier Chargen (A bis C), die für die Bemusterung des Langzeit-Provokationsversuches hergestellt wurden. Insgesamt wurden hier 151 Düsenkörper mit 0,03 mol/L 1H,1H,2H,2H-Tridecafluorooctyltriethoxysilan in drei Chargengängen beschichtet und anschließend mikroskopisch analysiert.

**Tabelle 5: Ergebnis der Düsenkörper-Kategorisierung anhand der Bemusterungsproben für den Langzeit-Provokationsversuch aus 0**

| Charge | Kategorie I | Kategorie II | Kategorie III | Kategorie IV | Gesamt |
|---|---|---|---|---|---|
| A | 26 (53 %) | 3 (6 %) | 14 (29 %) | 6 (12 %) | 49 |
| B | 28 (54 %) | 0 | 13 (25 %) | 11 (21%) | 52 |
| C | 21 (42 %) | 1 (2%) | 13 (26 %) | 15 (30 %) | 50 |
| Mittelwert | 49,64 % | 2,71 % | 26,52 % | 21,13 % | 151 |
| Stabw. | 6,62 % | 3,12 % | 1,84 % | 8,88 % | |

Für den Langzeit-Provokationsversuch werden 60 Zerstäuber mit beschichteten Düse bereitgestellt. Aus diesen Geräten wurden jeweils die ursprünglichen Düsen entnommen und beschichtete Düsen eingebaut. Die Verteilung der hier verwendeten beschichteten Düsen bezüglich der jeweiligen Düsenkörper-Kategorie kann Tabelle 6 entnommen werden.

**Tabelle 6: Probenverteilung der Düsenkörper-Kategorisierung für den Langzeit-Provokationsversuch**

| Kategorie I | Kategorie II | Kategorie III | Kategorie IV | Gesamt |
|---|---|---|---|---|
| 30 | 0 | 15 | 15 | 60 |

Alle beschichteten Düsen werden nachvollziehbar in bereit gestellte Zerstäuber eingebaut, so dass stets bekannt ist, in welchem Gerät welche Düsenkategorie eingebaut wurde. Der Einbau erfolgte partikelarm, wie dies unter Laborbedingungen realisierbar ist. Nach dem Provokationsversuch werden die Proben in einer großen Histogrammanalyse hinsichtlich ihres Spraybildverlaufes ausgewertet.

Nach 120 Versuchstagen im 1x2 In-Use Modus, d. h. zwei aufeinanderfolgende Hübe pro Tag, werden alle im Versuch abgegebenen 14400 Sprays der 60 beschichteten Düsen in einer umfassenden Histogramm-Analyse ausgewertet. Es zeigt sich, dass als erstes vereinzelte Gruppe II Spraybilder gebildet werden. Im weiteren Verlauf entstehen deutliche Reihen bzw. Banden, die veranschaulichen, dass verlegte Geräte bei den nächsten Hüben bevorzugt wieder ein Gruppe II Spray aufzeigen. Mit weiterer zunehmender Versuchszeit entwickeln sich hieraus nun Gruppe III Sprays, die im weiteren Verlauf dauerhaft ein derartiges Spraybild manifestieren. Die absolute Anzahl an Gruppe III Sprays war in diesem Versuch verhältnismäßig gering, so dass stattdessen die Gruppe II Sprays für die Histogrammanalyse herangezogen werden. Die Gruppe II Sprays stellen eine Vorstufe der Gruppe III Sprays dar. Es ist daher legitim diese für die weitere Auswertung heranzuziehen.

Es stellt sich heraus, dass Kategorie III- und Kategorie IV- Düsenkörper häufiger ein Gruppe II-Spray verursachen, als dies für die Düsenkategorie I und II der Fall ist. Sind es für die Düsenkörper-Kategorien I und II 15 %, so sind es für die Düsenkörper-Kategorien III und Kategorie IV zwischen 25 bis 30 %.

Es zeigt sich, dass saubere Düsenkörper der Kategorie I und II eine geringere Anzahl an Spraybildanomalien über die gesamte Versuchsdauer haben, als Kat III und IV Düsen. Rückstände im Düsenbereich führen also insgesamt zu mehr Spraybildanomalien. Die Anzahl ist umso größer, wenn beide Düsenkanäle (Kat IV) von Ablagerungen betroffen sind. Der überwiegende Teil der Spraybildanomalien in diesem Versuch tritt zum späteren Versuchszeitpunkt auf. Kat I und II Düsen zeigen auch aufgeschlüsselt nach dem Zeitintervall stets die bessere Sprayperformance. Sowohl die kurzfristige, als auch die langfristige Sprayperformance von sauberen Kategorie I und Kategorie II Düsenkörper ist deutlich besser als die Kategorie III und IV Düsenkörper.

Daraus lässt sich ableiten, dass die Rückstände die Beschichtung bzw. die Ausbildung der Beschichtung auf der Oberfläche beeinträchtigen und es zum Verlust von Sprayperformance kommt.

Eine RAMAN-spektroskopische Analyse der Rückstände zeigt, dass es sich hierbei um polymere Reste der Beschichtungslösung handelt. Vermutlich fließt die Beschichtungslösung in der Trocknungsphase nach der Funktionalisierung an den kleinen Kavitäten der Mikrostruktur zusammen und polymerisiert während des Temperns an diesen Stellen aus.

Die Ergebnisse reflektieren, dass für großtechnische Prozesse die Ausbeute an Kategorie I Düsen möglichst hoch sein sollte. Hierzu eignet sich der Einsatz von Systemen, die überschüssige Beschichtungslösung austreiben, insbesondere solchen Systemen, die auf der Nutzung von Rotationskräften beruhen, wie beispielsweise sogenannte SRD-Systeme ("Spin-Rinse-Dryers").

### 2.2.3. Performance funktioneller Alkylsilanbeschichtungen in In-Use Versuchen (Provokationsversuche)

Im Folgenden soll anhand einer standardisierten Testmethode die Oberflächenfunktionalisierung der Düsenkörper ein Beschichtungsverfahren als eine präventive Maßnahme gegen das Risiko einer Düsenverlegung hinreichend analysiert und bewertet werden.

### 2.2.3.2 Provokationsversuch mit funktionalisierten Düsen: Performance der Oberflächenfunktionalisierung gegenüber Plaque-Ablagerungen

Anhand eines Provokationsversuches (Verwendung der vorab bestimmten Provokationslösung in Zerstäubern) wird untersucht, wie sich unterschiedliche Schichtfunktionalitäten gegenüber dem Aufkommen von provozierten Plaque-Ablagerungen verhalten. Dabei werden Düsenkörper mit unterschiedlichen Beschichtungsreagenzien gemäß dem in Kapitel 1.1 beschriebenen Verfahren beschichtet (mit RCA-Aktivierung).

Das Versuchsdesign des Provokationsversuchen ist dabei wie folgt:

Beschichtungsreagenzien:

- Dynasylan® F8261 (=1H,1H,2H,2H-Tridecafluorooctyltriethoxysilan) (F13C8-OET)
- n-Octyltriethoxysilan (C8-OET)
- 1H,1H,2H,2H-Perfluorooctyldimethylchlorsilan (F13C8-Cl)
- n-Octyldimethylchlorislan (C8-Cl)

Konzentration: jeweils 0,03 mol / Liter
Formulierung: entsprechend 1.3.3
Anzahl Inhalatoren: 50 Stück für jede Beschichtung, 30 Stück für Referenz

In-Use Modus: 1x1 Hub/Tag
Versuchsdauer: 28 Tage
Testparameter: Sprühbild nach Spraybildkatalog

Das zugrundeliegende Versuchsdesign erlaubt den experimentellen Vergleich mehrerer Ansatzpunkte. Zum einen werden hier fluorierte und nichtfluorierte Alkylsilane eingesetzt und zum anderen handelt es sich um Alkyltrialkoxysilane und Alkyldimethylmonochlorsilane. Dies bedeutet, dass hier einerseits die grundsätzliche Beschichtungschemie hinsichtlich der Oberflächenanbindung getestet werden kann als auch andererseits die eigentliche Schichtperformance, die über die Alkylseitenkette bestimmt wird (fluoriert vs. nicht-fluoriert).

Im Folgenden ist der Spraybildverlauf für die Gruppe I Sprays (vgl. Fig. 9) Gruppe II Sprays (vgl. Fig. 10) und Gruppe III Sprays (vgl. Fig. 11) grafisch dargestellt.

2.2.3.1.1 Spraybildverlauf Gruppe I Sprays

Fig. 9 zeigt den Verlauf an Gruppe I Sprays ("Gut-Sprays") für die beschichteten Düsen und die unbeschichtete Referenz. Der Anteil an Gruppe I Sprays nimmt für alle Versuchsarme mehr oder weniger stark über die Zeit ab, jedoch zeigen alle Versuchsarme, in denen beschichtete Düsen verwendet werden, einen im Vergleich mit der unbeschichteten Referenz besseren Spraybildverlauf. Erst gegen Versuchsende weisen die Versuchsarme C8-OET, C8-CL und F13C8-CI eine Annäherung ihres Spraybildverlaufes an die Referenz auf. Der Anteil an Gruppe I Sprays fällt hier für die genannten Versuchsarme bis unter 20 % ab.

Demgegenüber steht die Beschichtungsreagenz F13C8-OET, die eine herausragende Performance im Spraybildverlauf aufzeigt. Bis zum Versuchsende wird ein Anteil von annähernd 80 % bei den Gruppe I Sprays ("Gutsprays") aufrecht erhalten. Im Vergleich mit den anderen Beschichtungsreagenzien ist dies ein Vorteil von über 60 %. Setzt man dies in Bezug zu der Beschichtungsreagenz C8-OET, so wird der Vorteil der perfluorierten Seitenkette von F13C8-OET deutlich.

Die Beschichtungsreagenz C8-OET erzielt ein vergleichbar schlechtes Ergebnis zum Versuchsende wie die beiden Chlorsilane F13C8-Cl und C8-Cl. Es wird jedoch deutlich, dass es in den davorliegenden Versuchstagen einen großen Vorteil gegenüber diesen beiden Verbindungen gibt. Hier nimmt es eindeutig eine Zwischenposition zwischen F13C8-OET und den beiden Chlorsilanen ein.

Insgesamt gesehen überrascht die relativ schlechte Sprayperformance der beiden Chlorsilane. Überraschend ist weiterhin das in Bezug auf F13C8Cl der Nutzen einer perfluorierten Seitenkette gegenüber dem C8-Cl ebenfalls nicht detektierbar ist. Die Tatsache, dass C8-Cl ähnliche Ergebnisse liefert, lässt vermuten, dass es bei den Chlorsilanen zu einer weniger stabilen Oberflächenanbindung kommt. Zwar ist ein Vorteil gegenüber der unbeschichteten Referenz in den ersten Versuchstagen feststellbar, aber dieser Vorteil ist nur bis Tag 17 zu registrieren.

**2.2.3.1.2 Spraybildverlauf Gruppe II Sprays**

Fig. 10 zeigt den Verlauf an Gruppe II Sprays für die beschichteten Düsen und die unbeschichtete Referenz. Der Anteil an Gruppe II Sprays ("Spraybildanomalien") nimmt für alle Versuchsarme mehr oder weniger stark über die Zeit zu. Die Referenz erreicht ihr Maximum bereits mit Versuchstag 4 mit über 60 %, während für die anderen Versuchsarme kein wirkliches Maximum identifiziert werden kann. Hier handelt es sich um eine kontinuierliche Zunahme an Gruppe II Sprays über die Versuchszeit. Auffallend ist auch hier der große Vorteil der Beschichtungsreagenz F13C8-OEt. Der Anteil an Spraybildanomalien liegt zum Versuchsende nur bei knapp 15 % und ist auch über den gesamten Versuchszeitraum ab Tag 5 stets unterhalb der anderen Versuchsarme zu aufzufinden.

**2.2.3.1.3 Spraybildverlauf Gruppe III Sprays**

Fig. 11 zeigt den Verlauf an Gruppe III Sprays ("Jet-Divergency") für die beschichteten Düsen sowie die unbeschichtete Referenz. Die Abbildung offenbart, dass auch der Anteil an Gruppe III Sprays für alle Versuchsarme mehr oder weniger stark über die Zeit zunimmt. Die beiden Chlorsilane erreichen gegen Versuchsende eine ähnliche Rate an Gruppe III Sprays (annähernd 60 %) wie die unbeschichtete Referenz. Gegenüber der Referenz zeigt sich für die Chlorsilane daher nur ein temporärer Nutzen von einigen wenigen Versuchstagen, da die Chlorsilane und die Referenz bereits ab Tag 16 eine vergleichbare Anzahl an Gruppe III Sprays zeigen (ca. 40%). Eine Zwischenposition nimmt auch in dieser Ansicht wieder das Beschichtungsreagenz C8-OET ein. Es liegt zum Versuchsende bei einer -Gruppe-III-Spray-Rate von annähernd 30 %. Die Beschichtungsreagenz F13C8-OET zeigt mit Abstand die beste Sprayperformance: Weniger als 10 % der Zerstäuber zeigen über alle Versuchstage ein Gruppe III Spray.

#### 2.2.3.1.4 Gruppe III Sprays: 10-Tage-Durchschnittsrate gegen Versuchsende

Der Vorteil der Beschichtungsreagenz F13C8-OET wird besonders deutlich bei Betrachtung der 10-Tage-Durchschnittsrate, wie sich aus Fig. 15 ergibt. Im 10-Tage Durchschnitt erreicht die Referenz eine Rate von annähernd 50 % Gruppe III-Sprays. Demgegenüber stehen die beiden Versuchsarme der Chlorsilane, die eine vergleichbar hohe 10 Tage-Durchschnittsrate von ca. 50 % aufweisen. Die niedrigste Rate an Gruppe III Sprays erzielt der Versuchsarm mit der Beschichtungsreagenz F13C8-OET. Hier wird gegen Versuchsende eine 10-Tage-Durchschnittsrate von unter 5 % erzielt, was gegenüber allen anderen Versuchsarmen exorbitant besser ist. Auch die Beschichtungsreagenz C8-OET weist eine deutlich bessere 10-Tage-Durchschnittsrate von nur ca. 25 % auf. Auch diese Beschichtungsreagenz schützt damit erheblich besser vor Plaque-Ablagerungen als die Alkylchlorsilane.

Insgesamt zeigt sich in diesem Versuch sehr deutlich der Vorteil der Alkyltrialkoxysilane gegenüber den Alkylchlorsilanen.

Das Ergebnis des Provokationsversuches mit funktionalisierten Düsen zeigt, dass die Wahrscheinlichkeit einer Düsenverstopfung mit dem richtigen Beschichtungsreagenz drastisch reduziert werden kann.

Unverständlicherweise schneiden die Alkyldimethylmonochlorsilane relativ schlecht ab und ergeben gegenüber der Referenz kaum einen messbaren Vorteil in der Sprayperformance. Dies war so nicht zu erwarten, da in der Literatur auch die Alkyldimethylmonochlorsilane gute Bindungseigenschaften aufzeigen (vgl. Fadeev, A.Y. and T.J. McCarthy, Trialkylsilane monolayers covalently attached to silicon surfaces: wettability studies indicating that molecular topography contributes to contact angle hysteresis. Langmuir, 1999. 15(11): p. 3759-3766). Der Spraybildverlauf ist über den Versuchszeitraum nicht besser als die unbeschichtete Referenz.

Gründe für die schlechte Anbindung der Alkyldimethylmonochlorsilane an die Oberfläche lassen sich nur vermuten. Ein möglicher entscheidender Nachteil der Alkyldimethylmonochlorsilane ist, dass diese Substanzklasse nur eine Bindungsstelle mit der Oberfläche besitzt. Vergleicht man dies mit den Alkyltrialkoxysilanen, so besitzen diese zwei Koordinationsstellen mehr, was offensichtlich zu mehr Bindungsstabilität führt. Berücksichtigt man zusätzlich die Kräfteverhältnisse im verwendeten Zerstäuber, die einen Druck von über 200 bar und eine Strömunggeschwindigkeit von über 130 m/s im Düsenkanal erreichen können, so könnte es möglich sein, dass diese Kräfte schlichtweg zu groß für nur eine Koordinationsstelle sind und Schichterosion bzw. Schichtablösung die Folge sind.

#### 2.2.4 Langzeit-Performance von 1H,1H,2H,2H-Tridecafluorooctyltriethoxysilan in In-Use Versuchen

In einem Provokationsversuch wird untersucht, wie lange sich mit beschichteten Düsen eine Düsenverlegung vermeiden lässt. Hierzu werden Düsen mit 1H,1H,2H,2H-Tridecafluorooctyltriethoxysilan beschichtet und in einem Provokationsversuch im In-Use Modus 1x2 über eine Zeitspanne von 120 Tagen getestet. Es handelt sich hierbei um insgesamt 60 beschichtete Düsen der Düsenkörper-Kategorien I+II, III und IV.
Das Versuchsdesign des Provokationsversuchen ist dabei wie folgt:

Beschichtungsreagenz:

- Dynasylan® F8261 (=Tridecafluorooctyltriethoxysilan) (F13C8-OET)
- Referenz (unbeschichtet)

Konzentration: 0,03 mol / Liter
Formulierung: entsprechend 1.3.3
Versuchsdauer: Ref = 45 Tage, F13C8 = 120 Tage
Anzahl Inhalatoren: 60 Stück beschichtet mit F13C8-OET,
30 Stück Referenz (unbeschichtet)
In-Use Modus: 1x2 Hub / Tag
Testparameter: Sprühbild nach Spraybildkatalog

#### 2.2.4.1 Spraybildverlauf Gruppe I-Sprays

Fig. 13 gibt den Verlauf an Gruppe I-Sprays ("Gutsprays") für den Langzeit-Provokationsversuch an. Figur 13 verdeutlicht den großen Vorteil der Beschichtung gegenüber der unbeschichteten Referenz. Während bei Versuchstag 45 die Referenz nur ca. 20 % Gruppe I-Sprays ("Gutspray") aufzeigt, sind dies bei den beschichteten Düsen mehr als 80 %. Sogar zum Versuchsende bei Tag 120 sind es für das F13C8-OET noch mehr als 60 % der Zerstäuber die ein Gruppe I-Spray ("Gutspray") besitzen. Wie der Grafik entnommen werden kann, wurde die Referenz an Tag 45 auf Stopp gesetzt, da die Gutspray-Rate ein Gleichgewicht bei ca. 20 % erreicht hat.

Bereits innerhalb der ersten Versuchstage entwickeln einige Zerstäuber trotz Beschichtung eine Abweichung

von einem Gruppe I-Spray. Dies ist auf das Vorhandensein von Beschichtungsrückständen zurückzuführen. In den ersten Tagen ist daher die Referenz tendenziell etwas besser als die Geräte mit beschichteten Düsen. Dieser Effekt reicht in etwa bis Tag 10, da ab hier ein deutlicher Abfall der Gutsprays für die Referenz einsetzt. Die Ursache für dieses anfängliche Phänomen liegt hier in der Verwendung von "guten" und "schlechten" Düsenkategorien. In diesem Versuch werden bezüglich der Beschichtungsrückstände sowohl Kategorie I und Kategorie II Düsen, als auch Kategorie III und Kategorie-IV Düsen verwendet.

Es zeigt sich in diesem Versuch sehr deutlich, dass Rückstände der Beschichtungslösung ebenfalls Gruppe II- und Gruppe III Sprays verursachen können.

### 2.2.4.2 Spraybildverlauf Gruppe II-Sprays

Fig. 14 gibt den Verlauf an Gruppe II-Sprays ("Sprayanomalien") für den Langzeit-Provokationsversuch an. Die Anzahl an Gruppe II-Sprays steigt vor allem für die Referenz sehr stark über die Zeit an. Hier werden bereits bei Tag 20 mehr als 70 % Gruppe II-Sprays beobachtet. Ein Blick auf den Spraybildverlauf der beschichteten Düsen (F13C8-OET) offenbart, dass auch hier eine moderate Steigung an Gruppe II-Sprays zu beobachten ist. Jedoch zeigen die beschichteten Düsen sogar zum Versuchende bei Tag 120 nur ca. 30 - 40 % Gruppe II Sprays, was im Vergleich mit der Referenz erheblich weniger ist.

Es ist auch hier deutlich zu erkennen, dass bereits ab dem ersten Versuchstag Spraybildanomalien (Gruppe II-Sprays) bei den Geräten mit beschichteten Düsen auftreten. Die Ursache sind hier die bereits vorab erwähnten Beschichtungsrückstände. Trotz der Beschichtung zeigen auch die F13C8-Geräte ab einem gewissen Zeitpunkt eine Zunahme an Gruppe II-Sprays, was auch hier den Prozess der Bildung von Plaque-Ablagerungen anzeigt. Dies ist im Spraybildverlauf ab ca. Tag 40 an einer stärkeren Steigung erkennbar.

Bzgl. der Beschichtungsrückstände kann vor Einbau der beschichteten Düsenkörpern im Rahmen einer optischen Kontrolle der beschichteten Düsenkörper eine Aussortierung der Düsenkörper mit Beschichtungsrückständen erfolgen. Durch so eine Aussortierung ist eine maßgebliche Reduzierung der Spraybildanomalien für Geräte mit beschichteten Düsen zu erwarten.

### 2.2.4.3 Spraybildverlauf Gruppe III Sprays

Fig. 15 stellt den Verlauf an Gruppe III Sprays ("Jet-Divergency") für den Langzeit-Provokationsversuch dar. Wie der Abbildung entnommen werden kann, treten insgesamt sehr wenig Gruppe III-Sprays auf (Chargen-Effekte spielen hierbei auch eine Rolle). Beim Vergleich der beschichteten Düsen mit der Referenz wird offensichtlich, dass die Referenz schon ab Versuchstag 10 vereinzelt Gruppe III-Sprays aufzeigt, während dies für die Beschichtung erst ab Tag 70 der Fall ist. Dies bedeutet, dass durch die Beschichtung das Auftreten des ersten Gruppe III-Sprays zeitlich um etwa 60 Tage verschoben werde konnte.

Die hier getestete Zerstäuber-Charge besitzt unter Stressbedingungen schon von vornherein eine sehr geringe Anzahl an Gruppe III-Sprays, obwohl sehr schnell bei der Referenz eine große Anzahl an Gruppe II-Sprays gebildet wird. In der Regel reagieren diese Gruppe II Sprays rasch weiter zu Gruppe III-Sprays, können also auch als Indikator für ein Risiko zur Düsenverlegung herangezogen werden

### 2.2.4.4 Gruppe III-Sprays: 10-Tage-Durchschnittsrate gegen Versuchsende

Fig. 16 stellt die 10-Tage-Durschnittsrate zum Versuchsende für den Langzeit-Provokationsversuch dar. Fig. 16 illustriert noch einmal zusammenfassend das Ergebnis des Spraybildverlaufes der Gruppe III-Sprays. Die Referenz weist an Tag 45 eine Rate von ca. 5 % Gruppe III-Sprays auf. Im Vergleich hierzu zeigen die Zerstäuber mit beschichteten Düsen noch kein Aufkommen von Gruppe III Sprays. Diese traten sporadisch in etwa ab Tag 70 auf. Erst zum Versuchsende bei Tag 120 haben die Zerstäuber-Geräte mit beschichteten Düsen eine vergleichbare 10 - Tage-Durchschnittsrate zur Referenz von in etwa 5 %.

### 2.2.4.5 Ergebnis der Performance von Düsenbeschichtungen 1H,1H,2H,2H-Tridecafluorooctyltriethoxysilan

Das Ergebnis des Langzeit-Provokationsversuches mit funktionalisierten Düsen zeigt, dass die Wahrscheinlichkeit einer Düsenverstopfung auch über eine sehr lange Versuchszeit drastisch reduziert werden kann. Die Versuchsdauer dieses Versuches betrug insgesamt 120 Tage. Die 120 Tage leiten sich aus einer möglichen viermonatigen Anwendung des Inhalators ab. Für die hier mit 1H,1H,2H,2H-Tridecafluorooctyltriethoxysilan beschichteten Düsen ist erst mit Versuchstag 70 das Aufkommen eines ersten Gruppe III Spray zu verzeichnen. Die Referenz hingegen zeigt bereits ab Tag 10 die ersten Gruppe III-Sprays. Dies ist für die Beschichtung ein Vorteil von 60 Tagen. Die Beschichtung hat das Aufkommen von Gruppe III-Sprays über diesen langen Zeitraum hinausgezögert, was ein hervorragendes

Ergebnis darstellt.

Auffallend in diesem Versuch ist auch, dass es generell über die gesamte Versuchszeit zu sehr wenig Gruppe III-Sprays kommt. Dies ist überraschend auch für die unbeschichtete Referenz zu registrieren. Die Ursache hierfür ist die Zerstäuber-Charge selbst, die auch in anderen Versuchen besonders wenig Gruppe III-Sprays gezeigt hat. Die Inzidenz an Gruppe II- und Gruppe III-Sprays ist abhängig von der verwendeten Geräte-Charge. Es fällt jedoch bei diesem Versuch auf, dass der Anteil an Gruppe II-Sprays sehr lange sehr hoch bleibt. In der Regel nimmt die Anzahl an Gruppe III-Sprays mit einem schnellen Erreichen eines Maximums bei den Gruppe II-Sprays gravierend zu (d.h. Geräte mit Gruppe II Sparys werden zu Geräten mit Gruppe III Sprays).

Die Referenz wurde ab Tag 45 auf Stopp gesetzt, da hier der Vorteil der Beschichtung bereits eindeutig war und die Referenz ein Gleichgewicht erreicht hat. Mit Tag 70 zeigen auch die Inhalatoren mit beschichteten Düsen erste Gruppe III Sprays. Die Kinetik der Zunahme entspricht jedoch nicht der unbeschichteten Referenz, sondern verläuft deutlich langsamer.

### 2.3. Langzeitstabilität der Beschichtung: Stabilitätsstudie an beschichteten Si/Glas-Flachsubstraten

In einer Stabilitätsstudie soll der Einfluss unterschiedlicher Stressgrößen (pH-Wert, Temperatur, Einlagerungszeit) auf die Schicht-Performance von 1H,1H,2H,2H-Tridecafluorooctyltriethoxysilan untersucht werden. Hierfür werden in mehreren Beschichtungsansätzen Silizium- und Glassubstrate bei drei Konzentrationen beschichtet und über die Zeit in ethanolischer Placebolösung eingelagert. Die Proben werden jeweils bei gleichen Beschichtungsparametern beschichtet und unterscheiden sich nur in der Konzentration der Beschichtungslösung. Die Stabilität der Proben wird dabei anhand von Schichtdickenmessungen und Kontaktwinkelmessungen beurteilt.

### 2.3.1 Studienlayout der Stabilitätsstudie

Beschichtet werden die Si/Glas-Flachsubstrate bei den drei Beschichtungskonzentrationen von 0,003 mol/l, 0,015 mol/l und 0,03 mol/l. Eingelagert werden die Proben verschlossen und lichtgeschützt in Polyethylenflaschen in einer ethanolischer Placebolösung bei einem pH-Wert von 2,0 und 4,5 jeweils bei Raumtemperatur im Labor (20°C±3 °C) und 40 °C im Klimaschrank. Die Einlagerungszeit beträgt für die hohe Beschichtungskonzentration 14, 30, 90 und 180 Tage. Die beiden niedrigeren Beschichtungskonzentrationen werden nur für 30 und 180 Tage eingelagert. Die Studienparameter sind in Tabelle 7 zusammengefasst.

**Tabelle 7: Studienlayout der Stabilitätsstudie für Si/Glas-Flachsubstrate**

| **Konzentration Probe [mol/l]** | **Substrat** | **Zeit [Tage]** | | | | **Temperatur** | | **pH-Wert** | |
|---|---|---|---|---|---|---|---|---|---|
| | | **14** | **30** | **90** | **180** | **RT** | **40°C** | **2,0** | **4,5** |
| 0.03 | Si+Glas | x | x | x | x | x | x | x | x |
| 0,015 | Si+Glas | | x | x | x | x | x | x | x |
| 0,003 | Si+Glas | | x | | x | x | x | x | x |
| | Referenz | x | x | x | x | x | x | x | x |
| **Referenz: x = durchgeführt ohne Silizium/Glas-Substrate** | | | | | | | | | |

### 2.3.2 Beschichtung der Si/Glas-Flachsubstrate

In mehreren Chargenansätzen werden die Si/Glas-Flachsubstrate für die Stabilitätsstudie gemäß Kapitel beschichtet.

### 2.3.3. Einlagerung der Substrate

Für die Stabilitätsstudie werden zwei ethanolische Placebolösungen mit dem pH Wert 2,0 und 4,5 angesetzt. Am Tag der Einlagerung werden in jeder Polyethylenflasche 170 Gramm ethanolische Placebolösung vorgelegt. Anschließend werden jeweils zwei Silizium- bzw. Glassubstrate mittels Wafer-Pinzette hinzugefügt. Dabei ist darauf zu achten, dass die Substrate so in der Flasche positioniert werden, dass die Substrate nicht aneinander haften. Nach dem Verschließen wird die Masse der gesamten PE-Flasche ermittelt und dokumentiert um am jeweiligen Auslagerungstag den Massenverlust durch die Einlagerung bestimmen zu können. Zusätzlich werden pro Einlagerungszeit, pH-Wert und

Temperatur jeweils Referenzmuster ohne Substrate eingelagert.

### 2.3.4. Kontrolle des pH Wertes und der Masse am Auslagerungstag

Am jeweiligen Auslagerungstag wird die Masse der PE-Flasche erneut bestimmt und somit der Massenverlust über die Einlagerungszeit ermittelt. Zusätzlich zum Massenverlust wird ebenfalls der pH-Wert der Lösung überprüft. Auf diese Weise lässt sich kontrollieren, ob die PE-Flasche ausreichend dicht über die Versuchszeit war und ob während der Einlagerung ein pH-Shift stattgefunden hat.

### 2.3.5. Auswertung der Stabilitätsstudie

Die Auswertung der Stabilitätsanalyse erfolgt anhand statischer Kontaktwinkelmessung sowie über Schichtdickenmessungen mittels spektroskopischer Ellipsometrie. Die Werte einer Probe am Auslagerungstag werden mit den zuvor ermittelten Startwerten für den Kontaktwinkel und die Schichtdicke in Bezug gesetzt. Die Startwerte werden anhand eines repräsentativen Musters erhoben, das nicht eingelagert wurde und Proben aus jedem zuvor durchgeführten Beschichtungslauf enthielt.

#### 2.3.5.1 Änderung des Kontaktwinkels über die Einlagerungszeit

Im Folgenden werden die Ergebnisse bezüglich der Kontaktwinkelmessungen auf Silizium und Glas vorgestellt.

In den Untersuchungen zeigt sich ein deutlicher Konzentrationseffekt für die Startwerte auf Silizium. Mit zunehmender Beschichtungskonzentration erhält man einen höheren Kontaktwinkel für Wasser und n-Dodecan und es resultiert eine kleinere Standardabweichung. Gemessen wurden jeweils fünf Tropfen Wasser und fünf Tropfen n-Dodecan pro Substrat. Dies resultiert bei zehn Messungen pro Tropfen in 50 Winkeln.

#### 2.3.5.1.1 Veränderungen des Kontaktwinkels auf Silizium bei der Einlagerung unter Raumtemperatur (25°C)

Es wird die Änderung des Wasser-Kontaktwinkels bzw. n-Dodecan-Kontaktwinkels auf Silizium bei der Einlagerung bei Raumtemperatur untersucht. Die Ergebnisse der Untersuchungen sind in Tabellen 8 und 9 zusammengefasst.

**Tabelle 8: Veränderungen des Wasser-Kontaktwinkels auf Silizium bei der Einlagerung unter Raumtemperatur (25°C)**

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 2,0 | 112,72 | 1,39 | 2,07 | 3,44 | 7,34 |
| 0,03 | 4,5 | 112,72 | 3,05 | 4,18 | 5,66 | 6,97 |
| 0,015 | 2,0 | 108,78 | n.b.[1] | 4,73 | 3,25 | 3,96 |
| 0,015 | 4,5 | 108,78 | n.b.[1] | 6,55 | 3,27 | 3,19 |
| 0,003 | 2,0 | 101,61 | n.b.[1] | 1,76 | n.b.[1] | 7,52 |
| 0,003 | 4,5 | 101,61 | n.b.[1] | 0,01 | n.b.[1] | -1,41 |
| [1]n.b. : nicht bestimmt | | | | | | |

**Tabelle 9: Veränderungen des Dodecan-Kontaktwinkels auf Silizium bei der Einlagerung unter Raumtemperatur (25°C)**

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 2,0 | 68,82 | 0,51 | 3,11 | 4,40 | 4,82 |

(fortgesetzt)

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 4,5 | 68,82 | 0,33 | 3,20 | 3,51 | 5,42 |
| 0,015 | 2,0 | 66,40 | n.b.[1] | -1,71 | 0,82 | 1,55 |
| 0,015 | 4,5 | 66,40 | n.b.[1] | 0,26 | 1,59 | 1,48 |
| 0,003 | 2,0 | 61,42 | n.b.[1] | 6,68 | n.b.[1] | 5,03 |
| 0,003 | 4,5 | 61,42 | n.b.[1] | 2,20 | n.b.[1] | -1,72 |
| [1]n.b. : nicht bestimmt | | | | | | |

In den Untersuchungen zeigt sich, dass der Wasser- als auch der Dodecan-Kontaktwinkel sich nur geringfügig ($x \leq 10°$) über die betrachtete Einlagerungszeit ändert. Eine stufenweise Abnahme des Kontaktwinkels über die Einlagerungszeit kann allerdings für die Beschichtungskonzentration 0,003 mol/l festgestellt werden. Dieser Effekt ist sowohl für Wasser als auch Dodecan detektierbar.

Es kann festgestellt werden, dass die Streuung im Kontaktwinkel bei einer Beschichtungskonzentration von 0,003 mol/l insgesamt sehr hoch ist. Sie liegt teilweise bei über 20° und überlagert damit mögliche Effekte. Im Vergleich hierzu werden bei den Beschichtungskonzentrationen 0,015 mol/l und 0,03 mol/l sehr präzise Werte mit wesentlich geringerer Streuung erzielt. Dies deutet an, dass die mit einer Konzentration von 0,003 mol/l nicht immer eine homogene Beschichtung erzielt wird.

Aus der Tatsache, dass alle mittleren Änderungen das Wasser- als auch das Dodecan-Kontaktwinkels unterhalb 10° bleiben, kann darauf geschlossen werden, dass in diesem Falle allenfalls lediglich zu einer Schichtveränderung, nicht jedoch zu einer Schichtablösung gekommen ist. Der Wasser-Kontaktwinkel für ein unbeschichtetes Silizium oder Glassubstrat würde bei ca. 30° - 40° liegen. Ein aktiviertes Substrat würde sogar noch hydrophiler sein. Die hier betrachteten Wasser-Kontaktwinkel liegen auch nach Einlagerung oberhalb von 100°. Eine Schichtablösung würde zu Wasser-Kontaktwinkelveränderungen von weit über 50° führen. Dies ist hier eindeutig nicht zu erkennen.

Ein genereller Unterschied durch eine Einlagerung bei pH 2,0 und pH 4,5 konnte ebenfalls nicht festgestellt werden (d.h. die Beschichtung ist im getesteten pH-Bereich säureresistent).

### 2.3.5.1.2 Veränderungen des Kontaktwinkels auf Silizium bei der Einlagerung unter 40°C

Es wird die Änderung des Wasser-Kontaktwinkels bzw. n-Dodecan-Kontaktwinkels auf Silizium bei der Einlagerung im Klimaschrank bei 40 °C untersucht. Die Ergebnisse der Untersuchungen sind in Tabellen 10 und 11 zusammengefasst.

**Tabelle 10: Veränderungen des Wasser-Kontaktwinkels auf Silizium bei der Einlagerung unter 40 °C**

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 2,0 | 112,72 | 0,01 | 5,20 | 4,17 | 8,13 |
| 0,03 | 4,5 | 112,72 | 3,49 | 3,41 | 6,74 | 9,08 |
| 0,015 | 2,0 | 108,78 | n.b.[1] | -6,50 | 0,13 | 4,39 |
| 0,015 | 4,5 | 108,78 | n.b.[1] | -3,53 | 3,42 | 6,34 |
| 0,003 | 2,0 | 101,61 | n.b.[1] | 0,90 | n.b.[1] | 16,73 |
| 0,003 | 4,5 | 101,61 | n.b.[1] | 3,83 | n.b.[1] | 6,08 |
| [1]n.b. : nicht bestimmt | | | | | | |

**Tabelle 11: Veränderungen des Dodecan-Kontaktwinkels auf Silizium bei der Einlagerung 40 °C**

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 2,0 | 68,82 | 0,88 | 2,17 | 1,73 | 7,19 |
| 0,03 | 4,5 | 68,82 | 2,29 | 2,42 | 5,36 | 5,93 |
| 0,015 | 2,0 | 66,40 | n.b.[1] | -1,71 | -0,19 | 1,19 |
| 0,015 | 4,5 | 66,40 | n.b.[1] | 0,26 | 2,11 | 3,21 |
| 0,003 | 2,0 | 61,42 | n.b.[1] | -4,68 | n.b.[1] | 11,67 |
| 0,003 | 4,5 | 61,42 | n.b.[1] | 1,67 | n.b.[1] | 2,21 |
| [1]n.b. : nicht bestimmt | | | | | | |

In den Untersuchungen zeigt sich, dass sich der Kontaktwinkel nur geringfügig hinsichtlich der abgebildeten Stressgrößen, wie gemäß Tabelle 7 in Kapitel 2.3.1 aufgeführt, ändert. Die mittlere Änderung des Wasser-Kontaktwinkel bleibt auch bei der Erhöhung der Temperatur unterhalb von 10°. Eine stufenweise Abnahme des Kontaktwinkels über die Zeit kann auch hier bei den Konzentrationen 0,015 mol/l und 0,03 mol/l analog zu den bei Raumtemperatur ermittelten Werten registriert werden.

Insgesamt kann festgestellt werden, dass auch durch eine Temperaturerhöhung keine Abnahme bzw. Veränderung der Hydrophobizität der Substrate festgestellt wird. Die Daten sind mit den Werten bei Raumtemperatur absolut vergleichbar. Es ist somit kein Temperatureffekt auf den Silizium-Substraten registrierbar.

**2.3.5.1.3 Veränderungen des Kontaktwinkels auf Glas bei der Einlagerung unter Raumtemperatur (25 °C)**

Es wird die Änderung des Wasser-Kontaktwinkels bzw. n-Dodecan-Kontaktwinkels auf Glas bei der Einlagerung bei Raumtemperatur untersucht. Die Ergebnisse der Untersuchungen sind in Tabellen 12 und 13 zusammengefasst.

**Tabelle 12: Veränderungen des Wasser-Kontaktwinkels auf Glas bei der Einlagerung unter Raumtemperatur (25°C)**

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 2,0 | 112,06 | 2,13 | -2,26 | -1,00 | 3,92 |
| 0,03 | 4,5 | 112,06 | -4,31 | 0,75 | -6,21 | 0,61 |
| 0,015 | 2,0 | 87,41 | n.b.[1] | -9,10 | -14,92 | -13,81 |
| 0,015 | 4,5 | 87,41 | n.b.[1] | -12,65 | -12,05 | -19,27 |
| 0,003 | 2,0 | 91,40 | n.b.[1] | -5,48 | n.b.[1] | 22,08 |
| 0,003 | 4,5 | 91,40 | n.b.[1] | -3,65 | n.b.[1] | 2,03 |
| [1]n.b. : nicht bestimmt | | | | | | |

**Tabelle 13: Veränderungen des Dodecan-Kontaktwinkels auf Glas bei der Einlagerung unter Raumtemperatur (25°C)**

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 2,0 | 68,91 | 3,74 | 1,27 | 2,61 | 2,79 |
| 0,03 | 4,5 | 68,91 | 2,64 | 5,84 | -2,66 | 1,59 |
| 0,015 | 2,0 | 47,52 | n.b.[1] | -11,50 | -12,20 | -2,78 |
| 0,015 | 4,5 | 47,52 | n.b.[1] | -14,49 | -9,92 | -15,51 |
| 0,003 | 2,0 | 60,57 | n.b.[1] | 16,11 | n.b.[1] | 2,85 |
| 0,003 | 4,5 | 60,57 | n.b.[1] | 9,65 | n.b.[1] | 6,89 |
| [1]n.b. : nicht bestimmt | | | | | | |

Es zeigt sich, dass es auch auf Glas nicht zu einer Schichtablösung kommt. Die mittlere Änderung des Kontaktwinkels bewegt sich bei den Substraten mit einer Beschichtungskonzentration von 0,03 mol/l ebenfalls bei unter 10°, wie es bereits für die Siliziumsubstrate festgestellt werden konnte. Betrachtet man die Daten für die Beschichtungskonzentration 0,003 mol/l und 0,015 mol/l so lässt sich hier vor allem für die Beschichtungskonzentration 0,003 mol/l eine ebenfalls sehr starke Streuung in den Werten feststellen. Diese ist mit der Streuung auf Silizium bei der gleichen Beschichtungskonzentration vergleichbar und überlagert auch hier etwaige mögliche Effekte.

Eine Auffälligkeit weisen die Ergebnisse für die Beschichtungskonzentration von 0,015 mol/l auf. Hier war der Kontaktwinkel für Wasser und n-Dodecan größer als der ermittelte Startwert. Gemessen wurde dieser Trend hier für alle Proben, ist aber unter Berücksichtigung der Standardabweichung nicht signifikant.

Es kann insgesamt festgehalten werden, dass auch für die Glassubstrate kein Einlagerungseffekt registriert werden kann. Dies umfasst auch die beiden getesteten pH-Werte.

### 2.3.5.1.4 Veränderungen des Kontaktwinkels auf Glas bei der Einlagerung unter 40 °C

Es wird die Änderung des Wasser-Kontaktwinkels bzw. n-Dodecan Kontaktwinkels auf Glas bei der Einlagerung bei 40 ° untersucht. Die Ergebnisse der Untersuchungen sind in Tabellen 14 und 15 zusammengefasst.

**Tabelle 14: Veränderungen des Wasser-Kontaktwinkels auf Glas bei der Einlagerung unter 40 °C**

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 2,0 | 112,06 | 0,79 | -2,19 | 17,55 | -1,56 |
| 0,03 | 4,5 | 112,06 | -1,89 | 2,74 | -1,67 | -1,08 |
| 0,015 | 2,0 | 87,41 | n.b.[1] | -17,06 | -13,58 | -14,47 |
| 0,015 | 4,5 | 87,41 | n.b.[1] | -17,54 | -18,90 | -23,69 |
| 0,003 | 2,0 | 91,40 | n.b.[1] | -16,71 | n.b.[1] | 14,21 |
| 0,003 | 4,5 | 91,40 | n.b.[1] | -13,74 | n.b.[1] | -16,11 |
| [1]n.b. : nicht bestimmt | | | | | | |

**Tabelle 15: Veränderungen des Dodecan-Kontaktwinkels auf Glas bei der Einlagerung unter 40 °C**

| Konzentration MTMS [mmol/l] | pH | Kontaktwinkel [°] | | | | |
|---|---|---|---|---|---|---|
| | | Ausgangswert | Änderung | | | |
| | | | 14 Tage | 30 Tage | 90 Tage | 180 Tage |
| 0,03 | 2,0 | 68,91 | 2,03 | -0,67 | 13,38 | -0,03 |
| 0,03 | 4,5 | 68,91 | 7,04 | 2,51 | 3,96 | -1,25 |
| 0,015 | 2,0 | 47,52 | n.b.[1] | -17,96 | -13,12 | -13,24 |
| 0,015 | 4,5 | 47,52 | n.b.[1] | -17,34 | -19,10 | -21,52 |
| 0,003 | 2,0 | 60,57 | n.b.[1] | 12,30 | n.b.[1] | 0,80 |
| 0,003 | 4,5 | 60,57 | n.b.[1] | 4,83 | n.b.[1] | -3,87 |
| [1]n.b. : nicht bestimmt | | | | | | |

Die ermittelten Werte für 40 °C sind grundsätzlich mit denen bei Raumtemperatur vergleichbar.

Insgesamt kann auch hier festgestellt werden, dass sich kein explizierter Einlagerungseffekt für Glas bei einer Temperatur von 40 °C feststellen lässt. Dies umfasst auch hier die beiden getesteten pH-Werte 2,0 und 4,5.

### 2.3.5.2 Effekt der Einlagerung auf die Schichtdicke

Im Folgenden werden die Ergebnisse bezüglich der mittels Ellipsometrie durchgeführten Schichtdickenmessungen auf Silizium und Glas vorgestellt.

Jedes Substrat wurde dreimal bei den Winkeln 65°, 70° und 75° mittels spektroskopischer Ellipsometrie vermessen.

Die Proben mit der Beschichtungskonzentration von 0,003 mol/l sind auch bei den ellipsometrischen Messungen auffällig hinsichtlich ihrer Standardabweichung. Desweiteren lässt sich feststellen, dass auf den Silizium-Substraten mit zunehmender Konzentrationserhöhung die Schichtdicke nicht messbar zugenommen hat. Dieser Effekt lässt sich allerdings auf den Glas-Substraten tendenziell feststellen.

Das angenommene Modell für die Auswertung entspricht den Ausführungen aus Abschnitt 1.4.4. Der Brechungsindex, der für die Schichtdickenmessung des SAM-Layers notwendig ist, konnte der Literatur entnommen werden und liegt bei 1,256 (vgl. Jung, J.-I., J.Y. Bae, and B.-S. Bae, Characterization and mesostructure control of mesoporous fluorinated organosilicate films. Journal of Materials Chemistry, 2004. 14(13): p. 1988-1994).

#### 2.3.5.2.1 Veränderungen der Schichtdicke auf Silizium bei der Einlagerung bei Raumtemperatur und bei 40°C

Es wird die Änderung der Schichtdicke auf Silizium bei der Einlagerung bei Raumtemperatur (25 °C) und 40 °C untersucht. Die Ergebnisse der Untersuchungen sind in Tabellen 16 und 17 zusammengefasst.

**Tabelle 16: Veränderungen der Schichtdicke der MTMS-Beschichtung auf Silizium bei der Einlagerung unter Raumtemperatur (25°C)**

| Konzentration MTMS [mmol/l] | pH | Schichdicke [nm] | | | |
|---|---|---|---|---|---|
| | | Ausgangswert | Änderung | | |
| | | | 30 Tage | 90 Tage | 180 Tage |
| 0,03 | 2,0 | 0,93 | -0,10 | -0,02 | -0,06 |
| 0,03 | 4,5 | 0,93 | -0,10 | -0,14 | -0,11 |
| 0,015 | 2,0 | 1,06 | -0,05 | 0,11 | 0,16 |
| 0,015 | 4,5 | 1,06 | -0,02 | 0,03 | -0,01 |
| 0,003 | 2,0 | 1,05 | 0,23 | n.b.[1] | 0,17 |

(fortgesetzt)

| Konzentration MTMS [mmol/l] | pH | Schichdicke [nm] | | | |
|---|---|---|---|---|---|
| | | Ausgangswert | Änderung | | |
| | | | 30 Tage | 90 Tage | 180 Tage |
| 0,003 | 4,5 | 1,05 | 0,15 | n.b.[1] | -0,02 |
| [1]n.b. : nicht bestimmt | | | | | |

**Tabelle 17: Veränderungen der Schichtdicke der MTMS-Beschichtung auf Silizium bei der Einlagerung unter 40 °C**

| Konzentration MTMS [mmol/l] | pH | Schichdicke [nm] | | | |
|---|---|---|---|---|---|
| | | Ausgangswert | Änderung | | |
| | | | 30 Tage | 90 Tage | 180 Tage |
| 0,03 | 2,0 | 0,93 | 0,04 | -0,11 | -0,08 |
| 0,03 | 4,5 | 0,93 | 0,01 | -0,17 | -0,14 |
| 0,015 | 2,0 | 1,06 | 0,00 | 0,04 | 0,07 |
| 0,015 | 4,5 | 1,06 | -0,08 | -0,04 | -0,03 |
| 0,003 | 2,0 | 1,05 | 0,04 | n.b.[1] | 0,16 |
| 0,003 | 4,5 | 1,05 | 0,01 | n.b.[1] | -0,07 |
| [1]n.b. : nicht bestimmt | | | | | |

Es zeigt sich, dass die generierte Beschichtung auf Silizium sehr dünn ist. Die Schichtdicke liegt für die Startwerte bei annähernd 0,7 bis 0,9 nm, entspricht damit aber in etwa den Literaturwerten (vgl. Plueddemann, E.P., Silane Coupling Agents. 2 ed. 1991, New York: Plenum Publishing Corporatio.) Berücksichtigt man die Streuung bei den Startwerten und bei den Werten zum jeweiligen Auslagerungstag, so kann kein Einlagerungseffekt auf die Schichtdicke abgeleitet werden. Dies betrifft sowohl die Temperaturänderung von Raumtemperatur (25 °C) auf 40 °C, als auch die Änderung des pH-Wertes von 2,0 auf 4,5.

Bezüglich des Einflusses der Beschichtungskonzentration kann die bereits erwähnte Beobachtung in der Entwicklung der Standardabweichung angeführt werden. Die Streuung wird auch hinsichtlich der Schichtdicke mit zunehmender Beschichtungskonzentration moderater, was eine homogenere Schichtausbildung bei höheren Konzentrationen anzeigt.

### 2.3.5.2.2 Veränderungen der Schichtdicke auf Glas bei der Einlagerung bei Raumtemperatur und 40 °C

Es wird die Änderung der Schichtdicke auf Glas bei der Einlagerung bei Raumtemperatur (25 °C) und 40 °C untersucht. Die Ergebnisse der Untersuchungen sind in Tabellen 18 und 19 zusammengefasst.

**Tabelle 18: Veränderungen der Schichtdicke der MTMS-Beschichtung auf Glas bei der Einlagerung unter Raumtemperatur (25°C)**

| Konzentration MTMS [mmol/l] | pH | Schichdicke [nm] | | | |
|---|---|---|---|---|---|
| | | Ausgangswert | Änderung | | |
| | | | 30 Tage | 90 Tage | 180 Tage |
| 0,03 | 2,0 | 1,50 | 0,29 | -0,96 | -0,26 |
| 0,03 | 4,5 | 1,50 | -1,03 | -0,27 | -0,27 |
| 0,015 | 2,0 | 1,43 | -0,10 | -0,12 | -0,13 |
| 0,015 | 4,5 | 1,43 | -0,26 | -0,14 | -0,18 |

(fortgesetzt)

| Konzentration MTMS [mmol/l] | pH | Schichdicke [nm] | | | |
|---|---|---|---|---|---|
| | | Ausgangswert | Änderung | | |
| | | | 30 Tage | 90 Tage | 180 Tage |
| 0,003 | 2,0 | 1,34 | -0,19 | n.b.[1] | -0,36 |
| 0,003 | 4,5 | 1,34 | 0,16 | n.b.[1] | -0,28 |
| [1]n.b. : nicht bestimmt | | | | | |

**Tabelle 19: Veränderungen der Schichtdicke der MTMS-Beschichtung auf Glas bei der Einlagerung unter 40 °C**

| Konzentration MTMS [mmol/l] | pH | Schichdicke [nm] | | | |
|---|---|---|---|---|---|
| | | Ausgangswert | Änderung | | |
| | | | 30 Tage | 90 Tage | 180 Tage |
| 0,03 | 2,0 | 1,50 | -0,68 | -0,49 | -0,64 |
| 0,03 | 4,5 | 1,50 | -0,17 | -0,28 | -0,19 |
| 0,015 | 2,0 | 1,43 | -0,01 | -0,24 | -0,58 |
| 0,015 | 4,5 | 1,43 | -0,35 | -0,09 | -0,30 |
| 0,003 | 2,0 | 1,34 | 0,06 | n. b.[1] | -0,71 |
| 0,003 | 4,5 | 1,34 | 0,03 | n. b.[1] | -0,38 |
| [1] n.b. : nicht bestimmt | | | | | |

Es zeigt sich, dass die Schichtdicke des generierten Filmes auf Glas ist signifikant größer als auf Silizium. Sie beläuft sich, betrachtet man die Startwerte, bei ca. 2 nm und ist damit mehr als doppelt so dick, wie die der generierte Film auf Silizium. Dies wird hier auf eine größere Dichte von Bindungsstellen (insbesondere OH-Bindungen) zwischen dem generierten Film und Glas (im Vergleich zu Silizium) und/oder eine größere Oberflächenrauigkeit des verwendeten Glassubstrats (im Vergleich zum verwendeten Siliziumsubstrat) zurückgeführt.

Wie bereits aufgezeigt wurde, lässt sich auf Glas ein Konzentrationseffekt für die Beschichtung hinsichtlich der Schichtdicke erkennen. Mit zunehmender Konzentration der Beschichtungslösung wird die Schichtdicke tendenziell größer. Allerdings ist dieser Effekt nicht signifikant und wird von einer nicht unerheblichen Streuung überlagert. Selbst für die hier höchste Beschichtungskonzentration von 0,03 mol/l beläuft sich die Streuung in den Startwerten noch immer auf annähernd 0,5 nm.

Es bleibt daher festzuhalten, dass die Schicht nachweisbar ist und sich keine signifikanten Änderungen in der Schichtdicke nachweisen lassen, die sich aufgrund des pH-Wertes oder aber der Temperatur ergeben. Zusätzlich kann ein Einfluss der Beschichtungskonzentration auf die Schichtdicke ermittelt werden. Die Verwendung von höheren Beschichtungskonzentrationen resultierte für Silizium in einer moderateren Streuung der Schichtdickenmesswerte. Für Glas ist tendenziell bei höheren Beschichtungskonzentrationen eine höhere Schichtdicke mit steigender Konzentration zu detektieren (dieser Effekt war innerhalb der Standardabweichungen der ermittelten Messwerte).

**2.3.5.3 Ergebnisse Stabilitätstudie**

Die Stabilitätsstudie ergibt, dass die Beschichtung in den hier getesteten Rahmenbedingungen als stabil zu bezeichnen ist. Dies zeigen die Werte der statischen Kontaktwinkelmessung als auch der ellipsometrischen Schichtdickenmessungen.

Eine vollständige Schichtablösung konnte für die verwendeten Substrate unter den getesteten Stressbedingungen in keinem Falle festgestellt werden, allerdings zeigen die Messungen der statischen Kontaktwinkelmessung auf Silizium durchaus, dass die Schicht über die Einlagerungszeit geringfügig hydrophiler wird. Dies korreliert gut mit den Daten aus den durchgeführten Provokationsversuchen, da auch hier ab einem gewissen Versuchszeitpunkt Gruppe III Sprays bei beschichteten Düsen auftreten, was ebenfalls als ein Indikator für eine mögliche Schichtveränderung angesehen werden kann. Die Kinetik der Zunahme an Gruppe III Sprays bei beschichteten Düsen entspricht nicht der Kinetik

einer unbeschichteten Referenz. Hier zeigt sich stets ein abgeschwächter Verlauf bei der Zunahme an Gruppe III Sprays, was eine noch vorhandene Schichtwirkung anzeigt.

Auch die Ergebnisse der ellipsometrischen Schichtdickenmessungen zeigen eine stabile Schicht bei allen Proben an. Die Schicht ist stets nachweisbar und es lässt sich effektiv keine Abnahme der Schichtdicke erkennen.

Das Ergebnis zeigt nochmals dass mit einem Beschichtungsverfahren die simultane Beschichtung von Glas- und Siliziumsubstraten möglich ist.

### 2.4 Auswirkung der Oberflächenfunktionalisierung auf die Zerstäuber-Performance

Der Einfluss beschichteter Düsen auf die Zerstäuber-Performance wird untersucht. Die untersuchten Düsen werden gemäß Kapitel 1.1 mit 1H,1H,2H,2H-Tridecafluorooctyltriethoxysilan als Beschichtungsmittel umgesetzt.

### 2.4.1 Auswirkung der Beschichtung auf das Priming-Verhalten

Der Einfluss beschichteter Düsen auf das Priming-Verhalten des Zerstäubers wird untersucht.

Als Priming wird die anfängliche Inbetriebnahme des Gerätes bezeichnet. Verglichen werden hierbei die ersten fünf Hübe direkt nach dem Einsetzen des verwendeten, mit Provokationslösung gefüllten Behälters in puncto Delivered Mass und Metered Mass (dies ist hier die Untersuchung des "Priming-Verhaltens", wobei festgestellt werden kann, nach wieviel Hüben die ausgebrachte Masse ihren vollen Wert bzw. Zielwert erreicht hat).

### 2.4.1.1 Verlauf Priming-Verhalten: Delivered Mass (DM) und Metered Mass (MM)

Es wird das Priming-Verhalten von Zerstäubern mit beschichteten und unbeschichteten Düsenkörpern untersucht.

Es zeigt sich, dass hinsichtlich des Verlaufes des Priming-Verhaltens in Bezug auf die Delivered Mass und Metered Mass keinen Unterschied zwischen den Ergebnissen aus den Versuchsgruppen von Zerstäubern mit beschichteten Düsenkörpern und unbeschichteten Düsenkörpern festgestellt werden kann. Die Beschichtung hat somit keinen Auswirkungen auf das Priming-Verhalten.

### 2.4.1.2 Auswirkung auf das Dosierungsverhalten: Vergleich von Delivered Mass und Metered Mass im 120 Tage In-Use Modus

Der Einfluss beschichteter Düsen auf das Dosierungsverhalten wird über eine In-Use-Zeit (Provokationsmodus) von 120 Tagen untersucht. Es wird Verlauf der Delivered Mass (DM) und der Metered Mass (MM) für Zerstäuber 9 mit beschichteten und unbeschichteten Düsen bestimmt. Die Ermittlung der Delivered Mass und Metered Mass kann Kapitel 1.4.3 entnommen werden.

### 2.4.1.2.1 Delivered Mass

Es wird der Verlauf der Delivered Mass für Zerstäuber 9 mit beschichtetem und unbeschichtetem Düsenkörper über einen In-Use-Zeitraum von 120 Tagen untersucht.

Es zeigt sich deutlich, dass es anfänglich keinen Unterschied im Verlauf der Delivered Mass für die Verwendung von Geräten mit beschichteten und unbeschichteten Düsenkörpern gibt. Dies ändert sich jedoch ab Tag 20, da es ab hier in der Versuchsgruppe der Geräte ohne Beschichtung des Düsenkörpers zu einem Anstieg innerhalb der Delivered Mass kommt. Der Anstieg in der Delivered Mass erreicht bis Tag 45 annähernd 12 mg und bleibt damit deutlich oberhalb der mittleren Delivered Mass der Geräte mit Beschichtung des Düsenkörpers.

Verursacht wird dieser Anstieg durch das Aufkommen von Spraybildanomalien in der Gruppe der Zerstäuber ohne Beschichtung des Düsenkörpers. Der Versuch läuft im Provokationsmodus und zeigt mit zunehmendem Verlauf mehr Gruppe II Sprays (Spraybildanomalien) und Gruppe III Sprays in der Referenzgruppe. Plaque-Ablagerungen können durchaus einen Einfluss auf die Delivered Mass haben, indem es zu Abweichungen im Impaktionswinkel kommt (die Sprayanomalien beeinflussen somit die Ausbildung der Impaktionsscheibe bei den DJI-Düsen und damit auch die Aerosol-Rückstreuung bzw. Ausbildung von Resttropfen an der Düse).

Grundsätzlich zeigt sich jedoch, dass die beiden Versuchsgruppen ein absolut vergleichbares Dosierungsverhalten aufzeigen. Erst das Aufkommen der unerwünschten Spraybildanomalien ändert etwas an dieser Tatsache.

### 2.4.1.2.2 Metered Mass

Es wird Verlauf der Metered Mass von Zerstäubern mit beschichteten und unbeschichteten Düsenkörpern über

einen In-Use-Zeitraum von 120 Tagen untersucht.

Die durchgeführten Versuchen zeigen auch hier keinen Unterschied im Verlauf der Metered Mass zwischen Zerstäubern mit beschichteten und unbeschichteten Düsenkörpern, vielmehr sind die Ergebnisse absolut miteinander vergleichbar.

Es kann daher geschlussfolgert werden, dass durch die Beschichtung kein Einfluss auf das Dosierungsverhalten des Zerstäubers festgestellt werden kann. Dies betrifft sowohl das Primingverhalten als auch die Delivered- und Metered Mass.

#### 2.4.2 Auswirkung von beschichteten Düsen auf die Partikelgrößenverteilung

Der Einfluss beschichteter Düsen auf die Partikelgrößenverteilung wird untersucht. Es wird der Verlauf der Partikelgrößenverteilung für Zerstäuber mit beschichteten und unbeschichteten Düsen experimentell bestimmt. Ermittelt wird die Partikelgrößenverteilung über Messungen am verwendeten Andersen Kaskadenimpaktor (gemäß Ph. Eur.) und via Laserbeugung (hierbei wird ein Messgerät vom Typ Helos BF der Fa. Sympatec verwendet).

Die getesteten Zerstäuber mit beschichteten und unbeschichteten Düsenkörpern besitzen eine identische Partikelgrößenverteilung innerhalb der Genauigkeit der jeweiligen Messmethoden.

Zusätzlich wird die Sprayzeit von Zerstäubern, in die beschichtete und unbeschichtete Düsen eingebaut wurden, experimentell bestimmt. Getestet werden jeweils zehn Geräte an denen jeweils fünf Einzelmessungen vorgenommen werden.

Es zeigt sich, dass sich die Sprayzeit für Zerstäuber mit beschichteten und unbeschichteten Düsen nicht signifikant unterscheidet. Für beschichtet Düsen kann eine Sprayzeit von 0,99±0,03 Sekunden und für unbeschichtete Düsen eine Sprayzeit von 0,96±0,03 Sekunden ermittelt werden. Es kann somit im Rahmen der Messgenauigkeit kein Unterschied bei der Sprayzeit für beschichtete und unbeschichtete Düsen festgestellt werden.

#### 2.4.3 Gesamtergebnis bezüglich der Auswirkung der Beschichtung auf die Sprayperformance

Es kann kein signifikanter Unterschied zwischen Zerstäubern mit beschichteter und unbeschichter Düse hinsichtlich der getesteten Device-Parameter festgestellt werden. Die Düsen sind diesbezüglich als identisch zu betrachten.

Das Ergebnis der hier vorliegenden Performance-Analyse von beschichteten und unbeschichteten DüsenkörperDüsenkörpern hat dargelegt, dass die Beschichtung keinen Einfluss auf die hier getesteten Device-Parameter hat. Dies betrifft das Priming-Verhalten, die Dosierungsgenauigkeit, die Partikelgrößenverteilung als auch die Sprayzeit.

Somit erfüllt das hier getestete Beschichtungsverfahren von Düsen bzw. Düsenkörpern eine elementare Voraussetzung für Maßnahmen, die dem Phänomen der Düsenverstopfung entgegen wirken sollen: Ein Einfluss der Beschichtung auf die charakteristischen Funktionsparameter des Zerstäubers kann ausgeschlossen werden.

### 2.5 Untersuchung weiterer Beschichtungsreagenzien: Einfluss der Länge der Alkylseitenkette

Die zuvor beschriebenen Versuche zeigen, dass Beschichtungen auf Basis fluorierter Silane, insbesondere Fluoralkylslane, hervorragend geeignet sind, Düsenverstopfungen bzw. Düsenverlegungen zu vermeiden. Darüber hinaus zeigen auch einige nichtfluorierte Silane, insbesondere Alkylsilane, vielversprechende Ergebnisse.

In nachfolgenden Versuchen sollen alternative, wirksame Beschichtungsmoleküle identifiziert werden.

Der Fokus des folgenden Versuches liegt dabei auf der Analyse des Einflusses der Länge der Alkylseitenkette. Hierfür werden Beschichtungsmoleküle getestet, die sich bezüglich der Alkylseitenkette an der homologen Reihe der Alkane orientieren. Im Zentrum des Versuches stehen hierbei auch wieder Alkylalkoxysilane und Alkyldimethylchlorsilane.

Die ersten Versuche bezüglich der Generierung einer erfolgreichen Beschichtung werden anhand von Silizium/Glas-Flachsubstraten gemacht. Anhand dieser Proben erfolgt anschließend auch die Charakterisierung der Homogenität und der Hydrophobizität der Beschichtung mittels statischer Kontaktwinkelmessungen. Mit Hilfe dieser Daten wird eine Auswahl an Beschichtungsmolekülen bestimmt, die später für einen Provokationsversuch herangezogen wird.

#### 2.5.1 Beschichtung von Silizium und Glas Flachsubstraten

Die Beschichtung der Substrate erfolgt nach dem bereits bekannten Protokoll für Alkylalkoxysilane und Alkylchlorsilane aus Kapitel 1.1. Tabelle 20 gibt eine Übersicht der getesteten Beschichtungsreagenzien.

**Tabelle 20: Auflistung der getesteten alternativen Beschichtungsreagenzien**

| Beschichtungsreagenz | Abkürzung | Beschreibung |
|---|---|---|
| Methyltrimethoxysilan | **C1** | Homologe-Reihe |
| Ethyltrimethoxysilan | **C2** | |
| n-Butyltrimethoxysilan | **C4** | |
| n-Octyltriethoxysilan | **C8** | |
| n-Decyltriethoxysilan | **C10** | |
| n-Dodecyltriethoxysilan | **C12** | |
| Trimethylchlorosilan | **C1-Cl** | Homologe-Reihe |
| Ethyldimethylchlorosilan | **C2-Cl** | |
| n-Butyldimethylchlorosilan | **C4-Cl** | |
| n-Octyldimethylchlorosilan | **C8-Cl** | |
| 1H, 1H, 2H, 2H-Perfluorodecyldimethylchlorosilan | **F13C8-Cl** | perfluoriert |

**2.5.1.1 Screening der Beschichtungsreagenzien anhand statischer Kontaktwinkelmessung**

Tabelle 21 zeigt das Ergebnis der statischen Kontaktwinkelmessungen für Silizium- und Glasflachsubstrate.

**Tabelle 21: Mittelwerte mit Standardabweichung für den Wasser-Kontaktwinkel für die weiteren Beschichtungsreagenzien**

| Beschichtungsreagenz | Glas | | Silizium | |
|---|---|---|---|---|
| | Mittelwert [°] | Standardabweichung [°] | Mittelwert [°] | Standardabweichung [°] |
| Methyltrimethoxysilan | 85,14 | 1,97 | 85,85 | 1,98 |
| Ethyltrimethoxysilan | 88,38 | 2,08 | 88,29 | 1,80 |
| n-Butyltrimethoxysilan | 88,05 | 2,13 | 87,83 | 0,99 |
| n-Octyltriethoxysilan | 108,35 | 1,39 | 103,38 | 1,15 |
| n-Decyltriethoxysilan | 108,15 | 2,17 | 108,96 | 2,09 |
| n-Dodecyltriethoxysilan | 108,19 | 1,89 | 104,45 | 2,88 |
| Trimethylchlorosilan | 91,51 | 4,77 | 86,20 | 6,55 |
| Ethyldimethylchlorosilan | 88,45 | 2,05 | 76,67 | 1,95 |
| n-Butyldimethylchlorosilan | 90,46 | 2,20 | 81,38 | 0,88 |
| n-Octyldimethylchlorosilan | 100,99 | 1,76 | 88,87 | 6,30 |
| 1H, 1H, 2H, 2H-Perfluorodecyldimethylchlorosilan | 115,36 | 2,703 | 110,47 | 1,158 |
| 1 H,1 H,2H,2H-Tridecafluorooctyltriethoxysilan (Referenz) | 110,47 | 2,20 | 105,40 | 2,88 |

Während für die Alkyltrialkoxysilane ein Anstieg des Kontaktwinkels bei zunehmender Länge der Alkylkette zu verzeichnen ist, bleibt diese Beobachtung für die Alkylmonochlorsilane aus. Hier ist eine uneinheitliche Entwicklung bei den Wasser-Kontaktwinkeln in Bezug zur Kettenlänge zu beobachten.

Nahezu allen Reagenzien ist gemein, dass der Kontaktwinkel auf Glas stets etwas höher ist als auf Silizium, d.h. dass für Glasoberflächen eine größere Dichte an Bindungsstellen auf der Oberfläche als für Siliziumoberflächen vorliegt. Den größten Kontaktwinkel erzielt die perfluorierte Beschichtungsreagenz F13C8-Cl. Alle getesteten Silizium-/Glas-Flachsubstrate zeigten stabile Kontaktwinkel mit einer moderaten Standardabweichung. Eine Schichtablösung konnte für kein Substrat festgestellt werden.

Für den im Anschluss stattfindenden Provokationsversuch mit Zerstäuber-Geräten wird anhand der hier getesteten Beschichtungsreagenzien folgende Auswahl getroffen:

- Methyltrimethoxysilan (C1)
- n-Octyltriethoxysilan (C8)
- n-Decyltriethoxysilan (C10)
- n-Dodeycyltriethoxysilan (C12)
- Trimethylchlorsilan (C1-Cl)
- n-Octyldimethylchlorsilan (C8-Cl)
- 1H, 1 H,2H,2H-Perfluorooctyldimethylchlorsilan (F13C8-Cl)

Die Auswahl ermöglicht die Untersuchung des Einflusses der Alkylseitenkette und den Einfluss der Beschichtungschemie. Zusätzlich beinhaltet die Auswahl Beschichtungsreagenzien mit sehr hohen und sehr niedrigen Kontaktwinkeln. Zusätzlich kann mit dieser Auswahl auch überprüft werden, ob der Kontaktwinkel tatsächlich ein geeigneter Bewertungsparameter für die Eignung einer Reagenz zur Beschichtung der betrachteten DJI-Düsen mit Zweck der Verhinderung des Phänomens Sprayanomalien bzw. der "Jet-Divergency" ist.

Die Ergebnisse der Kontaktwinkelstudie legen dar, dass die Beschichtung grundsätzlich auf allen Substraten nachweisbar ist. Alle Beschichtungsreagenzien lieferten stabile Kontaktwinkel oberhalb von 80° und dies auch mit einer sehr moderaten Streuung. Jedoch zeigen die Ergebnisse auch, dass es deutliche Unterschiede im resultierenden Wasser-Kontaktwinkel gibt.

Im Allgemeinen zeigen dichtgepackte, methyl-terminierte Monolayer Wasser-Kontaktwinkel von größer 110° auf. Der Kontaktwinkel wird dabei mit abnehmender Packungsdichte der Moleküle in dem Monolayer kleiner. Dieser Effekt spiegelt sich vermutlich auch hier für die niederkettigen Alkylketten C1, C2 und C4 wieder und ist sowohl für die Alkyltriethoxysilane als auch bei den Alkyldimethylmonochlorsilanen darstellbar. Die Reagenzien zeigen allesamt sowohl auf Silizium- als auch auf Glassubstraten Wasser-Kontaktwinkel unterhalb von 100°.

Es zeigt sich, dass mit zunehmender Kettenlänge der Wasser-Kontaktwinkel ansteigt. Dieser Effekt ist sowohl für Glas als auch für Silizium für beide getesteten Reagenzklassen feststellbar. Auch auf den hier verwendeten Materialien zeigen C10 und C12 die höchsten Kontaktwinkel, was mit goßer Wahrscheinlichkeit auf die höhere Packungsdichte der resultierenden Layer zurückzuführen ist. Sieval et al. veröffentlichte, dass die maximale Beladung von Si(111) generell nur etwa 0,5-0,55 der Molekular Modelling Simulation entspricht (vgl. Sieval, A.B., et al., Molecular modeling of covalently attached alkyl monolayers on the hydrogenterminated Si (111) surface. Langmuir, 2001. 17(7): p. 2172-2181).

### 2.5.2 Performance weiterer Beschichtungsreagenzien in einem Provokationsversuch

Das grundsätzliche Verfahren eines Provokationsversuches kann, wie bereits bekannt, Abschnitt 1.3 entnommen werden.

Das Versuchsdesign des Provokationsversuches ist wie folgt:

Formulierung: entsprechend 1.3.3
Beschichtung:
Alkyltrialkoxyislane:

Methyltrimethoxysilan (C1),
n-Octyltriethoxysilan (C8),
n-Decyltriethoxysilan (C10),
n-Dodeycyltriethoxysilan (C12),
1H,1H,2H,2H-Tridecafluorooctyltriethoxysilan (F13-C8)

Alkyldimethylchlorsilane:

Trimethylchlorsilan (C1-Cl),
n-Octyldimethylchlorsilan (C8-Cl),
1H,1H,2H,2H-Perfluorooctyldimethylchlorsilan(F13C8-Cl),
1H,1H,2H,2H-Perfluorodecyldimethylchlorsilan (F17C10-Cl)

Konzentration: jeweils 0,03 mol /Liter

Anzahl Inhalatoren: 30 Stück für jede Reagenz und Referenz
In-Use Modus: 1x1 Hub/Tag
Testparameter: Sprühbild nach Sprühbildkatalog
Versuchszeit:
Decyltriethoxysilan (C10) und n-Dodeycyltriethoxysilan (C12), 1H,1H,2H,2H-Tridecafluorooctyltriethoxysilan (F13-C8): 120 Tage, alle anderen Reagenzien 28 Tage

**2.5.2.1 Spraybildverlauf Gruppe I Sprays**

Fig. 17 zeigt den Verlauf an Gruppe I Sprays ("Gutsprays") für die beschichteten Düsen und die unbeschichtete Referenz.

Die Abbildung zeigt, dass die langkettigen Alkylalkoxysilane sehr lange eine hohe Anzahl an Gruppe I Sprays ("Gutsprays") gewährleisten, während alle anderen Beschichtungsreagenzien keinen Vorteil gegenüber der unbeschichteten Referenz aufzeigen. Innerhalb der hier getesteten Alkylalkoxysilane liefert das C12 (n-Dodecyltriethoxysilan) die besten Ergebnisse. Es besitzt in diesem Versuch sogar einen leichten Vorteil gegenüber dem perfluorierten F13C8 (1H,1H,2H,2H-Tridecafluorooctyltriethoxysilan). Die Alkylmonochlorsilane schneiden auch in diesem Versuch wieder relativ schlecht ab. Das Versuchsergebnis aus Abschnitt 2.2.3 wird somit bestätigt und lassen sogar auf fluorierten Verbindungen ausgedehnt werden. Auch hier in diesem Versuch gibt es keinen Vorteil gegenüber der unbeschichteten Referenz.

Es offenbart sich somit, dass die beste Sprayperformance durch Beschichtungen mit einem Alkyltrialkoxysilan - sowohl fluoriert als auch unfluoriert - mit einer langen Seitenkette (d.h. hier mit hier getesteten Kettenlängen von $C_{10}$ und $C_{12}$) gewährleistet wird.

**2.5.2.2 Spraybildverlauf Gruppe II Sprays**

Fig. 18 gibt den Verlauf an Gruppe II Sprays (Spraybildanomalien) für die beschichteten Düsen an und die unbeschichtete Referenz.

Figur 18 veranschaulicht sehr deutlich, dass die langkettigen Alkylalkoxysilane wesentlich weniger Gruppe II Sprays produzieren als die Chlorsilane oder aber die kurzkettigen Alkylalkoxysilane. Auch hier zeigt sich, dass sowohl die kurzkettigen Alkoxysilane als auch die gesamten Chlorsilane kaum einen Benefit gegenüber der unbeschichteten Referenz aufzeigen. Die langkettigen Alkylalkoxysilane besitzen einen klaren Vorteil gegenüber allen anderen getesteten Reagenzien.

**2.5.2.3 Spraybildverlauf Gruppe** III **Sprays**

Fig. 19 zeigt den Verlauf an Gruppe III Sprays ("Jet-Divergency") für die beschichteten Düsen und die unbeschichtete Referenz.

Auch bei Betrachtung des Verlaufs der Gruppe III Sprays bestätigt sich der bereits gewonnene Eindruck aus den vorherigen Spraybildverläufen. Die langkettigen Alkylalkoxysilane überwinden nicht einmal über die 120 Tage In-use Zeit die 20 % Marke, während diese bereits nach ca. 20 Tagen von allen anderen getesteten Reagenzien überschritten wird.

**2.5.2.4 Gruppe III Sprays: 10-Tage-Durchschnittsrate gegen Versuchsende**

Der Vorteil der Alkylalkoxysilane gegenüber den Alkylmonochlorsilanen wird besonders noch einmal bei der Betrachtung der 10-Tage-Durchschnittsrate der Gruppe III Sprays, wie in Figur 20 dargestellt, deutlich.

Fig. 20 gibt die 10-Tage Durchschnittsrate an Gruppe III Sprays für die alternativen Beschichtungsreagenzien nach Tag 28 an. Die Referenz ohne Beschichtung erreicht hier eine Rate von annähernd 40 % Gruppe III-Sprays. Ähnlich schlecht sind die Beschichtungen mit C1 (Methyltrimethoxysilan), F13C8-Cl (1H,1 H,2H,2H-Perfluorooctyldimethylchlorsilan) und F17C10-Cl (1H,1H,2H,2H-Perfluorodecyldimethylchlorsilan). Sie zeigen keinen Vorteil hinsichtlich ihrer Beschichtung. Eine sehr gute Performance erzielen die bereits erwähnten Beschichtungsreagenzien F13C8 (1 H,1 H,2H,2H-Tridecafluorooctyltriethoxysilan), C10 (n-Decyltriethoxy) und C12 (n-Dodecyltriethoxysilan). Für das hier ersichtliche Kartuschenintervall (28 Tage Modus) haben F13C8 (1H,1H,2H,2H-Tridecafluorooctyltriethoxysilan) und C12 (n-Dodecyltriethoxysilan) das Auftreten von Gruppe III Sprays gänzlich unterdrücken können.

Das Ergebnis zeigt ein sehr gutes Abschneiden für die Gruppe der Alkyltrialkoxysilane. Das relativ schlechte Abschneiden für die Alkylmonochlorsilane überrascht, zumal stabile Kontaktwinkel in der zuvor durchgeführten Studie erzielt werden konnten. Aus der Gruppe der getesteten Alkylchlorsilane gibt es keinen Vertreter, der eine Rate an Gruppe III Sprays von unterhalb 10 % erreichen kann. Demgegenüber stehen die Alkyltrialkoxysilane, wo es allein drei Vertreter

gibt, die einen Wert unterhalb von 5 % für die 10-Tage Durchschnittsrate an Gruppe III Sprays erreichen.

### 2.5.2.5 Ergebnisse des Provokationstests

Wie das Ergebnis des Provokationsversuches wiedergibt, schneiden die Alkyldimethylmonochlorsilane, analog zu Kapitel 2.2.3 vergleichsweise schlecht ab. Die 10-Tage-Durchschnittrate der Gruppe III Sprays liegt für die Alkyldimethylmonochlorsilane im Vergleich zu den höherkettigen Alkyltrialkoxysilanen um ein vielfaches höher. Überraschenderweise zeigt auch hier das Methyltrimethoxsilan eine mit den Alkyldimethylmonochlorsilanen vergleichbare Performance in diesem Versuch.

Die Gründe für das schlechte Abschneiden des Methyltrimethoxsilans sind nicht eindeutig zu identifizieren. Im Spraybildverlauf zeigt sich jedoch, dass der Verlauf über die gesamte Versuchsdauer sehr nahe im Bereich der unbeschichteten Referenz liegt.

Eine mögliche Erklärung für das schlechte Ergebnis für Methyltrimethoxsilan wäre eine nicht vorhandene oder nicht-ausreichend stabile Beschichtung oder eine unzureichende Packungsdichte.

Die Alkylmonochlorsilane weisen einen vergleichbaren Verlauf mit der unbeschichteten Referenz auf, aber es gibt auch Vertreter, wo sich durchaus ein geringer Schichteinfluss ableiten lässt. Betrachtet man die 10-Tage-Durchschnittrate der Gruppe III Sprays, so ist durchaus ein leichter Effekt für das Trimehtylchlorsilan (C1-Cl) oder aber das n-Octyldimethylmonochlorsilan (C8-Cl) zu identifizieren.

Die Alklytrialkoxysilane besitzen gegenüber den Alkylmonochlorsilanen zwei Koordinantionstellen mehr mit der Oberfläche. Sie sind daher viel stärker an die Oberfläche gebunden, als dies für die Alkyldimethylmonochlorsilane der Fall ist. Dies würde den systematischen Unterschied zwischen den Alkylmonochlorsilane und den Alkyltrialkoxysilanen erklären. Dieser Erklärungsansatz gilt allerdings nicht für das schlechte Abschneiden des Methyltrialkoxysilans, denn diese Reagenz besitzt ebenfalls drei Koordinationsstellen mit der Oberfläche. Vermutet wird hier auch eine Schichterosion oder aber der bereits angesprochene Einfluss der Alkylkettenlänge auf die Packungsdichte.

Die Provokationsversuche ergaben eine gute Eignung von n-Decyltriethoxysilan (C10) und n-Dodeycyltriethoxysilan (C12) als Beschichtungsreagenz. Sie offenbaren vergleichsweise gute Ergebnisse in den Spraybildverläufen wie das 1H,1H,2H,2H-Tridecafluorooctyltriethoxysilan (F13C8). Betrachtet man die 10-Tage-Durchschnittsrate, so ist das n-Dodeycyltriethoxysilan (C12) sogar noch leicht im Vorteil. Diese Ergebnisse zeigen, dass nichtfluorierte Alkylsilane eine ernsthafte Alternative zu fluorierte Silane darstellt. Dies überrascht, resultiert doch gerade aus den Fluoralkylsilanen in der Regel die besseren Anti-Haft-Wirkung (vgl. Giessler, S., E. Just, and R. Störger, Easy-to-clean properties-Just a temporary appearance, Thin Solid Films, 2006. 502(1): p. 252-256). Die Daten aus dem Provokationsversuch zeigen jedoch, dass eine lange Alkylkettenlänge im Bereich von C10 und C12 ausreicht.

Die hier erhobenen Daten führen weiterhin zu dem Ergebnis, dass ein hoher Wasser-Kontaktwinkel allein nicht unbedingt ein Marker für eine wirksame Beschichtungsreagenz ist. Das perfluorierte Chlorsilan F17C10-Cl hat in der Vorabstudie hervorragende Kontaktwinkel im Bereich von 110° erzielt, besaß jedoch eine sehr schlechte Performance im anschließenden Provokationsversuch. Anhand des Kontaktwinkels allein ist es daher nicht möglich die Sprayperformance im Provokationsversuch abzuleiten, da zusätzlich die Stabilität der Beschichtung und deren Packungsdichte in Betracht gezogen werden muß. Ein hoher Kontaktwinkel im Sinne von Ishizaki et al. kann aber ein Indiz für eine hohe Packungsdichte sein.

### 2.5.2.6 Ausbeute an Kategorie I Düsen aus der Beschichtung mit alternativen Beschichtungsreagenzien

Fig. 21 stellt das Ergebnis der mikroskopischen Analyse der beschichteten Düsenkörper gemäß des Düsenkörper-Kategorisierungskatalogs dar.

Fig. 21 ist zu entnehmen, dass unter den getesteten Laborbedingungen das Beschichtungsreagenz F13C8 (1H,1H,2H,2H-Tridecafluorooctyltriethoxysilan) die saubersten Düsen generiert. Mehr als 90 % der hier beschichteten Düsen besaßen eine Kategorie I-Bewertung. Die Ausbeute an Kategorie I Düsen liegt für das C12 (n-Dodecyltriethoxysilan), das im Provokationsversuch sogar besser abgeschnitten hat als das Dynasylan® F8261, nur bei etwa 25 %. Eine durchaus gute Ausbeute von ca. 55 % Kategorie I Düsen erzielt auch das C10 (n-Decyltriethoxysilan).

1H,1H,2H,2H-Tridecafluorooctyltriethoxysilan generiert mit Abstand die meisten sauberen Kategorie I Düsen. In diesem Versuch war die Ausbeute mit 90 Prozent überraschend hoch und wurde durch die zusätzliche Vereinzelung der Düsen bei der Trocknungsphase vor dem Tempern erzielt.

Grundsätzlich sollte sich die Ausbeute an sauberen Düsen über zusätzliche automatisierte Prozessschritte weiter erhöhen lassen. Dies könnte beispielsweise durch Einsatz eines bereits erwähnten "Spin-Rinse-Dryers" erfolgen, der Restlösung aus den Düsen über Rotation heraus treibt. Alternativ oder zusätzlich können Spülprozesse z.B. mit alkolischen Lösemitteln durchgeführt werden. Dies sollte die Ausbeute an sauberen Düsen zusätzlich erhöhen, da bei der weiteren Abtrocknung der Düsenkörper viel weniger Restlösung im Düsenkörper vorhanden ist.

**2.5.2.7 Gesamtergebnis bezüglich weiterer Beschichtungsreagenzien**

Abschließend lässt sich feststellen, dass besten Ergebnisse mit einem langkettigen Alkylalkoxysilan erzielt werden. Hier konnten gute Alternativen zu den fluorierten Verbindungen aufgefunden werden. Dies sind das n-Decyltriethoxysilan (C10) und das n-Dodecyltriethoxysilan (C12).

**Bezugszeichenliste:**

| | | | |
|---|---|---|---|
| 1 | mikrostrukturiertes Bauteil | 14 | Gehäuseinnenteil |
| 2 | Einlassöffnung | 15 | Gehäuseunterteil |
| 3 | Auslassöffnung | 16 | Behälter |
| 4 | Kanäle | 17 | Antriebsfeder |
| 5 | Feinfilter | 18 | Druckerzeuger |
| 6 | Plenumkammer | 19 | Sperring |
| 7 | Säulenstruktur | 20 | Taste |
| 8 | Beschichtung | 21 | Hohlkolben |
| 9 | Zerstäuber | 22 | Rückschlagventil |
| 10 | Flüssigkeit | 23 | Halterung |
| 11 | Aerosol | 24 | Zählwerk |
| 12 | Druckkammer | 25 | Filtersystem |
| 13 | Gehäuseoberteil | | |

**Patentansprüche**

1. Verfahren zur Oberflächenmodifizierung, insbesondere Hydrophobierung, von mikrostrukturierten Bauteilen mit polarer Oberfläche, insbesondere für Hochdruckanwendungen,
**dadurch gekennzeichnet,**
**dass** ein mikrostrukturiertes Bauteil, welches Silizium aufweist, mit einem Modifizierungsreagenz in Kontakt gebracht, insbesondere behandelt, wird, wobei durch chemische und/oder physikalische Wechselwirkung der Bauteiloberfläche und des Modifizierungsreagenzes die Oberflächeneigenschaften des Substrats modifiziert werden, wobei das Modifizierungsreagenz mindestens ein Modifizierungsmittel aufweist, wobei das Modifizierungsreagenz das Modifizierungsmittel in Konzentrationen von 0,001 bis 2 mol/l, bezogen auf das Modifizierungsreagenz, enthält und wobei als Modifizierungsmittel ein Silan gemäß der allgemeinen Formel I

$$R^1_{4-(n+m)}SiR^2_mX_n \qquad (I)$$

mit

$R^1$ = $C_8$- bis $C_{18}$-Alkyl;
$R^2$ = $C_1$- bis $C_3$-Alkyl;
X = Halogenid; Alkoxy; und
n = 1 bis 3 und
m = 0 bis 2,

eingesetzt wird.

2. Verfahren nach Anspruch 1, **dadurch gekennzeichnet, dass** als Modifizierungsmittel ein Silan gemäß der allgemeinen Formel I

$$R^1_{4-(n+m)}SiR^2_mX_n \qquad (I)$$

mit

$R^1$ = $C_{10}$- bis $C_{16}$-Alkyl;
$R^2$ = $C_1$- bis $C_3$-Alkyl, vorzugsweise Methyl;
X = Halogenid, insbesondere Chlorid und/oder Bromid, vorzugsweise Chlorid; Alkoxy, insbesondere $C_1$- bis

$C_6$-Alkoxy, besonders bevorzugt $C_1$- bis $C_4$- Alkoxy, ganz besonders bevorzugt $C_1$- und/oder $C_2$-Alkoxy; und
n = 1 bis 3, insbesondere 3, und
m = 0 bis 2, insbesondere 0 oder 2, vorzugsweise 0,

eingesetzt wird.

3. Verfahren nach Anspruch 1 oder 2, **dadurch gekennzeichnet, dass** das Silan drei reaktive chemische Funktionen und/oder Gruppen, insbesondere drei hydrolysierbare chemische Funktionen und/oder Gruppen, aufweist, vorzugsweise ein Trialkoxysilan ist, insbesondere wobei das Silan ein Alkyltrialkoxysilan ist, insbesondere ausgewählt aus Gruppe von $C_{12}$-Alkyltrilalkoxysilanen, $C_{14}$-Alkyltrilalkoxysilanen und $C_{16}$-Alkyltrilalkoxysilanen sowie deren Mischungen.

4. Verfahren nach einem der vorangehenden Ansprüche, **dadurch gekennzeichnet, dass** das Bauteil mindestens zwei unterschiedlichen Materialien, insbesondere Silizium und Glas, insbesondere silikatisches Glas, vorzugsweise Quarzglas und/oder Borosilikatglas, bevorzugt Borosilikatglas, aufweist, insbesondere wobei die Materialien polare Oberflächen aufweisen.

5. Verfahren nach Anspruch 4, **dadurch gekennzeichnet, dass** die Oberflächen der Materialien des Bauteils gemeinsam modifiziert werden.

6. Verfahren nach einem der vorangehenden Ansprüche **dadurch gekennzeichnet, dass** die gesamte Oberfläche des Bauteils modifiziert wird.

7. Verfahren nach einem der vorangehenden Ansprüche, **dadurch gekennzeichnet, dass** durch das Inkontaktbringen des Modifizierungsreagenzes mit der Oberfläche des Bauteils eine Schicht, insbesondere eine Hydrophobierungsschicht, auf die Oberfläche des Bauteils aufgebracht wird, insbesondere wobei die Schicht über chemische Bindungen, insbesondere über kovalente Bindungen, an das Bauteil gebunden ist.

8. Verfahren nach einem der vorangehenden Ansprüche, **dadurch gekennzeichnet, dass** als Modifizierungsmittel ein Silan gemäß der allgemeinen Formel VII

$$R_{4-n}SiX_n \qquad \text{(VII)}$$

mit

R = $C_8$- bis $C_{18}$-Alkyl, bevorzugt $C_{10}$- bis $C_{16}$-Alkyl;
X = Alkoxy, bevorzugt $C_1$- bis $C_4$- Alkoxy, ganz besonders bevorzugt $C_1$- und/oder $C_2$-Alkoxy; und
n = 3,

eingesetzt wird.

9. Mikrostrukturiertes Bauteil, insbesondere Düsensystem, bevorzugt zum Einsatz in einem mikrofluiden System, aufweisend mindestens eine Einlassöffnung, mindestens eine Auslassöffnung sowie durch Mikrostrukturen gebildete innere Oberflächen, **dadurch gekennzeichnet, dass** die inneren Oberflächen zumindest teilweise mit einem Modifizierungsmittel in Form eines Silans gemäß der allgemeinen Formel I

$$R^1_{4-(n+m)}SiR^2_mX_n \qquad \text{(I)}$$

mit

$R^1$ = $C_8$- bis $C_{18}$-Alkyl;
$R^2$ = $C_1$- bis $C_3$-Alkyl;
X = Halogenid; Alkoxy; und
n = 1 bis 3 und
m = 0 bis 2,

modifiziert, insbesondere beschichtet, sind.

**10.** Mikrostrukturiertes Bauteil nach Anspruch 9, **dadurch gekennzeichnet, dass** das Bauteil weiterhin einen Filterbereich aufweist, vorzugsweise zwischen der Einlassöffnung und der Auslassöffnung.

**11.** Mikrostrukturiertes Bauteil nach Anspruch 9, **dadurch gekennzeichnet, dass** das Bauteil aus mindestens zwei unterschiedlichen Materialen besteht, insbesondere Glas und Silizium beinhaltet, insbesondere wobei die unterschiedlichen Materialien fest miteinander verbunden, insbesondere gebondet, sind.

**12.** Austragsvorrichtung, insbesondere Zerstäuber, für Fluide, insbesondere medizinische Flüssigkeiten, vorzugsweise flüssige Arzneimittel, aufweisend mindestens ein mikrostrukturiertes Bauteil nach einem der Ansprüche 9 bis 11.

**13.** Austragsvorrichtung nach Anspruch 12, aufweisend mindestens ein flüssiges Arzneimittel, insbesondere wobei das Arzneimittel eine Dispersion oder Lösung mindestens eines pharmazeutischen Wirkstoffs ist.

**14.** Austragsvorrichtung nach Anspruch 13, **dadurch gekennzeichnet, dass** der pharmazeutische Wirkstoff ausgewählt ist aus der Gruppe von Terbutalin, Salbutamol, Trospium, insbesondere Trospiumchlorid, Flutropium, insbesondere Flutropiumbromid, Tiotropium, insbesondere Tiotropiumbromid, Oxitropium, insbesondere Oxitropiumbromid, Ipratropium, insbesondere Ipratropiumbromid, Fenoterol, Budesonid, Fluticason, insbesondere Fluticasonpropionat, Glycopyrronium, insbesondere Glycopyrroniumbromid, Ciclesonid, und Beclometason, insbesondere, Beclometasondipropionat, sowie deren physiologisch verträglichen Salzen und Derivaten.

## Claims

**1.** Method for surface modifying, in particular hydrophobing, of microstructured components having a polar surface, in particular for high-pressure applications,
**characterized in that**
a microstructured component comprising silicon is brought into contact, in particular treated, with a modification reagent, the surface properties of the substrate being modified by chemical and/or physical interaction between the component surface and the modification reagent, the modification reagent comprising at least one modifier, the modification reagent containing the modifier in concentrations of from 0.001 to 2 mol/l, based on the modification reagent, and wherein as the modifier a silane corresponding to the general formula I

$$R^1_{4-(n+m)}SiR^2_mX_n \qquad (I)$$

with

$R^1$ = $C_8$- to $C_{18}$-alkyl;
$R^2$ = $C_1$- to $C_3$-alkyl;
X = halide; alkoxy; and
n = 1 to 3 and
m = 0 to 2,

is used.

**2.** Method according to claim 1, **characterized in that** as the modifier a silane according to the general formula I

$$R^1_{4-(n+m)}SiR^2_mX_n \qquad (I)$$

with

$R^1$ = $C_{10}$- to $C_{16}$-alkyl;
$R^2$ = $C_1$- to $C_3$-alkyl, preferably methyl;
X = halide, in particular chloride and/or bromide, preferably chloride; alkoxy, in particular $C_1$- to $C_6$-alkoxy, preferably $C_1$- to $C_4$-alkoxy, more preferably $C_1$- and/or $C_2$-alkoxy; and
n = 1 to 3, in particular 3, and
m = 0 to 2, in particular 0 or 2, preferably 0,

is used.

3. Method according to claim 1 or 2, **characterized in that** the silane comprises three reactive chemical functions and/or groups, in particular three hydrolysable chemical functions and/or groups, preferably is a trialkoxysilane, in particular wherein the silane is an alkyltrialkoxysilane, in particular selected from the group of $C_{12}$-alkyltrilalkoxysilanes, $C_{14}$-alkyltrilalkoxysilanes and $C_{16}$-alkyltrilalkoxysilanes and mixtures thereof.

4. Method according to one of the preceding claims, **characterized in that** the component comprises at least two different materials, in particular silicon and glass, preferably silicate glass, more preferably quartz glass and/or borosilicate glass, particularly preferably borosilicate glass, in particular wherein the materials comprise polar surfaces.

5. Method according to claim 4, **characterized in that** the surfaces of the materials of the component are modified together.

6. Method according to one of the preceding claims, **characterized in that** the entire surface of the component is modified.

7. Method according to one of the preceding claims, **characterized in that** a layer, in particular a hydrophobing layer, is applied to the surface of the component by bringing the modification reagent into contact with the surface of the component, in particular wherein the layer is bound to the component by means of chemical bonds, in particular by means of covalent bonds.

8. Method according to one of the preceding claims, **characterized in that** as the modifier a silane according to the general formula VII

$$R_{4-n}SiX_n \qquad (VII)$$

with

R = $C_8$- to $C_{18}$-alkyl, preferably $C_{10}$- to $C_{16}$-akyl;
X = alkoxy, preferably $C_1$- to $C_4$-alkoxy, most preferably $C_1$- and/or $C_2$-alkoxy; and
n = 3,

is used.

9. Microstructured component, in particular nozzle system, preferably for use in a microfluidic system, comprising at least one inlet opening, at least one outlet opening and inner surfaces formed by microstructures, **characterized in that** the inner surfaces are at least partially modified, in particular coated, with a modifier in the form of a silane according to the general formula I

$$R^1_{4-(n+m)}SiR^2_mX_n \qquad (I)$$

with

$R^1$ = $C_8$- to $C_{18}$-alkyl;
$R^2$ = $C_1$- to $C_3$-alkyl;
X = halide; alkoxy; and
n = 1 to 3 and
m = 0 to 2.

10. Microstructured component according to claim 9, **characterized in that** the component further comprises a filter region, preferably between the inlet opening and the outlet opening.

11. Microstructured component according to claim 9, **characterized in that** the component consists of at least two different materials, in particular comprising glass and silicon, in particular wherein the different materials are rigidly interconnected, in particular bonded.

12. Discharge apparatus, in particular an atomizer, for fluids, in particular medicinal liquids, preferably liquid medicinal products, comprising at least one microstructured component according to any of claims 9 to 11.

13. Discharge apparatus according to claim 12, comprising at least one liquid medicinal product, in particular wherein the medicinal product is a dispersion or solution of at least one pharmaceutical active ingredient.

14. Discharge apparatus according to claim 13, **characterized in that** the pharmaceutical active ingredient is selected from the group consisting of terbutaline, salbutamol, trospium, in particular trospium chloride, flutropium, in particular flutropium bromide, tiotropium, in particular tiotropium bromide, oxitropium, in particular oxitropium bromide, ipratropium, in particular ipratropium bromide, fenoterol, budesonide, fluticasone, in particular fluticasone propionate, glycopyrronium, in particular glycopyrronium bromide, ciclesonide, and beclometasone, in particular beclometasone dipropionate, and the physiologically compatible salts and derivatives thereof.

## Revendications

1. Procédé de modification de surface, en particulier d'hydrophobisation, de composants microstructurés à surfaces polaires, en particulier pour des applications haute pression,
**caractérisé en ce qu'**un composant microstructuré, qui présente du silicium, est mis en contact, notamment traité, avec un réactif de modification, les propriétés de surface du substrat étant modifiées par interaction chimique et/ou physique de la surface du composant et du réactif de modification, le réactif de modification présentant au moins un agent de modification, le réactif de modification contenant l'agent de modification à des concentrations de 0,001 à 2 mol/l, rapporté au réactif de modification, et un silane selon la formule générale I

$$R^1_{4-(n+m)}SiR^2_mX_n \qquad (1)$$

avec

$R^1$ = alkyle en $C_8$ à $C_{18}$ ;
$R^2$ = alkyle en $C_1$ à $C_3$ ;
X = halogénure ; alcoxy ; et
n = 1 à 3 et
m = 0 à 2,

étant utilisé comme agent de modification.

2. Procédé selon la revendication 1, **caractérisé en ce qu'**un silane selon la formule générale I

$$R^1_{4-(n+m)}SiR^2_mX_n \qquad (1)$$

avec

$R^1$ = alkyle en $C_{10}$ à $C_{16}$ ;
$R^2$ = alkyle en $C_1$ à $C_3$, de préférence méthyle ;
X = halogénure, en particulier chlorure et/ou bromure, de préférence chlorure ; alcoxy, en particulier alcoxy en $C_1$ à $C_6$, de manière particulièrement préférée alcoxy en $C_1$ à $C_4$, de manière tout particulièrement préférée alcoxy en $C_1$ et/ou $C_2$ ; et
n = 1 à 3, en particulier 3 et
m = 0 à 2, en particulier 0 ou 2, de préférence 0,

est utilisé comme agent de modification.

3. Procédé selon la revendication 1 ou 2, **caractérisé en ce que** le silane présente trois fonctions et/ou groupes chimiques réactifs, en particulier trois fonctions et/ou groupes chimiques hydrolysables, est de préférence un trialcoxysilane, en particulier le silane étant un alkyltrialcoxysilane, en particulier choisi dans le groupe des alkyltrialcoxysilanes en $C_{12}$, des alkyltrialcoxysilanes en $C_{14}$ et des alkyltrialcoxysilanes en $C_{16}$ et leurs mélanges.

4. Procédé selon l'une quelconque des revendications précédentes, **caractérisé en ce que** le composant présente au moins deux matériaux différents, en particulier du silicium et du verre, en particulier du verre de silicate, de préférence du verre de quartz et/ou du verre borosilicaté, de préférence du verre borosilicaté, en particulier lorsque

les matériaux ont des surfaces polaires.

5. Procédé selon la revendication 4, **caractérisé en ce que** les surfaces des matériaux du composant sont modifiées ensemble.

6. Procédé selon l'une quelconque des revendications précédentes, **caractérisé en ce que** toute la surface du composant est modifiée.

7. Procédé selon l'une quelconque des revendications précédentes, **caractérisé en ce que** par mise en contact du réactif de modification avec la surface du composant, une couche, en particulier une couche hydrophobe, est appliquée sur la surface du composant, en particulier la couche étant fixée au composant par des liaisons chimiques, en particulier par des liaisons covalentes.

8. Procédé selon l'une quelconque des revendications précédentes, **caractérisé en ce qu'**un silane selon la formule générale VII

$$R_{4-n}SiX_n \qquad (VII)$$

avec

R = alkyle en $C_8$ à $C_{18}$, de préférence alkyle en $C_{10}$ à $C_{16}$ ;
X = alcoxy, de préférence alcoxy en $C_1$ à $C_4$, de manière tout particulièrement préférée alcoxy en $C_1$ et/ou $C_2$ ; et
n = 3,

est utilisé comme agent de modification.

9. Composant microstructuré, en particulier système de buses, de préférence destiné à être utilisé dans un système microfluidique, présentant au moins une ouverture d'entrée, au moins une ouverture de sortie et des surfaces internes formées par des microstructures, **caractérisé en ce que** les surfaces internes sont au moins partiellement modifiées, en particulier revêtues, par un agent de modification sous forme de silane selon la formule générale I

$$R^1_{4-(n+m)}SiR^2_mX_n \qquad (1)$$

avec

$R^1$ = alkyle en $C_8$ à $C_{18}$ ;
$R^2$ = alkyle en $C_1$ à $C_3$ ;
X = halogénure ; alcoxy ; et
n = 1 à 3 et
m = 0 à 2.

10. Composant microstructuré selon la revendication 9, **caractérisé en ce que** le composant présente en outre une zone de filtre, de préférence entre l'ouverture d'entrée et l'ouverture de sortie.

11. Composant microstructuré selon la revendication 9, **caractérisé en ce que** le composant est constitué d'au moins deux matériaux différents, **en ce qu'**il contient en particulier du verre et du silicium, en particulier les différents matériaux étant solidaires l'un de l'autre, et en particulier liés.

12. Dispositif de décharge, en particulier atomiseur, de fluides, en particulier de liquides médicaux, de préférence de médicaments liquides, présentant au moins un composant microstructuré selon l'une des revendications 9 à 11.

13. Dispositif de décharge selon la revendication 12, présentant au moins un médicament liquide, en particulier le médicament étant une dispersion ou solution d'au moins un principe actif pharmaceutique.

14. Dispositif de décharge selon la revendication 13, **caractérisé en ce que** le principe actif pharmaceutique est choisi dans le groupe de la terbutaline, du salbutamol, du trospium, en particulier du chlorure de trospium, du flutropium, en particulier du bromure de flutropium, du tiotropium, en particulier du bromure de tiotropium, de l'oxitropium, en particulier du bromure d'oxitropium, de l'ipratropium, en particulier du bromure d'ipratropium, du fénotérol, du budésonide, de la fluticasone, en particulier du propionate de fluticasone, du glycopyrronium, en particulier du bromure de glycopyrronium, du ciclésonide et de la béclométasone, en particulier du dipropionate de béclométasone et leurs sels et dérivés physiologiquement acceptables.

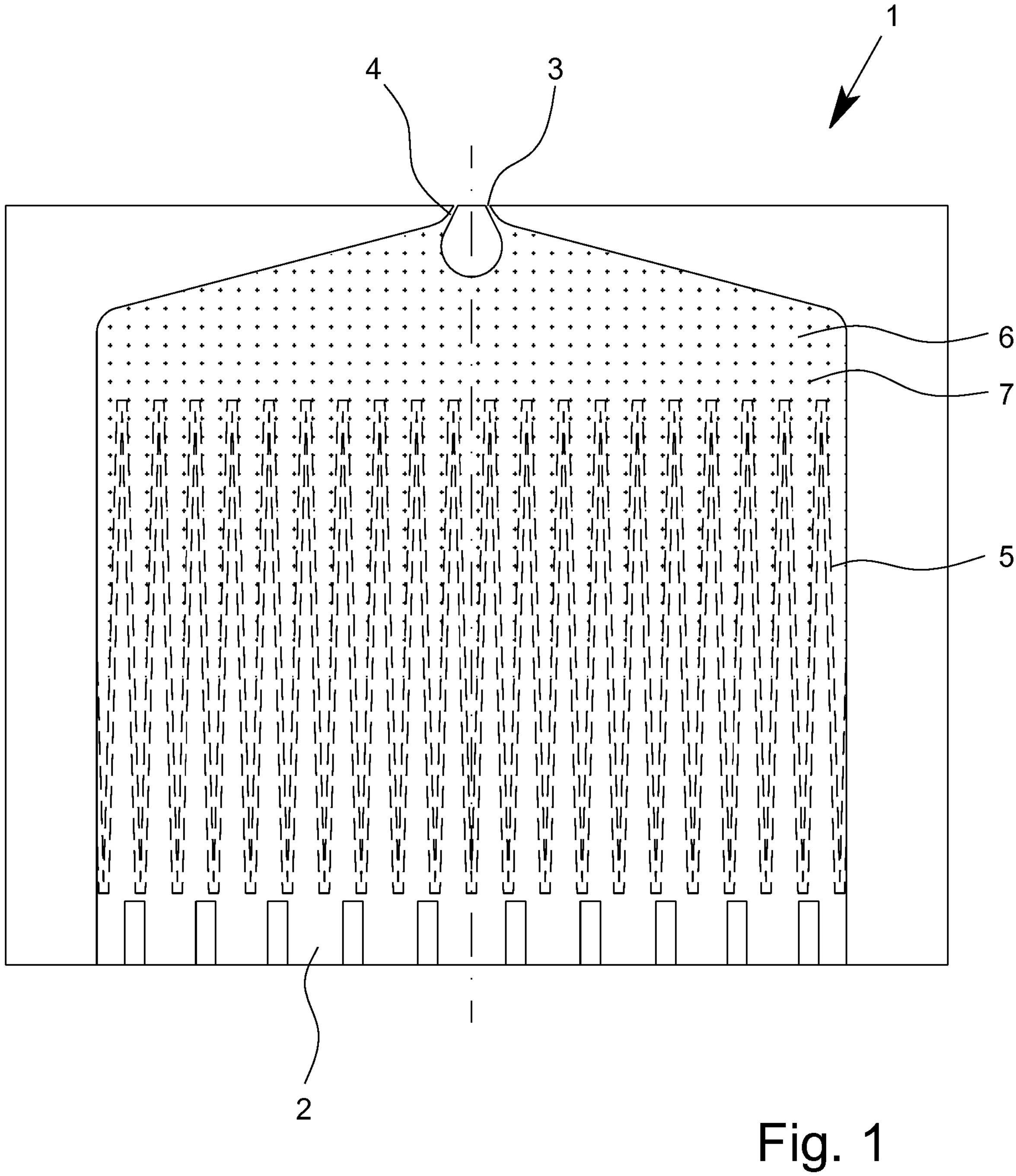


Fig. 1

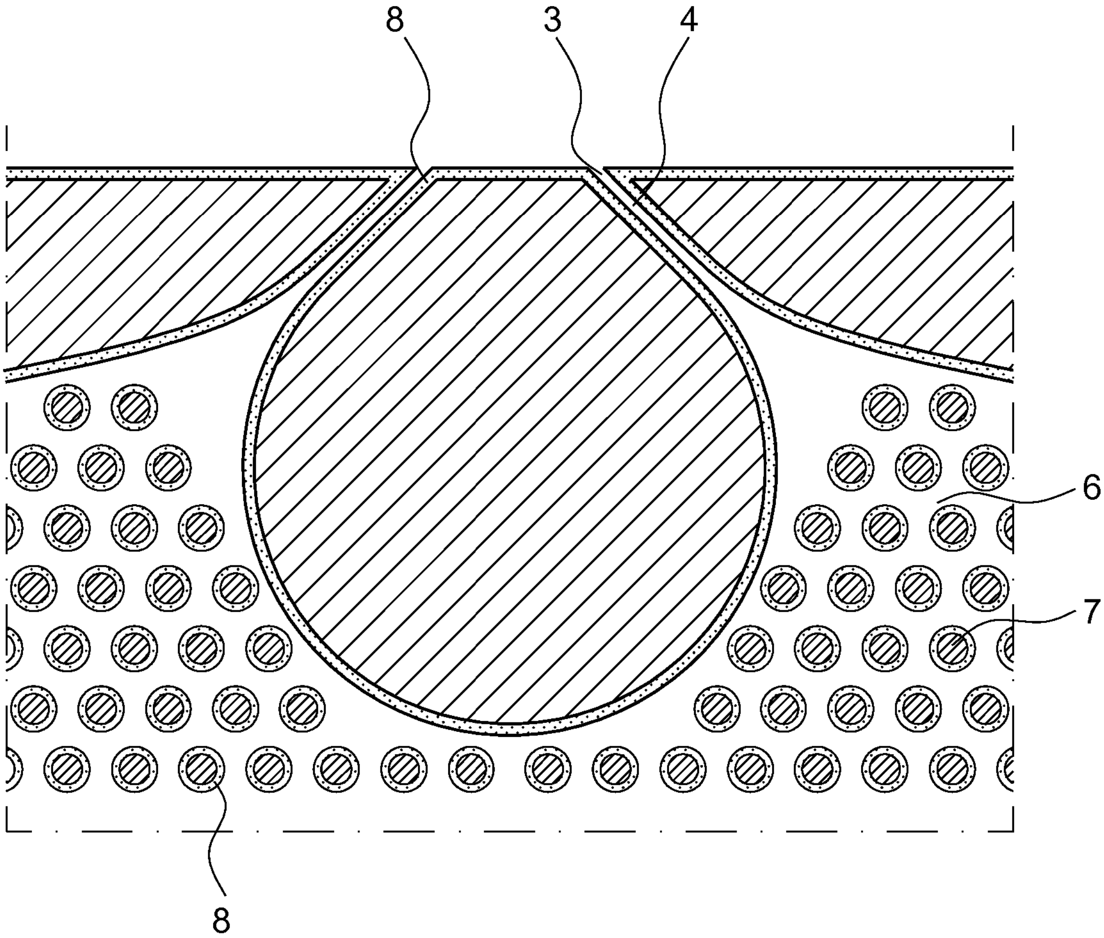


Fig. 2

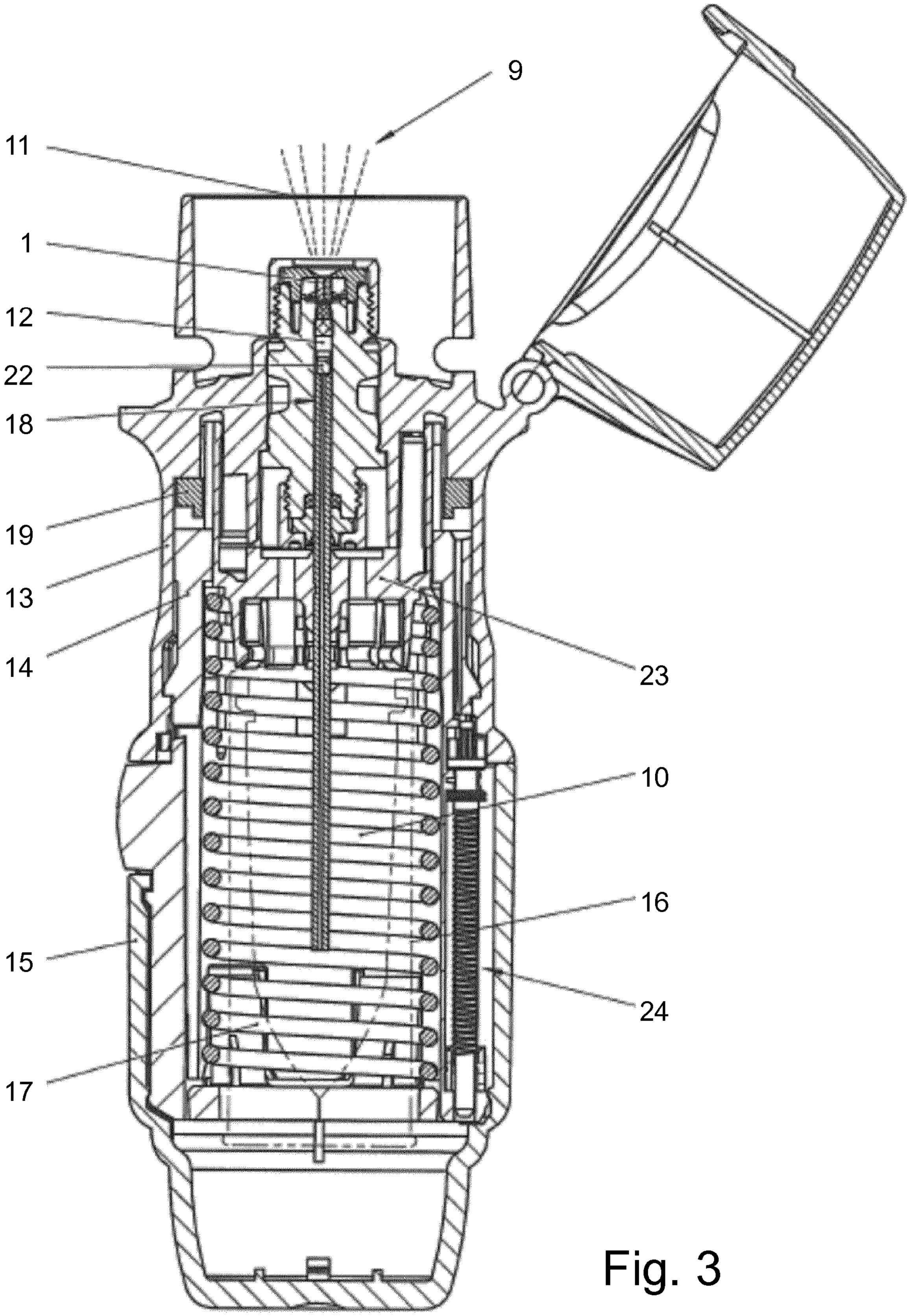


Fig. 3

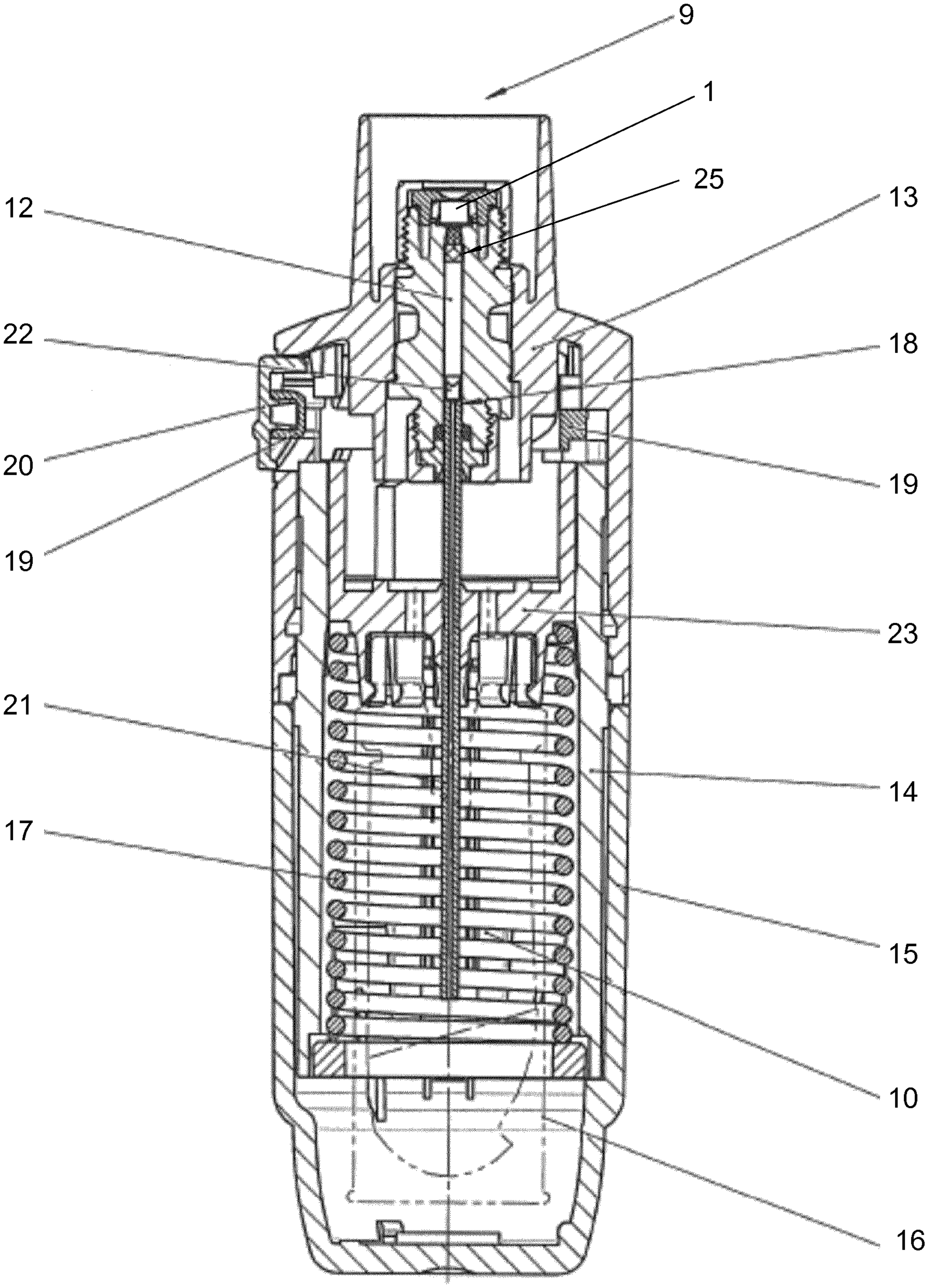


Fig. 4

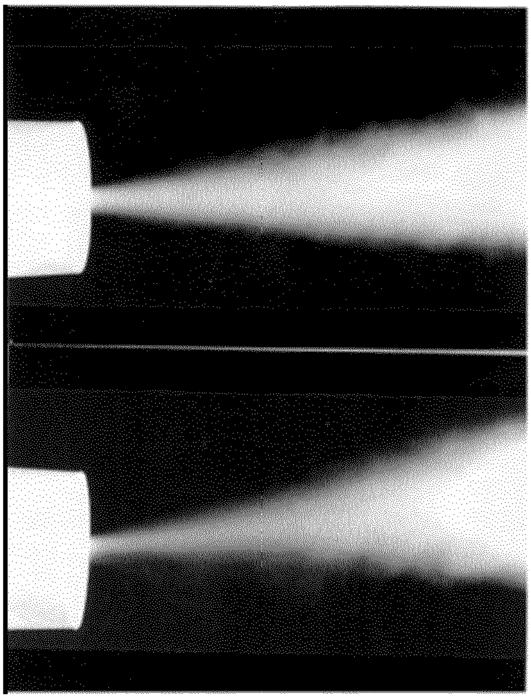

Fig. 5

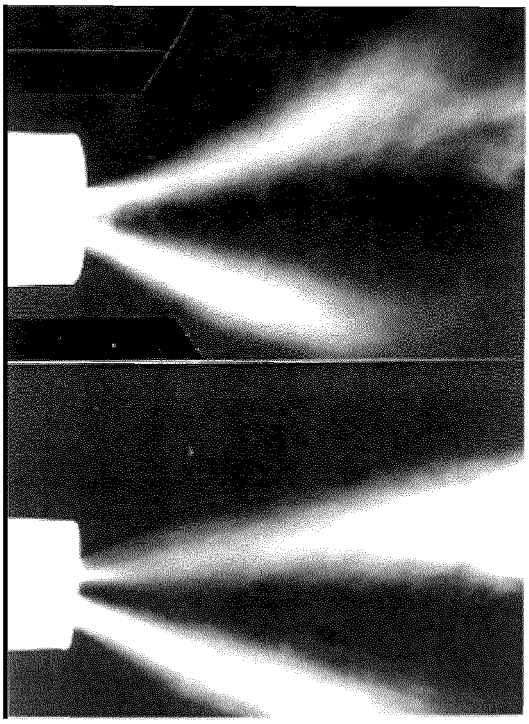

Fig. 6

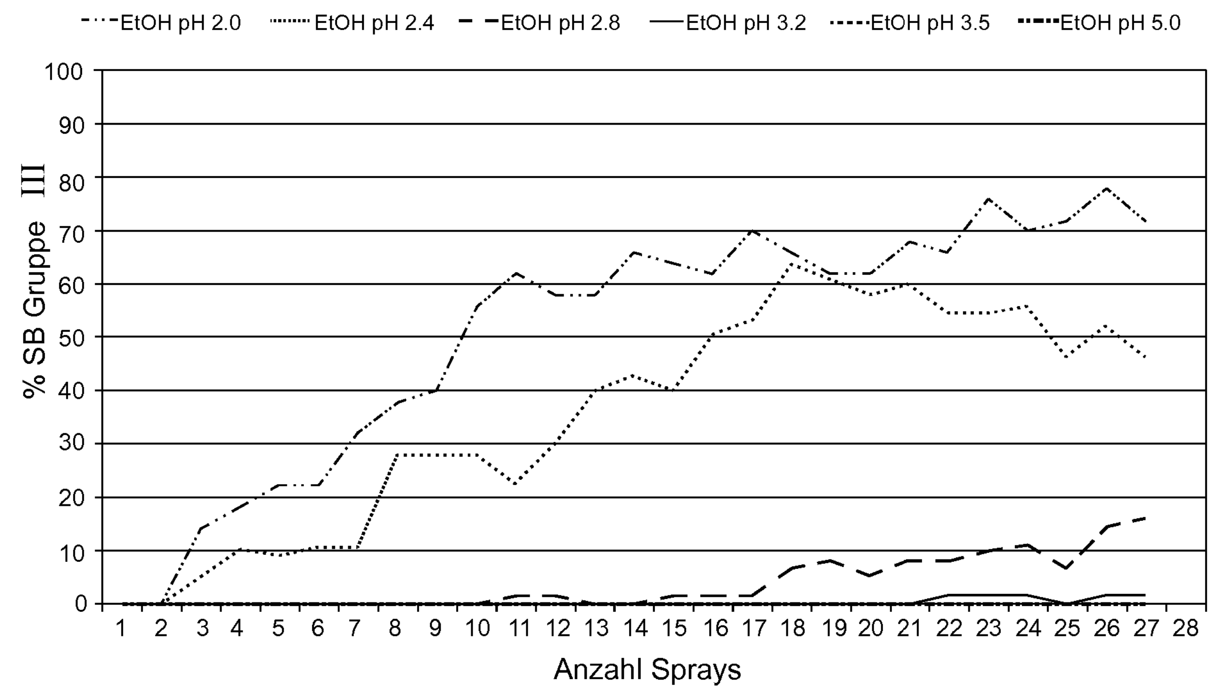
EtOH pH 2.0
EtOH pH 2.4
EtOH pH 2.8
EtOH pH 3.2
EtOH pH 3.5
EtOH pH 5.0
% SB Gruppe III
0
10
20
30
40
50
60
70
80
90
100
1 2 3 4 5 6 7 8 9 10 11 12 13 14 15 16 17 18 19 20 21 22 23 24 25 26 27 28
Anzahl Sprays

Fig. 7

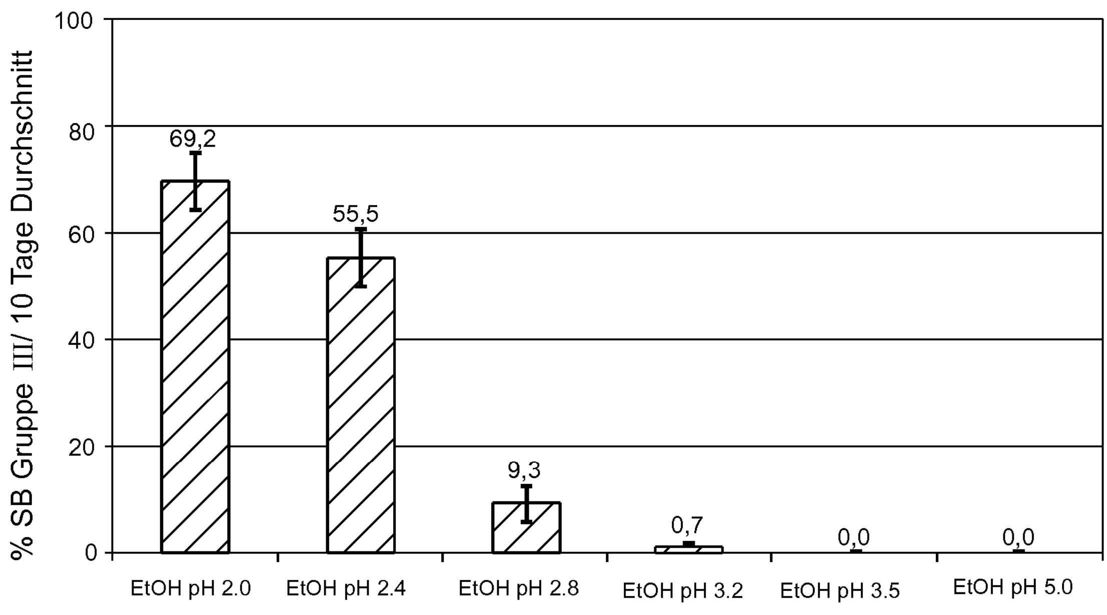
% SB Gruppe III/ 10 Tage Durchschnitt
0
20
40
60
80
100
69,2
55,5
9,3
0,7
0,0
0,0
EtOH pH 2.0
EtOH pH 2.4
EtOH pH 2.8
EtOH pH 3.2
EtOH pH 3.5
EtOH pH 5.0

Fig. 8

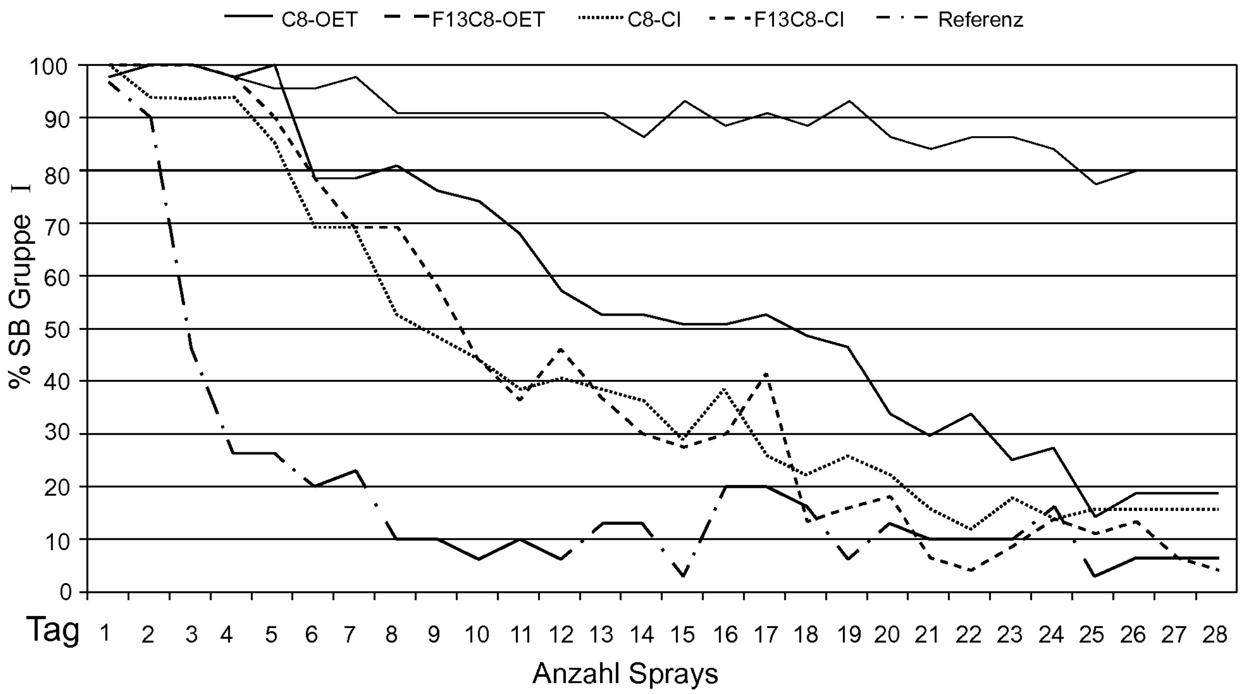
C8-OET
F13C8-OET
C8-CI
F13C8-CI
Referenz
% SB Gruppe I
Tag
Anzahl Sprays

Fig. 9

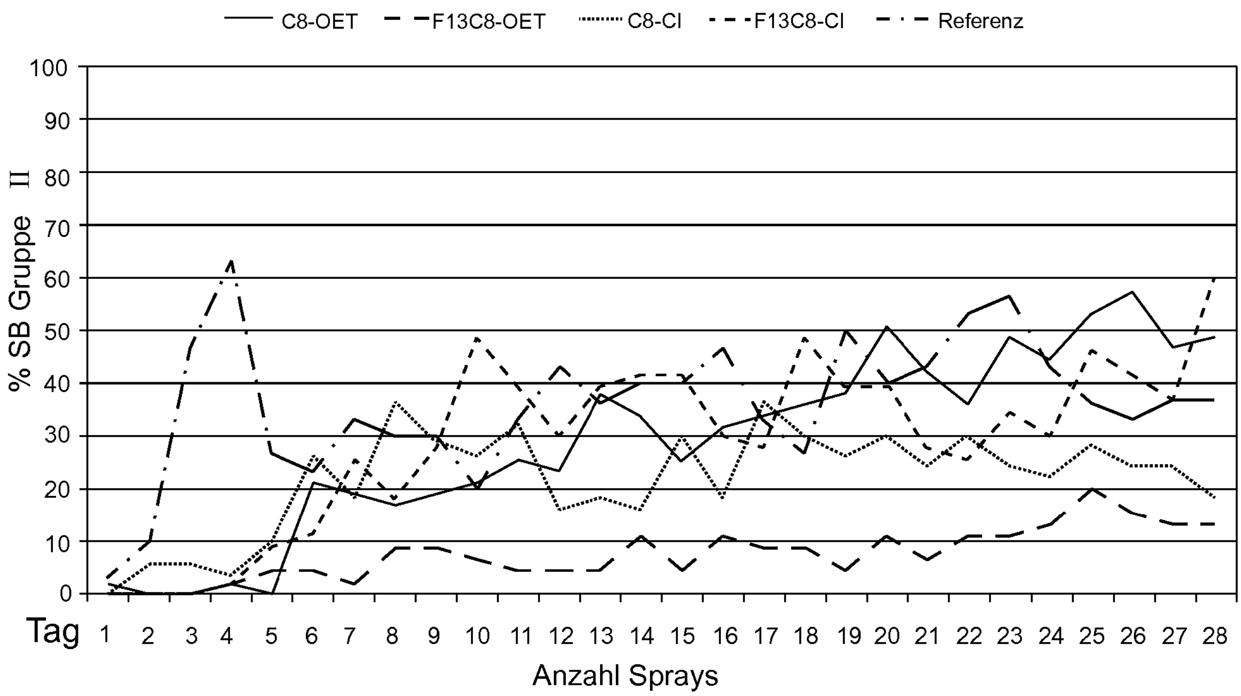
C8-OET
F13C8-OET
C8-CI
F13C8-CI
Referenz
% SB Gruppe II
Tag
Anzahl Sprays

Fig. 10

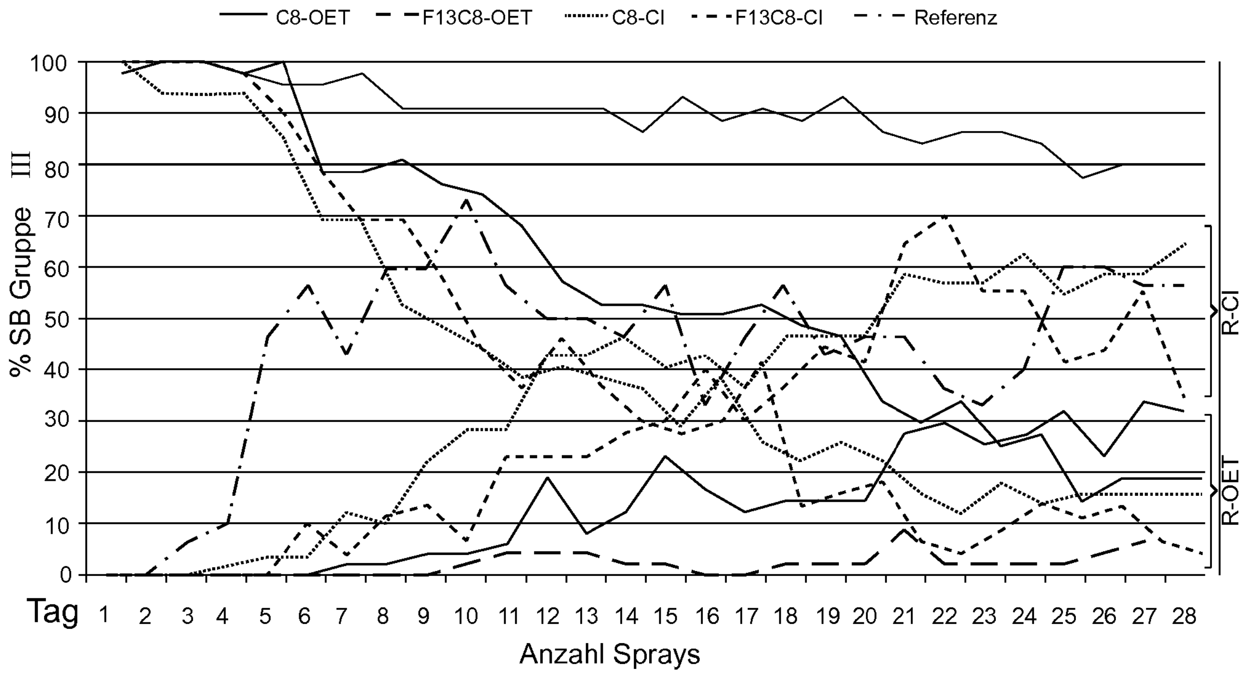
C8-OET
F13C8-OET
C8-CI
F13C8-CI
Referenz
% SB Gruppe III
100
90
80
70
60
50
40
30
20
10
0
Tag 1 2 3 4 5 6 7 8 9 10 11 12 13 14 15 16 17 18 19 20 21 22 23 24 25 26 27 28
Anzahl Sprays
R-CI
R-OET

Fig. 11

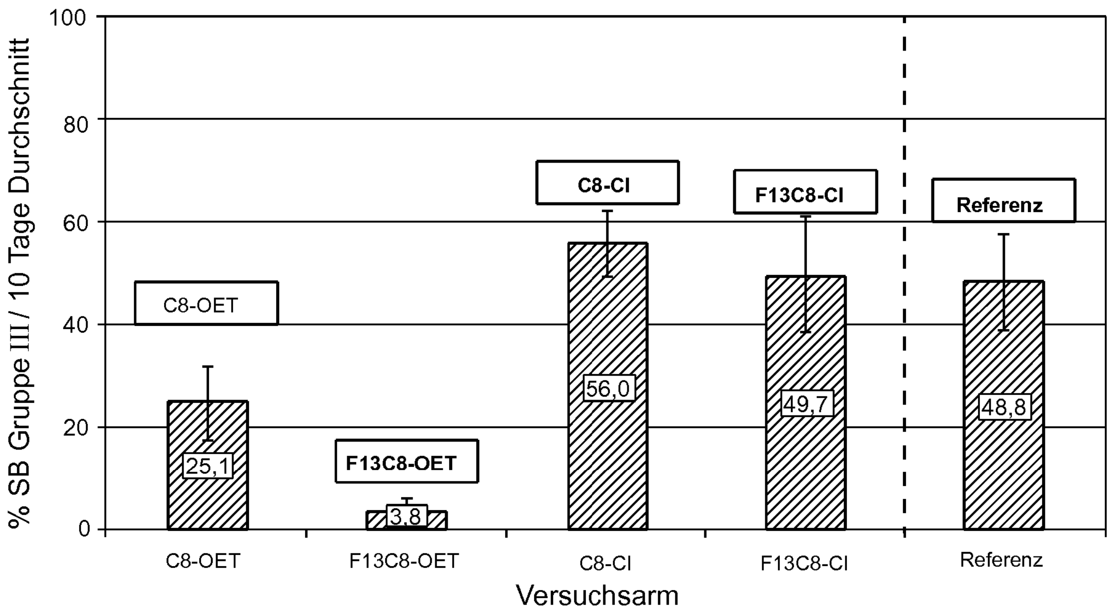
% SB Gruppe III / 10 Tage Durchschnitt
100
80
60
40
20
0
C8-OET
F13C8-OET
C8-CI
F13C8-CI
Referenz
25,1
3,8
56,0
49,7
48,8
C8-OET
F13C8-OET
C8-CI
F13C8-CI
Referenz
Versuchsarm

Fig. 12

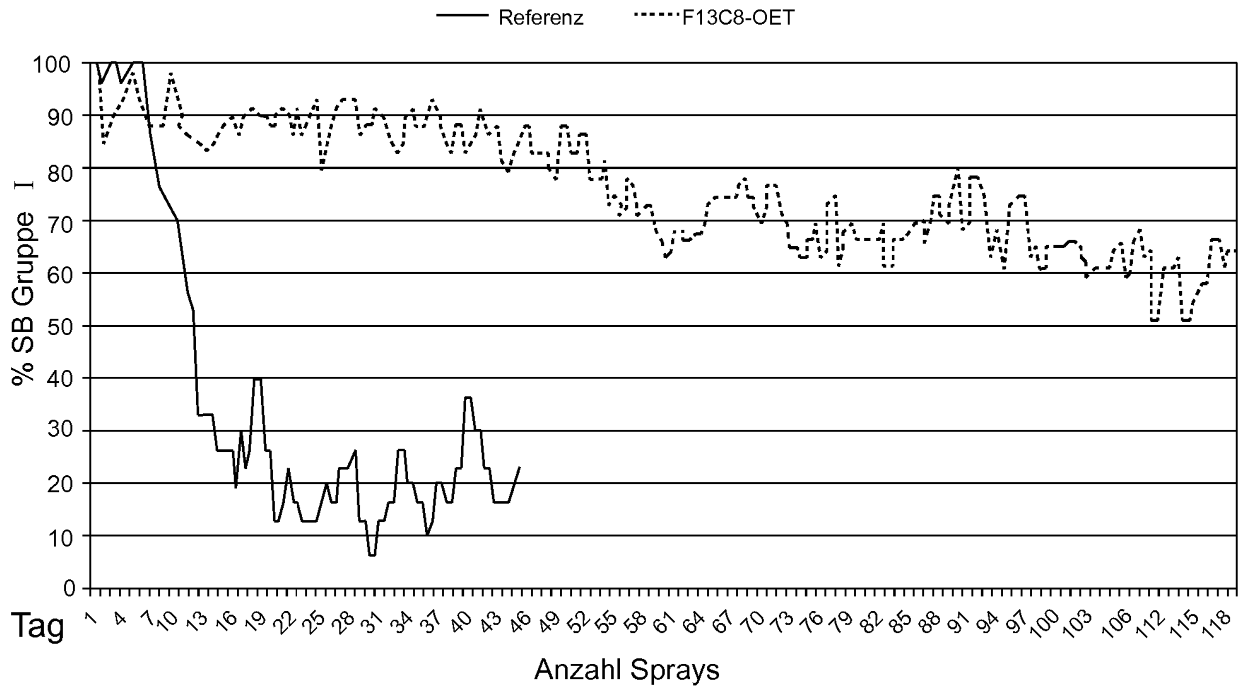
Referenz
F13C8-OET
% SB Gruppe I
Tag
Anzahl Sprays

Fig. 13

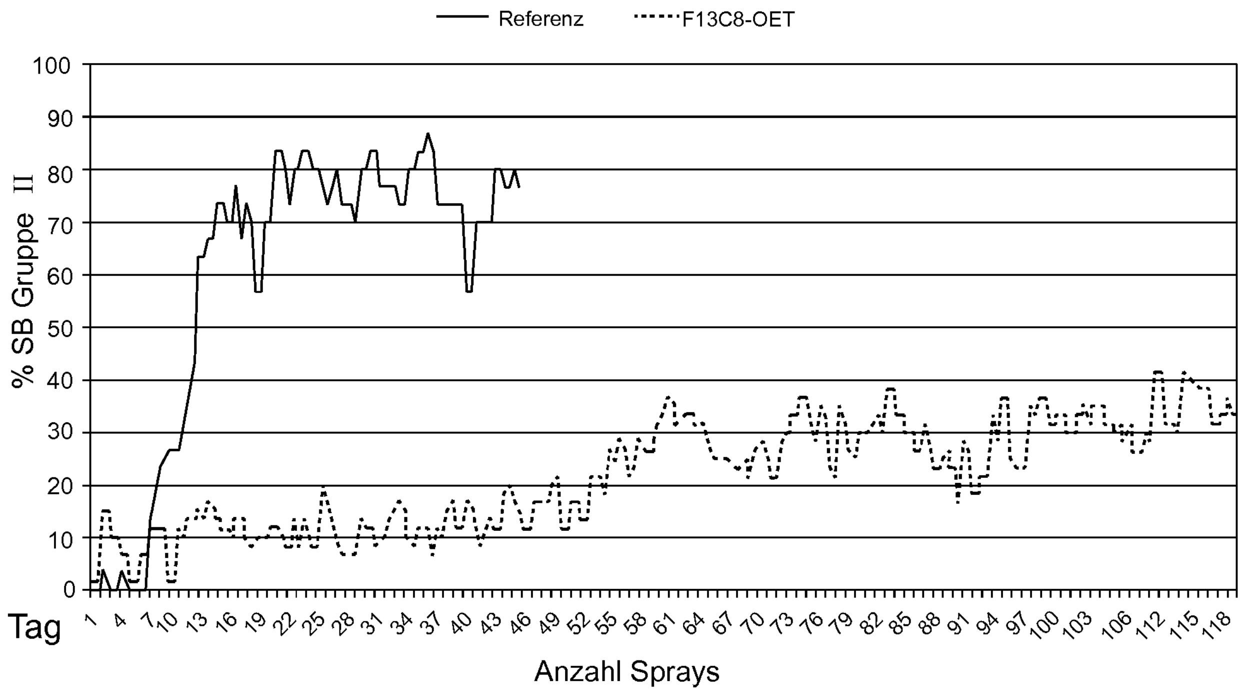
Referenz
F13C8-OET
% SB Gruppe II
Tag
Anzahl Sprays

Fig. 14

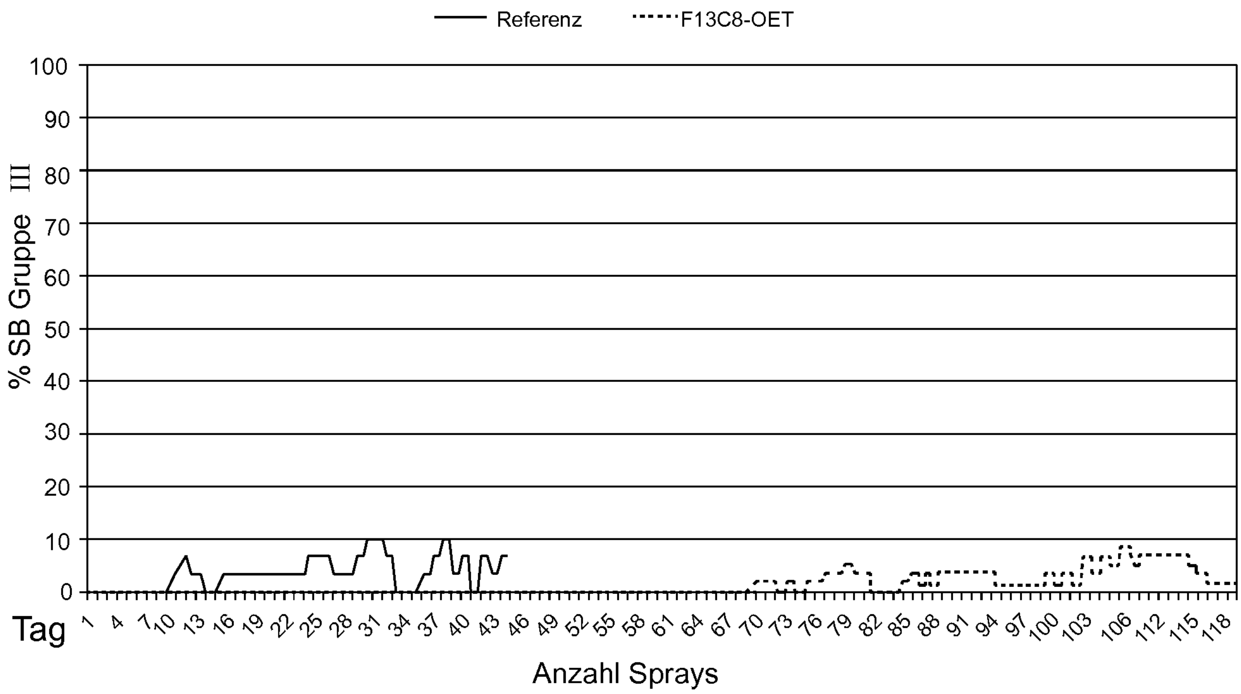

Referenz
F13C8-OET
% SB Gruppe III
100
90
80
70
60
50
40
30
20
10
0
Tag
1 4 7 10 13 16 19 22 25 28 31 34 37 40 43 46 49 52 55 58 61 64 67 70 73 76 79 82 85 88 91 94 97 100 103 106 112 115 118
Anzahl Sprays


Fig. 15

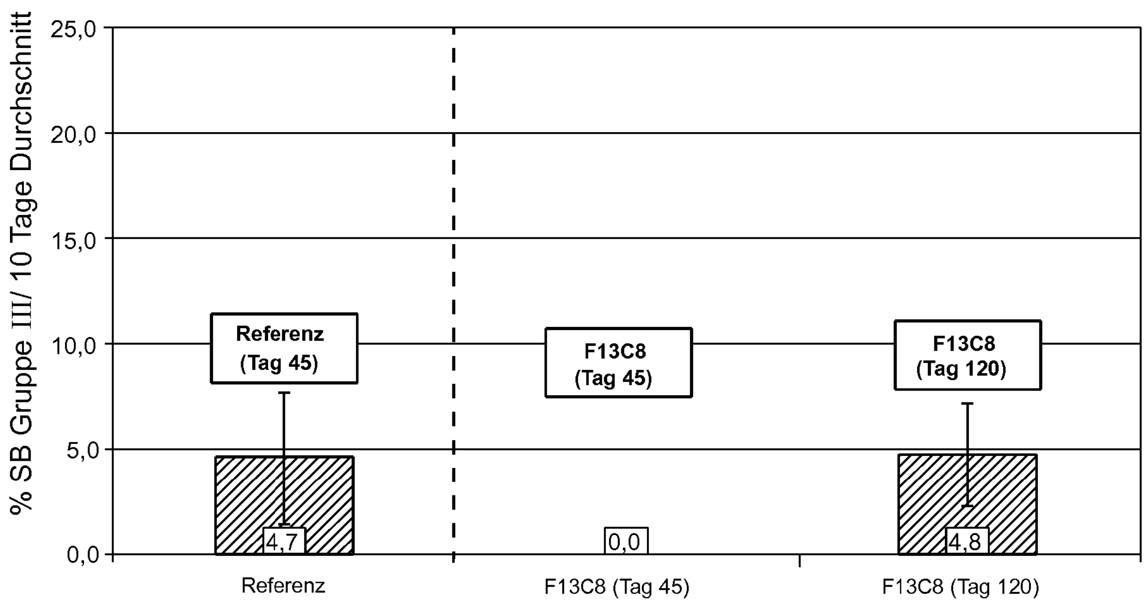

% SB Gruppe III/ 10 Tage Durchschnitt
25,0
20,0
15,0
10,0
5,0
0,0
Referenz (Tag 45)
F13C8 (Tag 45)
F13C8 (Tag 120)
4,7
0,0
4,8
Referenz
F13C8 (Tag 45)
F13C8 (Tag 120)


Fig. 16

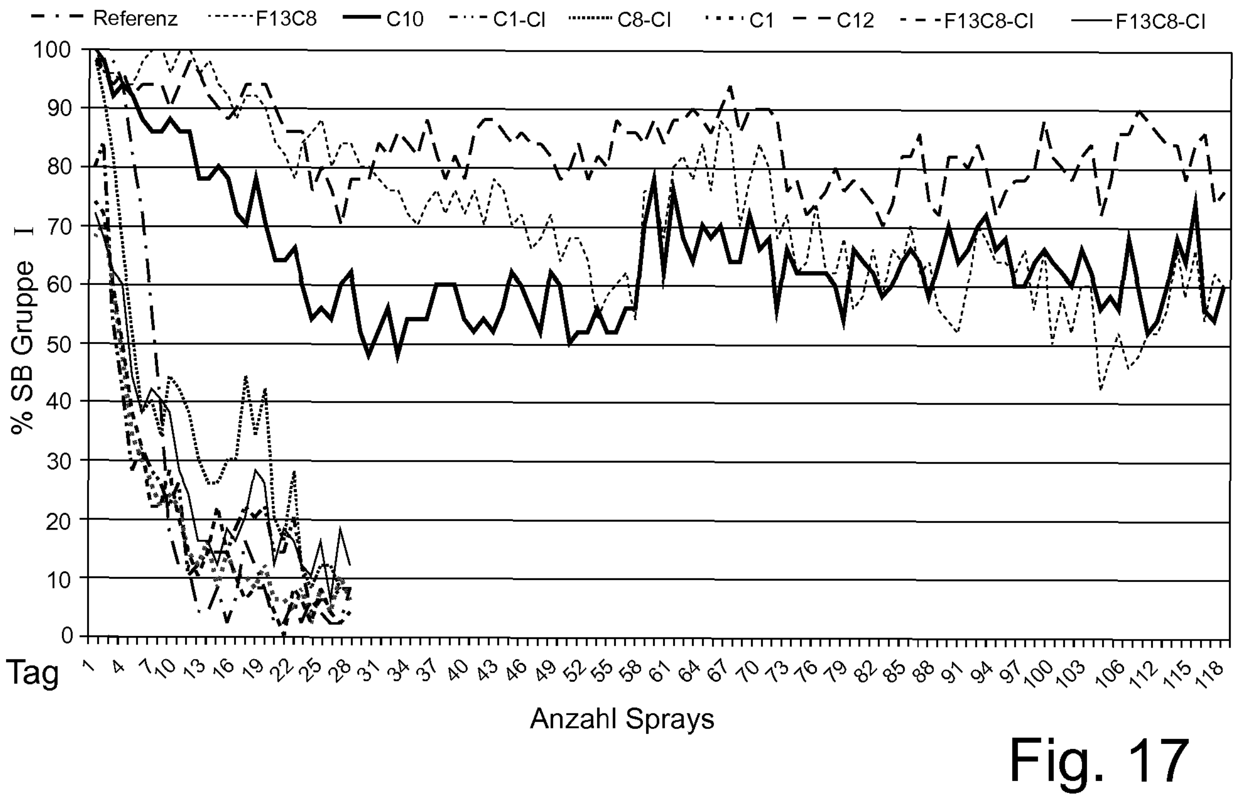
Referenz
F13C8
C10
C1-Cl
C8-Cl
C1
C12
F13C8-Cl
F13C8-Cl
% SB Gruppe I
Tag
Anzahl Sprays

Fig. 17

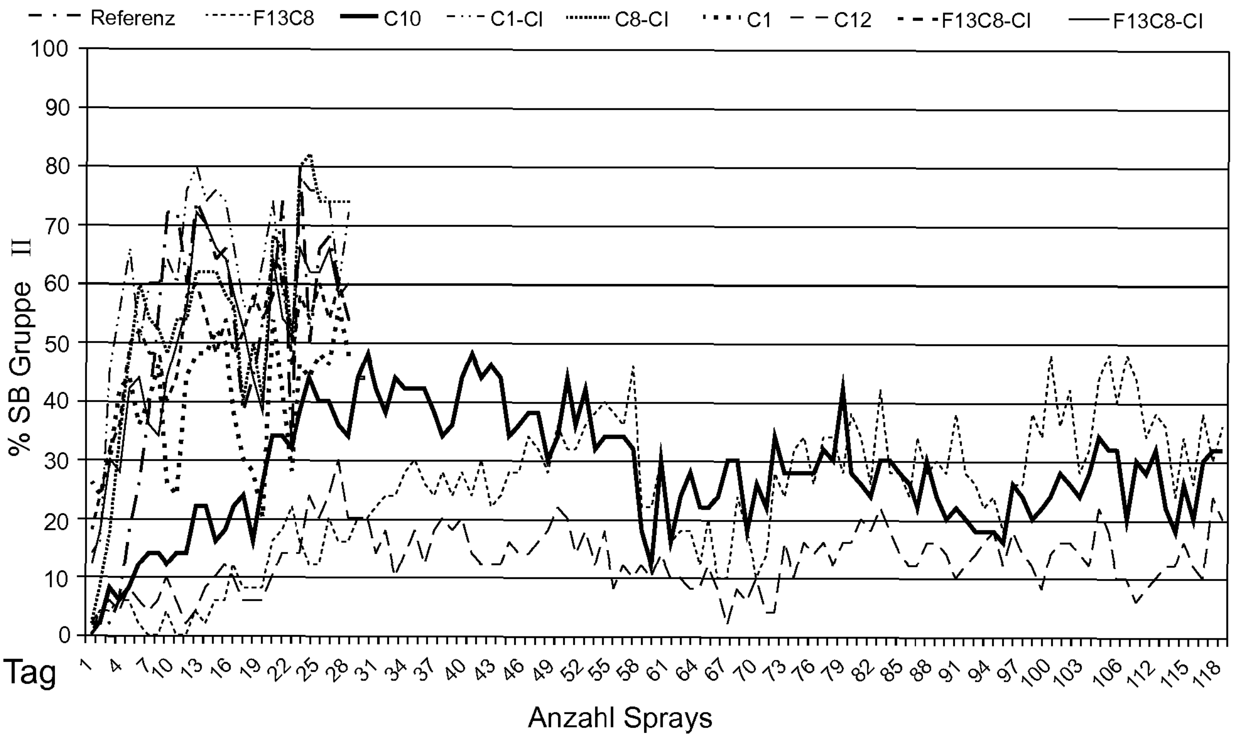
Referenz
F13C8
C10
C1-Cl
C8-Cl
C1
C12
F13C8-Cl
F13C8-Cl
% SB Gruppe II
Tag
Anzahl Sprays

Fig. 18

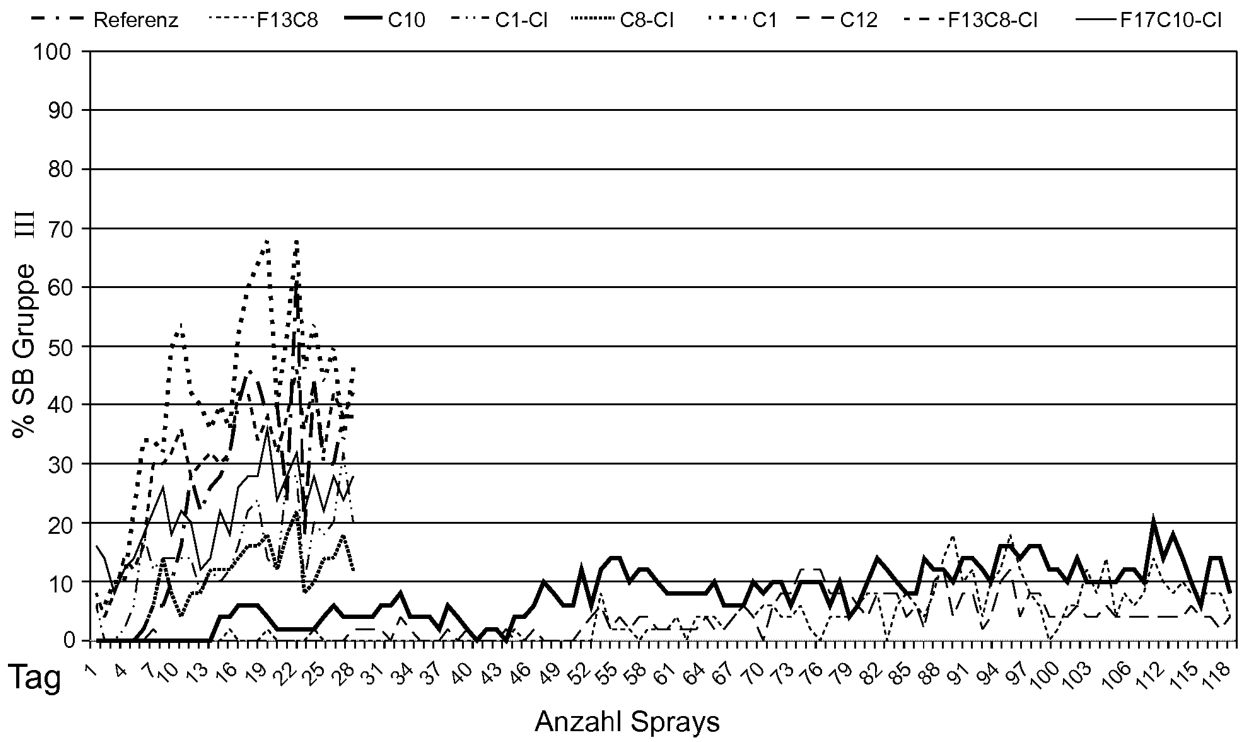
Referenz
F13C8
C10
C1-Cl
C8-Cl
C1
C12
F13C8-Cl
F17C10-Cl
% SB Gruppe III
Tag
Anzahl Sprays

Fig. 19

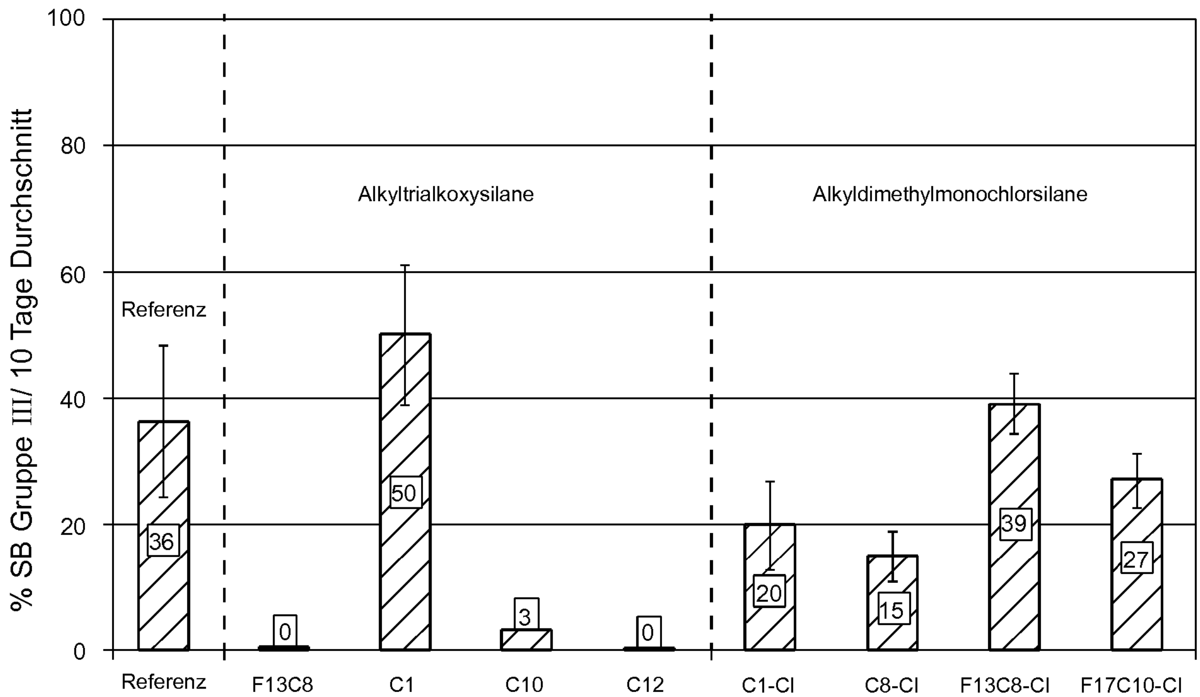
% SB Gruppe III/ 10 Tage Durchschnitt
Alkyltrialkoxysilane
Alkyldimethylmonochlorsilane
Referenz
36
0
50
3
0
20
15
39
27
Referenz
F13C8
C1
C10
C12
C1-Cl
C8-Cl
F13C8-Cl
F17C10-Cl

Fig. 20

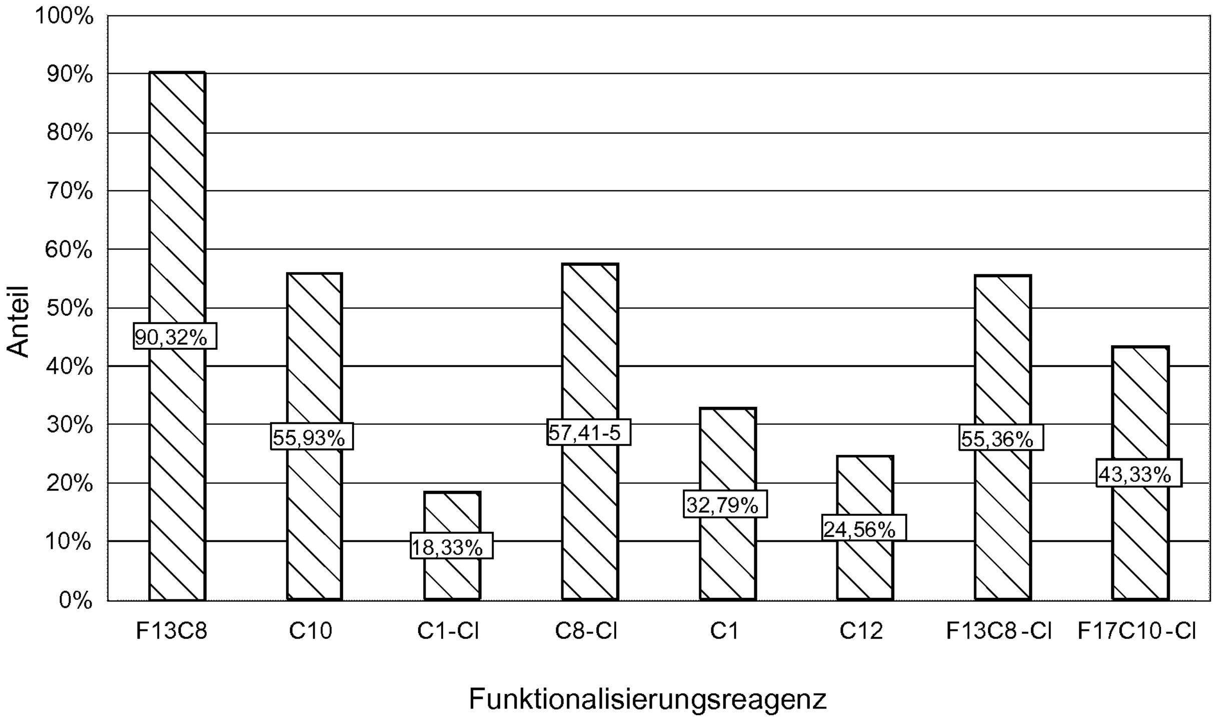
100%
90%
80%
70%
60%
50%
40%
30%
20%
10%
0%
Anteil
90,32%
55,93%
18,33%
57,41-5
32,79%
24,56%
55,36%
43,33%
F13C8
C10
C1-Cl
C8-Cl
C1
C12
F13C8-Cl
F17C10-Cl
Funktionalisierungsreagenz

Fig. 21

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9114468 A1
- WO 2009047173 A2
- WO 2004089551 A1
- WO 2010112358 A2
- EP 1644129 B1
- WO 9407607 A1
- WO 07123381 A1
- WO 06136426 A1
- WO 0049988 A2
- WO 01076849 A1
- WO 9943571 A1
- WO 09115200 A1
- WO 09103510 A1
- WO 2012007315 A


### In der Beschreibung aufgeführte Nicht-Patentliteratur


- **AMBROSINO, N. ; P. PAGGIARO.** The management of asthma and chronic obstructive pulmonary disease: current status and future perspectives. *Expert.Rev.Respir.Med.,* 2012, vol. 6 (1), 117-127
- **GANDERTON, D.** Targeted delivery of inhaled drugs: current challenges and future goals. *J.Aerosol Med.,* 1999, vol. 12, S3-S8
- **PAVIA, D.** Efficacy and safety of inhalation therapy in chronic obstructive pulmonary disease and asthma. *Respirology,* 1997, vol. 2, 5-10
- **ARIYANANDA, P.L. ; J.E. AGNEW ; S.W. CLARKE.** Aerosol delivery systems for bronchial asthma. *Postgrad.Med.J.,* 1996, vol. 72 (845), 151-156
- **DALBY, R. ; M. SPALLEK ; T. VOSHAAR.** A review of the development of Respimat Soft Mist Inhaler. *Int.J.Pharm.,* 2004, vol. 283 (1-2), 1-9
- **PASO, K. et al.** Hydrophobic monolayer preparation by Langmuir-Blodgett and chemical adsorption techniques. *Journal of colloid and interface science,* 2008, vol. 325 (1), 228-235
- **KERN, W.** Cleaning solutions based on hydrogen peroxide for use in silicon semiconductor technology. *RCA review,* 1970, vol. 31, 187-206
- **IRENE, E.A. ; H.G. TOMPKINS.** Handbook of Ellipsometry. William Andrew Pub, 2005
- **FADEEV, A.Y. ; T.J. MCCARTHY.** Trialkylsilane monolayers covalently attached to silicon surfaces: wettability studies indicating that molecular topography contributes to contact angle hysteresis. *Langmuir,* 1999, vol. 15 (11), 3759-3766
- **JUNG, J.-I. ; J.Y. BAE ; B.-S. BAE.** Characterization and mesostructure control of mesoporous fluorinated organosilicate films. *Journal of Materials Chemistry,* 2004, vol. 14 (13), 1988-1994
- **PLUEDDEMANN, E.P.** Silane Coupling Agents. Plenum Publishing Corporatio, 1991
- **SIEVAL, A.B. et al.** Molecular modeling of covalently attached alkyl monolayers on the hydrogenterminated Si (111) surface. *Langmuir,* 2001, vol. 17 (7), 2172-2181
- **GIESSLER, S. ; E. JUST ; R. STÖRGER.** Easy-to-clean properties-Just a temporary appearance. *Thin Solid Films,* 2006, vol. 502 (1), 252-256